# EUROPEAN PATENT SPECIFICATION

(11) **EP 4 114 391 B1**
(45) Date of publication and mention of the grant of the patent: **09.07.2025**
(21) Application number: 21763872.5
(22) Date of filing: 05.03.2021
(51) Int. Cl.: A61K 31/445, A61K 31/4523, A61K 31/4545, C07D 471/00, C07D 471/02, C07D 471/04, A61K 31/436, A61K 31/138, A61K 31/4015, A61K 31/437, A61K 31/4439, A61K 31/00, A61K 45/06, A61P 35/00, A61P 35/04

(54) **COMPOSITIONS FOR USE IN A METHOD OF TREATING AN ESTROGEN RECEPTOR ASSOCIATED CANCER**
ZUSAMMENSETZUNGEN ZUR VERWENDUNG IN EINEM VERFAHREN ZUR BEHANDLUNG EINES ESTROGEN-REZEPTOR-ASSOZIIERTEN KREBS
COMPOSITIONS À UTILISER DANS UNE MÉTHODE DE TRAITEMENT D'UN CANCER ASSOCIÉ À DES RÉCEPTEURS D'OESTROGÈNES

(30) Priority: 06.03.2020 US 202062985929 P
(43) Date of publication of application: 11.01.2023
(73) Proprietor: Olema Pharmaceuticals, Inc., San Francisco, CA 94107 (US)
(72) Inventor: HARMON, Cyrus L., San Francisco, CA 94107 (US); KUSHNER, Peter J., San Francisco, CA 94107 (US); MYLES, David C., San Francisco, CA 94107 (US); GALLAGHER, Leslie Hodges, San Francisco, CA 94107 (US)
(74) Representative: Schiweck Weinzierl Koch Patentanwälte Partnerschaft mbB
(86) International application number: PCT/US2021/021151
(87) International publication number: WO 2021/178846

(56) References cited:
- WO-A1-2015/149045
- WO-A1-2017/059139
- WO-A1-2020/023297
- WO-A2-2020/037203
- US-A1- 2012 157 402
- US-A1- 2018 214 393
- US-A1- 2018 235 945
- US-A1- 2019 247 372

## Description

### BACKGROUND

The estrogen receptor (ER) plays important roles in various cancers, including breast cancers. A variety of treatments have been developed to target the estrogen receptor and/or its activities. Cancer cells in the brain are particularly problematic. For hormone positive disease e.g., ER-positive breast cancer, the incidence of breast cancer brain metastases (BCBMs) is 14%, with a median overall survival after the development of brain metastases of 9-10 months. Brosnan, Ann Transl Med., 2016;6(9):163.

### SUMMARY

The scope of the present invention is defined in the claims; any other disclosure in the present description not protected by the appended claims, regardless of whether it is indicated as being preferred or exemplified, is provided for information purposes, only.

Any references in the description to methods of treatment refer to the compounds, pharmaceutical compositions and medicaments of the present invention for use in a method for treatment by therapy. In some aspects, the present disclosure provides new insights regarding compounds and/or regimens useful for the treatment of estrogen receptor (ER)-associated diseases, disorders, and conditions (e.g., cancer cells) and/or for otherwise modulating (e.g., inhibiting) the estrogen receptor in the brain.

Among other things, some aspects of the present disclosure define particular structural and/or functional attributes that characterize compounds useful for such treatment and/or modulation.

In some aspects, the present disclosure provides certain non-invasive technologies for treatment of ER-associated diseases, disorders, and conditions (e.g., cancer cells) and/or for otherwise modulating (e.g., inhibiting) the estrogen receptor in the brain. For example, in some embodiments, the present disclosure defines compounds that, when administered systemically (e.g., orally), inhibit the estrogen receptor in the brain. Among other things, the present disclosure defines complete estrogen receptor antagonist compounds useful as described herein (e.g., orally bioavailable complete receptor antagonists that cross the blood brain barrier).

There remains a need for anti-estrogen agents that can completely inhibit estrogen receptors, including those coded for by both wild-type and mutant versions (e.g., those containing activating mutations) of the gene encoding Estrogen Receptor-alpha (ERα), Estrogen Receptor 1 (ESR1). Selective estrogen receptor modulators (SERMs) or degraders (SERDs) are a particularly useful or promising tools for such therapy. The estrogen receptor is a tripartite protein comprising two distinct transcriptional activation functions (AF1 and AF2). Complete anti-estrogen activity requires inactivation of both AF1 and AF2. Activating mutations in the gene that codes for estrogen receptor 1 allows for activation of both AF1 and AF2 even in the absence of estrogen.

Further, there remains a need for identification, characterization, and or treatment of certain brain lesions (e.g., brain tumors such as brain metastases) that do not require invasive techniques such as whole-brain radiotherapy and/or surgery, in particular for estrogen-receptor (ER)-associated diseases, disorders or conditions, such as ER-positive cancers. The present application provides technologies that relate to such identification, characterization, and/or treatment. In some embodiments, provided technologies comprise administering a composition that comprises and/or delivers a complete estrogen receptor antagonist as described herein (e.g., a complete receptor antagonist that is orally bioavailable and/or that crosses the blood brain barrier).

Those skilled in the art are aware that many existing strategies for treating ER-associated diseases, e.g., cancer, include first line therapies, such as tamoxifen and/or endoxifen, to which patients eventually develop resistance. Certain second line therapies, such as fulvestrant, have been developed, but work continues in pursuit of improved therapeutic strategies.

Previous therapies, such as tamoxifen, AZD9496, and ARN-810 are not complete estrogen receptor antagonists because they fail to disable both activation functions (i.e., do not disable both AF1 and AF2). As such, there remains a need for therapies that disable both AF1 and AF2 in order to completely inhibit the estrogen receptor, and moreover, there remains a need for therapies that inhibit the estrogen receptor despite activating mutations. The present disclosure documents, among other things, that certain compounds, alone or in combination with other agents, can be used as a treatment for patients or subjects suffering from a cancer, and wherein the patient or subject carries a mutation of estrogen receptor 1 (ESR1).

Fulvestrant has been celebrated as a so-called "complete" estrogen receptor antagonist because, unlike other approved ER antagonists (e.g., antiestrogens, etc), fulvestrant is characterized by its ability to (1) inhibit *both* activating function 1 (AF1) and activating function 2 (AF2), as *complete* anti-estrogen activity requires inactivation of both AF1 and AF2; (2) promote ER degradation; and (3) avoid partial ER agonist activity. Fulvestrant is currently the only approved such "complete" ER antagonist; it has been approved for use by intramuscular injection in certain hormone receptor (HR)-positive breast cancers, including specifically in HR-positive metastatic breast cancer in postmenopausal women both with disease progression following endocrine therapy and as initial treatment. Fulvestrant may also be used in concert with cyclin dependent kinase 4/6 inhibitors such as pablociclib, ribociclib, and abemaciclib for either initial treatment or following progression on endocrine therapy for metastatic or locally advanced breast cancer.

However, fulvestrant suffers from poor oral bioavailability and has to be administered parenterally. Moreover, fulvestrant is not useful to treat brain tumors (e.g., metastases), as it does not cross the blood brain barrier.

The international Patent application publication No. WO 2015/149045 A1 discloses methods of treating subjects suffering from estrogen receptor positive cancer of the brain by administering a particular selective estrogen receptor modulator (SERM). Also disclosed are methods of treating a cancer that is resistant to an estrogen receptor modulator by administering a SERM.

According to the present invention, there is provided a composition for use in a method of treating an ER-associated cancer in a subject in accordance with claim 1. There is further provided a compound for use in a method of preventing metastatic spread of an ER-associated cancer to the brain of a subject according to claim 8. Further advantageous embodiments of the present invention are set out in the appended dependent claims. Aspects of the disclosure provided herein are for illustration purposes only and are not part of the invention.

Some aspects of the present disclosure provide insights relating to complete estrogen receptor antagonists that cross the blood brain barrier and/or are orally available, and furthermore describes usefulness of such compounds in detection, assessment, and/or treatment of one or more ER-associated diseases, disorders or conditions (e.g., metastasized ER-positive cancer such as metastasized ER-positive breast cancer). Aspects of the present disclosure specifically exemplify blood-brain-barrier crossing by certain orally-bioavailable complete estrogen receptor antagonists, and furthermore provide insights regarding particular structural features and/or combinations of features that may contribute to and/or be responsible for blood-brain-barrier-crossing and/or complete estrogen receptor antagonism activity. Without wishing to be bound by any particular theory, aspects of the present disclosure define a structure-function correlation for complete estrogen receptor antagonists that cross the blood-brain barrier and/or are orally bioavailable.

Among other things, aspects of the present disclosure provide compositions for use in methods of using such complete estrogen receptor antagonists (i.e., that cross the blood-brain barrier and/or are orally bioavailable), including specifically to detect, assess, and/or treat brain lesions (e.g., tumors such as metastases). In some aspects, the present disclosure provides such composition for use in methods with respect to an ER-associated disease, disorder or condition (e.g., an ER-associated cancer). In some aspects, a provided composition for use in a method comprises a step of: administering to a subject suffering from an ER-associated cancer a composition that comprises and/or delivers to the subject's brain (e.g., upon oral administration) a complete estrogen receptor antagonist, wherein the subject has been determined to have or is suspected of having brain metastases.

Some aspects of the present disclosure define structural features characteristic of complete receptor antagonists that cross the blood-brain barrier (e.g., upon oral administration). For example, in some aspects, without wishing to be bound by any particular theory, the present disclosure defines structure-function correlation(s) between one or more of complete ER antagonism (as defined herein), ability to cross the blood-brain barrier, and/or oral bioavailability. In some aspects, the present disclosure defines structure-function correlation(s) for (e.g., defines structural characteristics characteristic of) compounds that demonstrate all of these. For example, without wishing to be bound by any particular theory, in some aspects, the present disclosure provides an insight that certain structural element(s) contribute to this combination of desirable activities, and, in certain embodiments, defines a potential structure-function correlation therewith.

In some aspects, the present disclosure teaches that compounds having a pyrido[3,4-b]indole group and/or an azetidinyl group, and/or a substituted or unsubstituted phenyl moiety are particularly useful as complete estrogen receptor antagonists that are orally bioavailable and capable of crossing the blood brain barrier. For example, in some aspects, the present disclosure teaches that compounds having a pyrido[3,4-b]indole group covalently bound to a phenyl linker, and an azetidinyl moiety bound to the phenyl linker via a heteroatom or heteroalkyl linker, is a particularly useful complete estrogen receptor antagonist that is orally bioavailable and capable of crossing the blood brain barrier. In some aspects, a phenyl moiety is additionally substituted with one or more halogen atoms (e.g., F, Br, Cl, I). In some aspects, a phenyl moiety is not additionally substituted (e.g., contains no additional substitution aside from the bonds to the pyrido[3,4-b]indole group and the heteroatom or heteroalkyl linking the phenyl group and the azetidinyl moiety). In some aspects, the azetidinyl moiety is distal to the phenyl group. In some embodiments, the azetidinyl moiety is proximal to the phenyl group. In some aspects of the disclosure, the azetidinyl moiety is further substituted with alkyl or haloalkyl.

In some aspects, the present disclosure teaches that compounds having a structure as set forth in Formula I may be particularly useful as described herein: 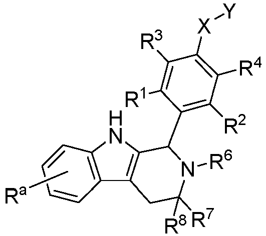 wherein R^{a}, R¹, R², R³, R⁴, R⁶, R⁷, R⁸, X, and Y are described herein.

In particular aspects, the present disclosure provides an insight that one or more features of the pyrido[3,4-b]indole group and/or of the X-Y group of Formula I, and/or of combination(s) thereof may provide or participate in described function(s) (e.g., complete estrogen receptor antagonism, ability to cross the blood-brain barrier, oral bioavailability and/or combinations thereof).

In some aspects, the present disclosure teaches that compounds having a structure as set forth in Formula I, wherein the compound comprises an azetidinyl moiety, may provide or participate in desired function(s) (e.g., complete estrogen receptor antagonism, oral bioavailability, an ability to cross the blood-brain barrier, and/or combinations thereof).

In some aspects, the present disclosure teaches that compounds having a structure as set forth in Formula I, wherein the compound does not comprise a difluorophenyl moiety, may provide or participate in desired function(s) (e.g., complete estrogen receptor antagonism, oral bioavailability, an ability to cross the blood-brain barrier, and/or combinations thereof).

In some aspects, the present disclosure teaches that compounds having a structure as set forth in Formula I, wherein the compound comprises a difluorophenyl moiety, may provide or participate in desired function(s) (e.g., complete estrogen receptor antagonism, oral bioavailability, an ability to cross the blood-brain barrier, and/or combinations thereof).

In some aspects, the present disclosure teaches that one or more of the following compounds may be particularly useful as described herein: GDC-9545, SAR439859, AZD9833, 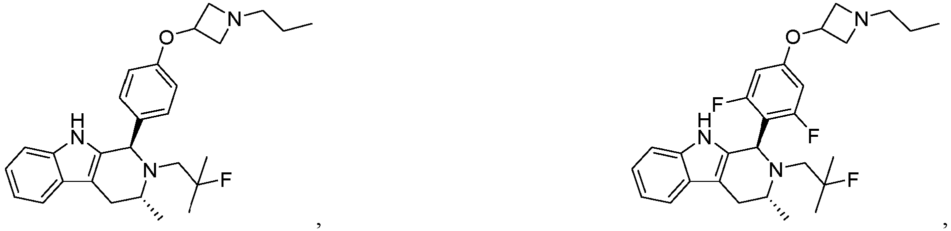 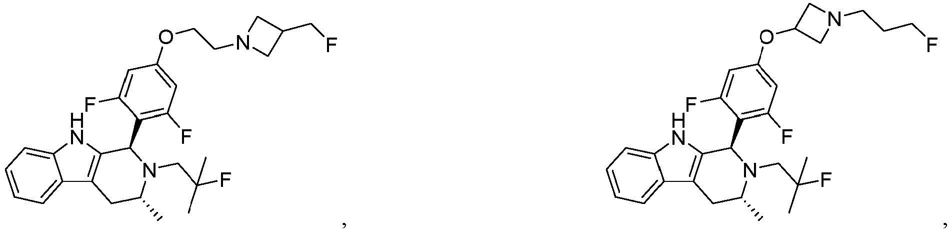 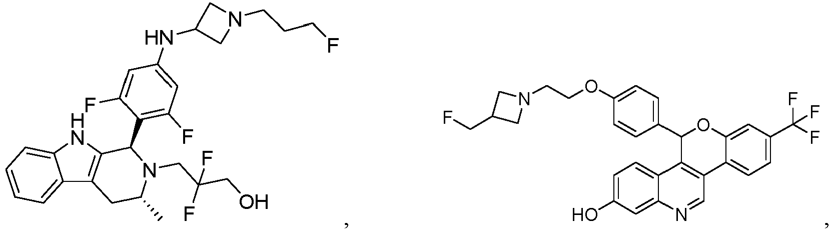 and

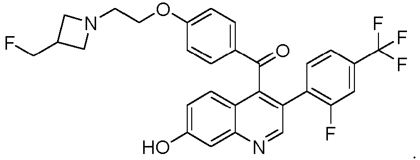

Still further, aspects of the present disclosure demonstrates that certain complete estrogen antagonists as described herein (e.g., having a structure within Formula I), when administered according to an appropriate regimen, can accumulate preferentially in tumor(s) relative to plasma, including to a degree significantly greater than that observed for fulvestrant.

Still further, aspects of the present disclosure provides various effective dosing regimens for certain complete estrogen antagonists as described herein (e.g., having a structure within Formula I). Aspects of the present disclosure provide methods of treating an ER-associated disease disorder or condition (e.g., an ER-associated cancer, including but not limited to one that is or comprises tumor(s) in the brain such as brain metastases) by administering such certain complete estrogen receptor antagonist(s) according to such regimen(s).

### BRIEF DESCRIPTION OF THE DRAWING

FIG. 1A is a scatter plot measuring the volume (mm³) by MRI at inclusion in ST941 brain metastases for each mouse within each group A-E.
FIG. 1B is a scatter plot measuring the weight (g) by MRI at inclusion in ST941 brain metastases for each mouse within each group A-E.
FIG. 2 is a scatter plot measuring the mean tumor volume for each group A-E over time.
FIG. 3A is a scatter plot measuring the mean body weight for each group A-E over time.
FIG. 3B is a scatter plot measuring the percent change in mean body weight for each group A-E over time.
FIG. 4 is a Kaplan-Meier plot showing the percentage of mice surviving within each of groups A-E over time. Tick marks indicate censored data reflecting that mice were enrolled on different days.
FIG. 5A is a scatter plot measuring percent of estrogenic response as a function of Log[M] for certain estrogen receptor antagonist compounds where no estrogen has been added.
FIG. 5B is a scatter plot measuring percent of estrogenic response as a function of Log[M] for certain estrogen receptor antagonist compounds where estrogen has been added.
FIG. 6 is a chart measuring degradation of the estrogen receptor protein across multiple cell lines for multiple estrogen receptor antagonists.

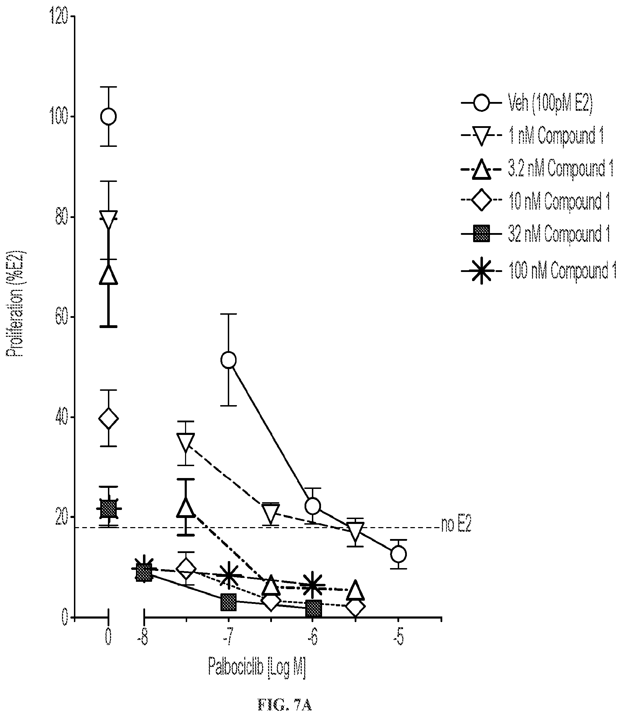

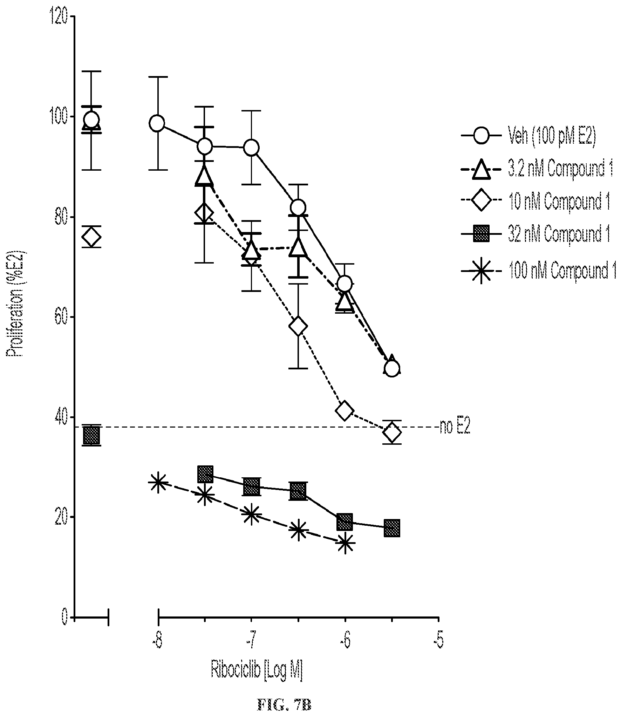

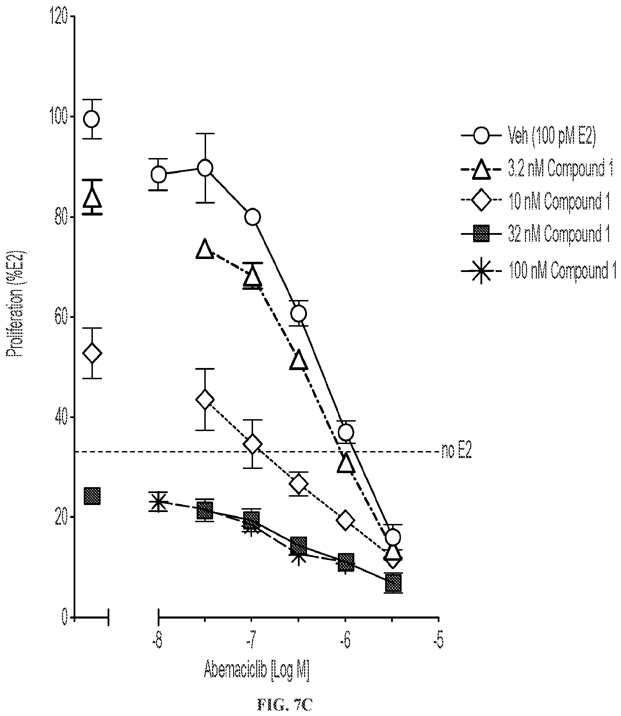

FIGs. 7A-7C are scatter plots illustrating the decreased percent of estrogen response for Compound 1 in combination with various CDK4/6 inhibitors.

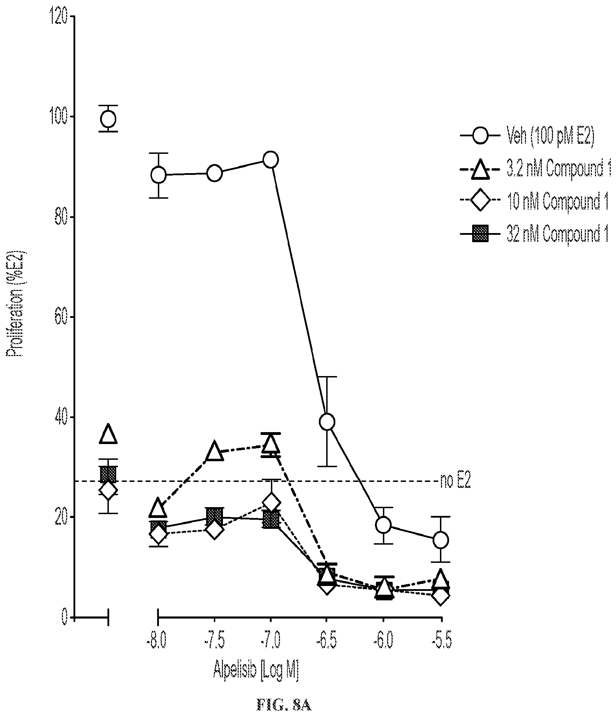

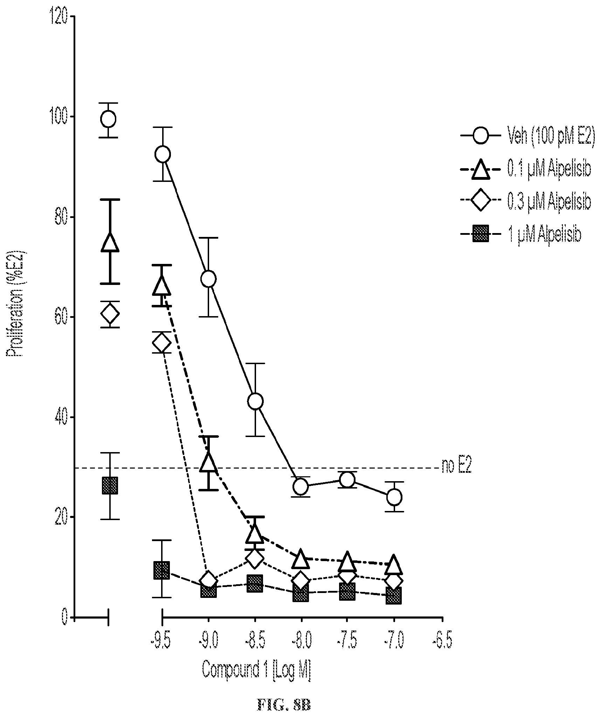

FIGs. 8A-8B are scatter plots illustrating the decreased percent of estrogen proliferation to MCF-7 cells when treated with Compound 1 in combination with a PIK3CA inhibitor.
FIGs. 9A-9F provides scatter plots, one for each cell line of ERα (FIG. 9A - wild type; FIG. 9B - D538G; FIG. 9C - Y537S; FIG. 9D - Y537C; FIG. 9E - Y537N; and FIG. 9F - Y537R) illustrating dose response of Compound 1 for AF1 inhibition with most common ESR1 mutations.

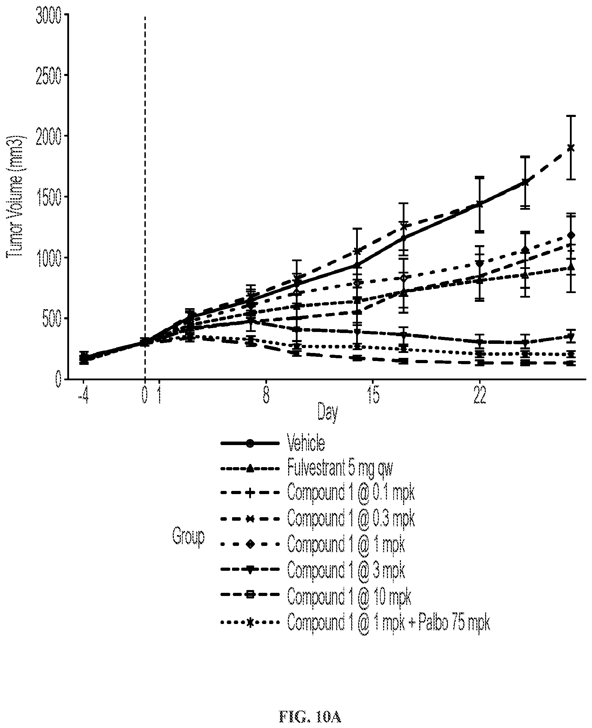

FIG. 10A is a scatter plot illustrating mean tumor volume for varying doses of Compound 1 in mice.

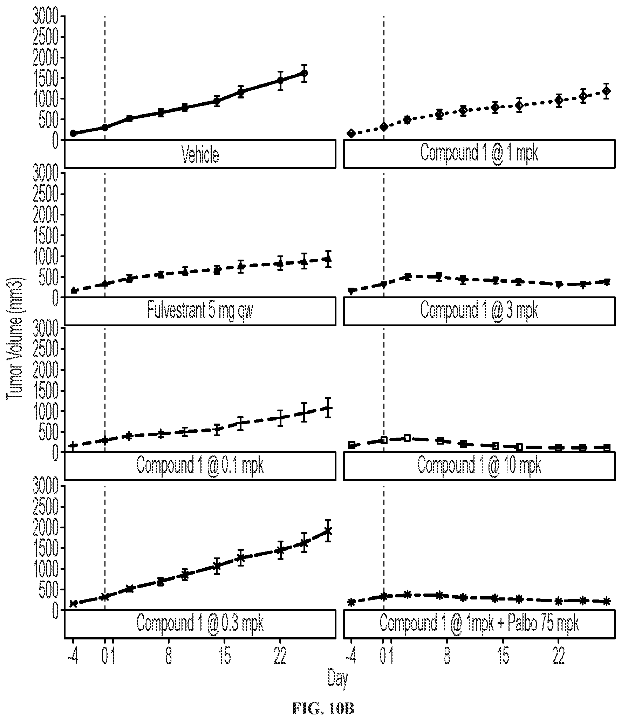

FIG. 10B is a series of plots isolated from FIG. 10A illustrating mean tumor volume for varying doses of Compound in mice.

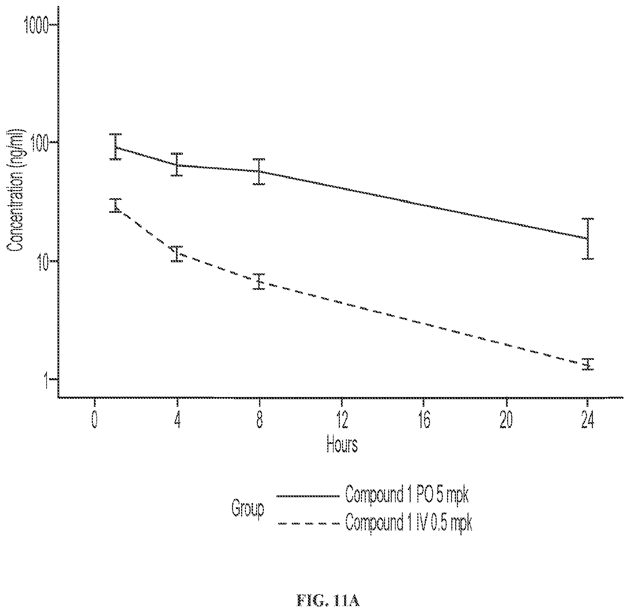

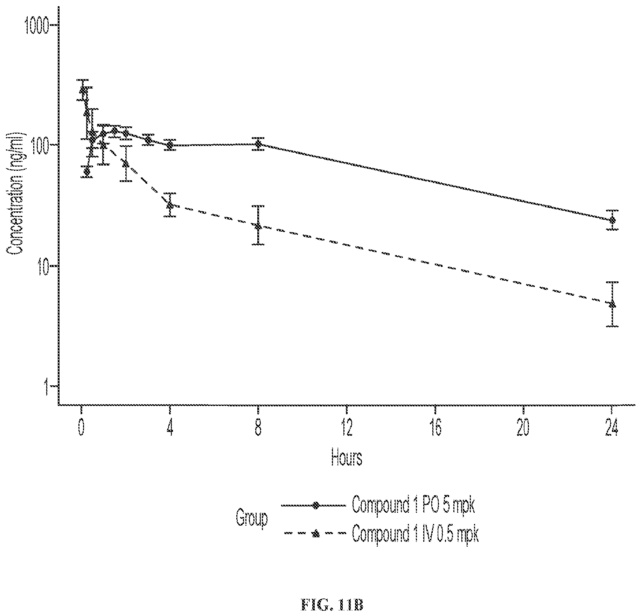

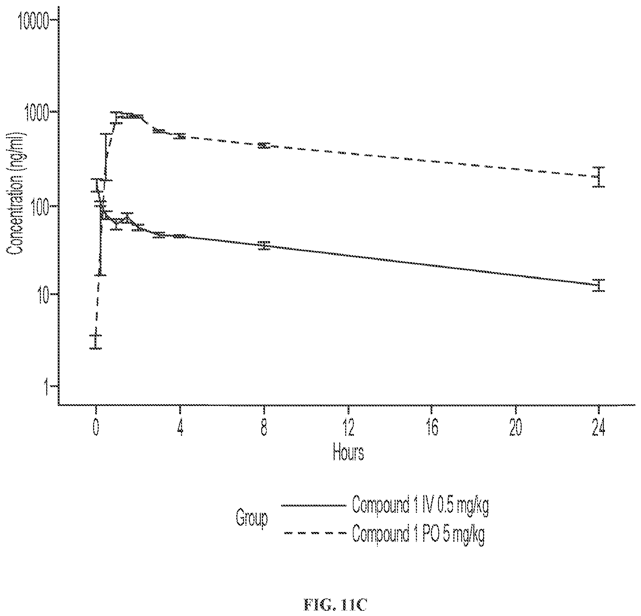

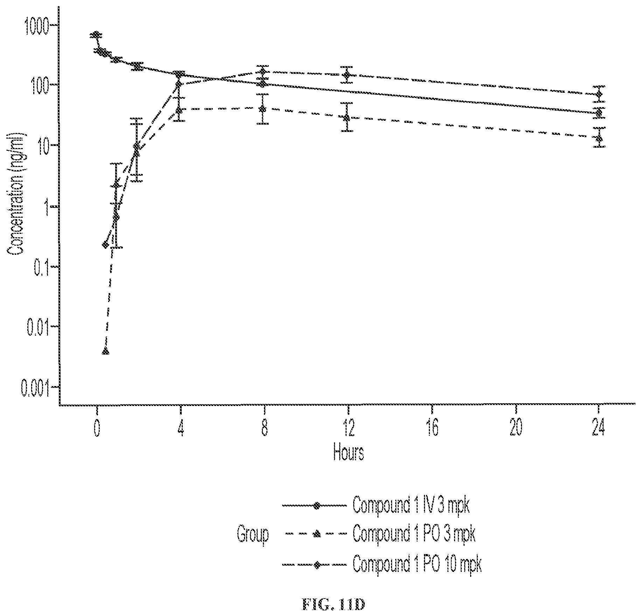

FIGs. 11A-11D are scatter plots measuring drug exposure (ng/ml) over time for mouse FIG. 11A), rat (FIG. 11B), dog (FIG. 11C), and monkey (FIG. 11D).
FIGs. 12A-12D are scatter plots illustrating the decreased in estrogen concentration across different cell lines.
FIG. 13 is a bar graph illustrating that mutant ERs increase ligand-independent alkaline phosphatase activity (AP) in Ishikawa endometrial cancer cells.

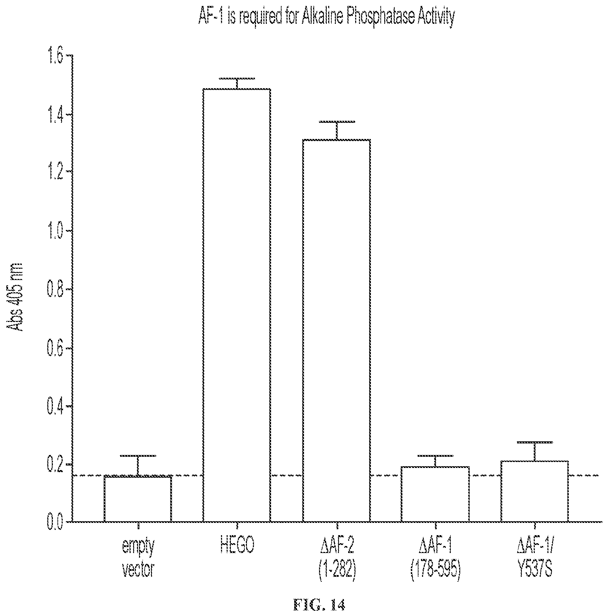

FIG. 14 is a bar graph illustrating that activation domain 1 (AF1) of the ER is required for AP activity.

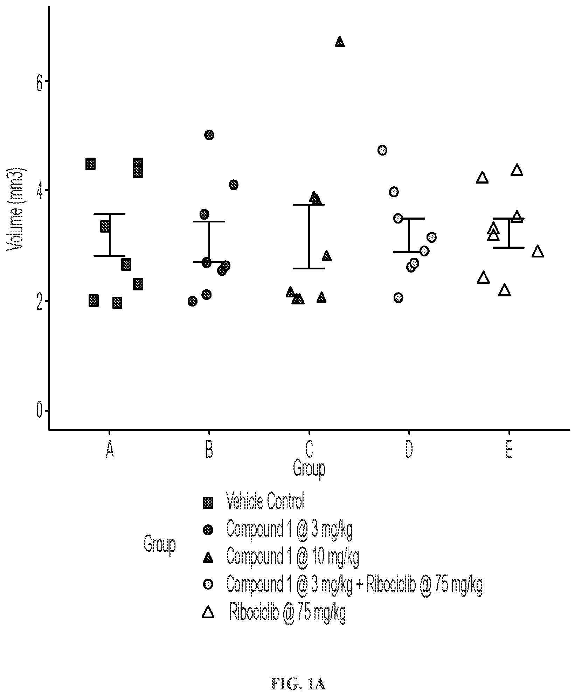

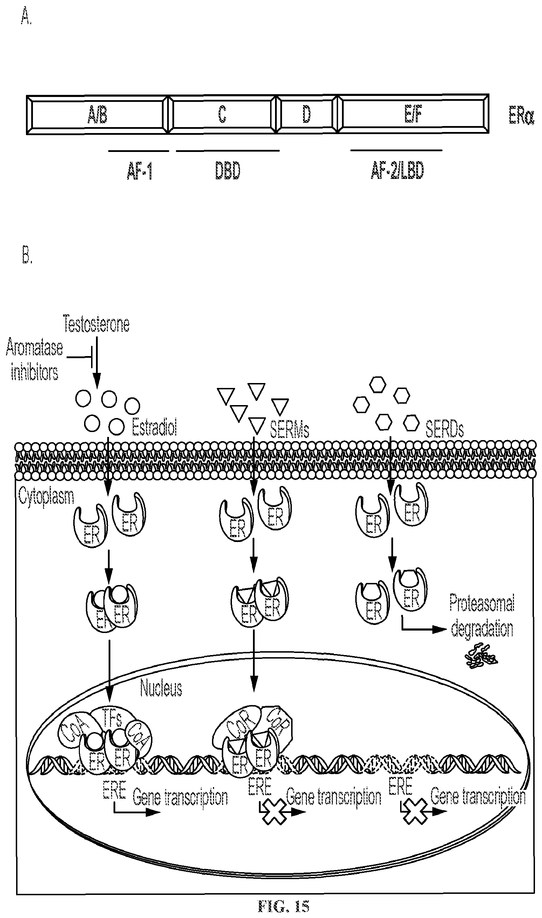

FIG. 15 is a reproduction of Figure 1A of Patel & Bihani Pharm & Therap 186:1, 2018, illustrating that mammals express two major isoforms of the ER, known as ERα and ERβ, each of which is a member of the nuclear hormone receptor family. A) The A-F domains that constitute the estrogen receptor, which include the activation function 1 (AF1) domain, the DNA binding domain (DBD), the hinge region, and the ligand binding domain (LBD)/activation function 2 (AF2 domain). B) The effects of endocrine therapies (aromatase inhibitors, SERMs, and SERDs) on the estrogen receptor pathway. Aromatase inhibitors prevent ER signaling by inhibiting synthesis of estradiol, SERMs prevent ER signaling by binding to ER and causing an inactive complex, and SERDs prevent ER signaling by causing degradation of ER.

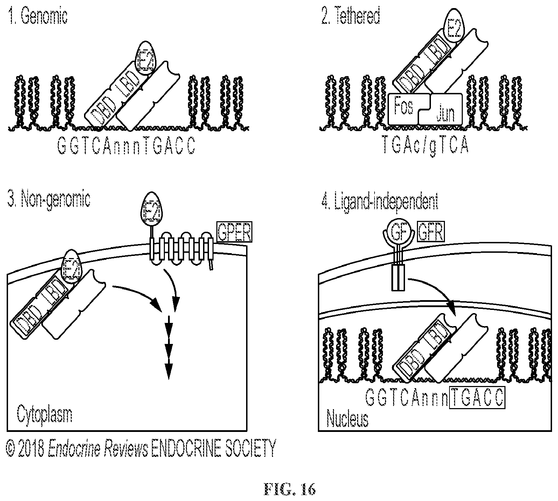

FIG. 16 is a reproduction of Figure 3 of Hewitt & Korach Endocrine Rev. 39:664-674 (June 12, 2018), illustrating variations in the basic mechanism of E2 response.

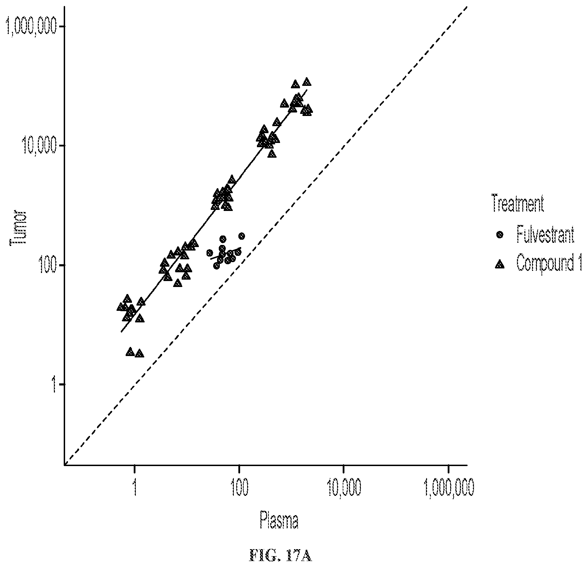

FIG. 17A is a scatter plot measuring the concentration of fulvestrant and Compound 1 in the tumor vs the plasma (log scale with fit).

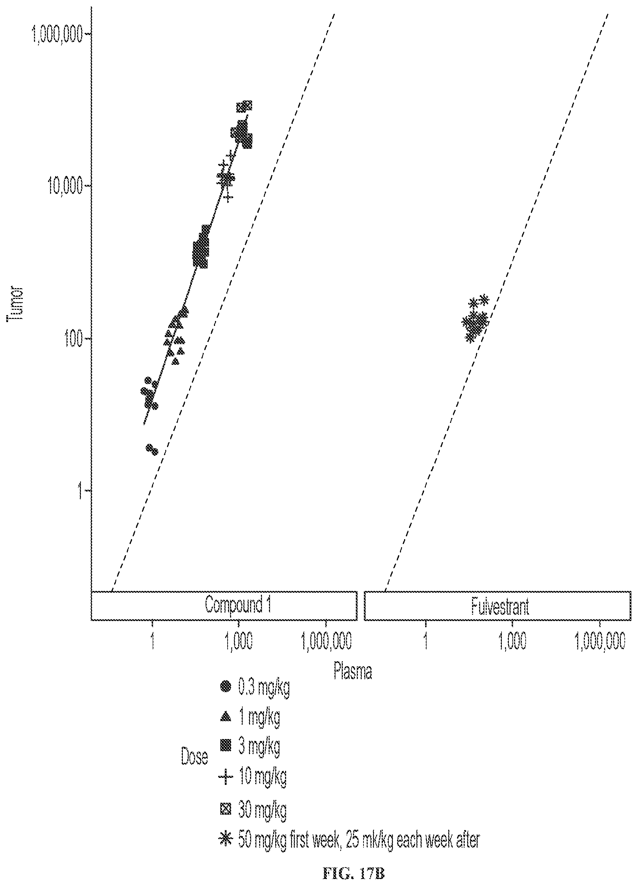

FIG. 17B is a scatter plot measuring the concentration of fulvestrant and Compound 1 in the tumor vs the plasma (log scale with fit).

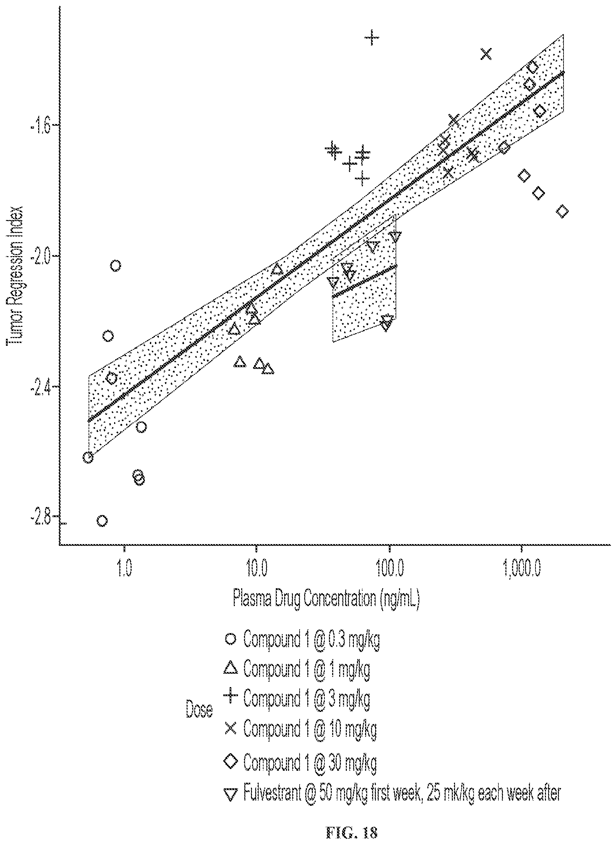

FIG. 18 is a scatter plot measuring tumor regression (y-axis) vs plasma concentration (x-axis) for fulvestrant and Compound 1.

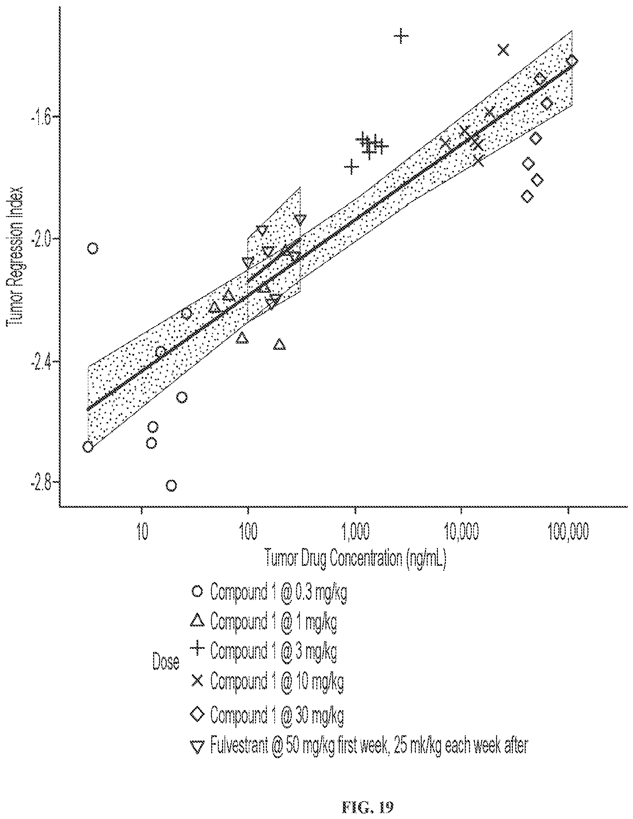

FIG. 19 is a scatter plot measuring tumor regression (y-axis) vs tumor concentration (x-axis) for fulvestrant and Compound 1.
FIG. 20A is a scatter plot measuring each animal's body weight at time 0. "Cmpd 1" refers to "Compound 1" as described herein.
FIG. 20B is a scatter plot measuring each animal's tumor volume at time 0. "Cmpd 1" refers to "Compound 1" as described herein.
FIG. 21 is a Kaplan-Meier plot showing the percentage of surviving mice in each Group over time. Tick marks indicate censored data reflecting that mice were enrolled on different days. "Cmpd 1" refers to "Compound 1" as described herein.
FIG. 22A is a scatter plot measuring mean tumor volume over time for the each Group. "Cmpd 1" refers to "Compound 1" as described herein.
FIG. 22B is a scatter plot measuring percent change in tumor volume over time for each Group. "Cmpd 1" refers to "Compound 1" as described herein.
FIG. 23A is a waterfall plot measuring the percent change in tumor volume for each Group. "Cmpd 1" refers to "Compound 1" as described herein.
FIG. 23B is a zoomed in waterfall plot of FIG. 23A, illustrating the decrease in tumor volume size for certain Groups. "Cmpd 1" refers to "Compound 1" as described herein.
FIG. 24 is a scatter plot measuring mean tumor volume over time for individual animals of each Group. "Cmpd 1" refers to "Compound 1" as described herein.
FIG. 25 is a scatter plot measuring percent change in tumor volume over time for individual animals of each Group. "Cmpd 1" refers to "Compound 1" as described herein.
FIG. 26 is a scatter plot measuring animal weight over time for each Group. "Cmpd 1" refers to "Compound 1" as described herein.
FIG. 27 is a scatter plot measuring percent change in animal weight over time for each Group. "Cmpd 1" refers to "Compound 1" as described herein.

### DETAILED DESCRIPTION

There remains a need for treatment of certain brain metastases that do not require invasive techniques such as whole-brain radiotherapy and surgery, in particular for estrogen receptor (ER) positive cancer types.

The present invention provides a composition for use in a method of treating an ER-associated cancer in a subject in accordance with claim 1. There is further provided a compound for use in a method of preventing metastatic spread of an ER-associated cancer to the brain of a subject according to claim 8. Further advantageous embodiments of the present invention are set out in the appended dependent claims. Aspects of the disclosure provided herein are for illustration purposes only and are not part of the invention.

### Definitions

***Administration*:** As used herein, the term "administration" typically refers to the administration of a composition to a subject or system, for example to achieve delivery of an agent that is, or is included in or otherwise delivered by, the composition.

***Agent*:** As used herein, the term "agent" refers to an entity (e.g., for example, a lipid, metal, nucleic acid, polypeptide, polysaccharide, small molecule, etc., or complex, combination, mixture or system [e.g., cell, tissue, organism] thereof), or phenomenon (e.g., heat, electric current or field, magnetic force or field, etc.).

***Alkyl*:** The term "alkyl", used alone or as part of a larger moiety, refers to a saturated, optionally substituted straight or branched chain or cyclic hydrocarbon group having (unless otherwise specified) 1-12, 1-10, 1-8, 1-6, 1-4, 1-3, or 1-2 carbon atoms (e.g., C₁-C₁₂, C₁-C₁₀, C₁-C₈, C₁-C₆, C₁-C₄, C₁-C₃, or C₁-C₂). Exemplary alkyl groups include methyl, ethyl, propyl (e.g., n-propyl, isopropyl), butyl (e.g., n-butyl, sec-butyl, isobutyl, t-butyl, etc.), pentyl, hexyl, and heptyl. The term "cycloalkyl" refers to an optionally substituted saturated ring system of about 3 to about 10 ring carbon atoms. Exemplary monocyclic cycloalkyl rings include cyclopropyl, cyclobutyl, cyclopentyl, cyclohexyl, and cycloheptyl.

***Alkylene*:** The term "alkylene" and "alkylenyl" are used interchangeably and refers to a bivalent alkyl group. In some aspects of the disclosure, "alkylene" is a bivalent straight or branched alkyl group. In some aspects of the disclosure, an "alkylene chain" is a polymethylene group, i.e., -(CH₂)ₙ-, wherein n is a positive integer, e.g., from 1 to 6, from 1 to 4, from 1 to 3, from 1 to 2, or from 2 to 3. An optionally substituted alkylene chain is a polymethylene group in which one or more methylene hydrogen atoms is optionally replaced with a substituent. Suitable substituents include those described below for a substituted aliphatic group and also include those described in the specification herein. It will be appreciated that two substituents of the alkylene group may be taken together to form a ring system. In certain aspects of the present disclosure, two substituents can be taken together to form a 3- to 7-membered ring. The substituents can be on the same or different atoms.

***Alkenyl*:** The term "alkenyl", used alone or as part of a larger moiety, refers to an optionally substituted straight or branched chain or cyclic hydrocarbon group having at least one double bond and having (unless otherwise specified) 2-12, 2-10, 2-8, 2-6, 2-4, or 2-3 carbon atoms(e.g., C₂-C₁₂, C₂-C₁₀, C₂-C₈, C₂-C₆, C₂-C₄, or C₂-C₃). Exemplary alkenyl groups include ethenyl, propenyl, butenyl, pentenyl, hexenyl, and heptenyl. The term "cycloalkenyl" refers to an optionally substituted non-aromatic monocyclic or multicyclic ring system containing at least one carboncarbon double bond and having about 3 to about 10 carbon atoms. Exemplary monocyclic cycloalkenyl rings include cyclopentenyl, cyclohexenyl, and cycloheptenyl.

***Antagonist*:** As used herein, the term "antagonist" may refer to an agent, or condition whose presence, level, degree, type, or form is associated with a decreased level or activity of a target. An antagonist may include an agent of any chemical class including, for example, small molecules, polypeptides, nucleic acids, carbohydrates, lipids, metals, and/or any other entity that shows the relevant inhibitory activity. In some aspects of the present disclosure, an antagonist may be a "direct antagonist" in that it binds directly to its target; in some aspects of the present disclosure, an antagonist may be an "indirect antagonist" in that it exerts its influence by means other than binding directly to its target; e.g., by interacting with a regulator of the target, so that the level or activity of the target is altered). In some aspects of the present disclosure, an "antagonist" may be referred to as an "inhibitor".

***Aryl:*** The term "aryl" refers to monocyclic and bicyclic ring systems having a total of five to fourteen ring members (e.g., C₅₋₁₄), wherein at least one ring in the system is aromatic and wherein each ring in the system contains three to seven ring members. The term "aryl" may be used interchangeably with the term "aryl ring". In certain aspects of the present disclosure, "aryl" refers to an aromatic ring system which includes, but not limited to, phenyl, biphenyl, naphthyl, anthracyl and the like, which may bear one or more substituents. Unless otherwise specified, "aryl" groups are hydrocarbons.

***Associated*:** Two events or entities are "associated" with one another, as that term is used herein, if the presence, level, degree, type and/or form of one is correlated with that of the other. For example, a particular entity (e.g., polypeptide, genetic signature, metabolite, microbe, etc) is considered to be associated with a particular disease, disorder, or condition, if its presence, level and/or form correlates with incidence of and/or susceptibility to the disease, disorder, or condition (e.g., across a relevant population). In some aspects of the present disclosure, two or more entities are physically "associated" with one another if they interact, directly or indirectly, so that they are and/or remain in physical proximity with one another. In some aspects of the present disclosure, two or more entities that are physically associated with one another are covalently linked to one another; in some aspects of the present disclosure, two or more entities that are physically associated with one another are not covalently linked to one another but are non-covalently associated, for example by means of hydrogen bonds, van der Waals interaction, hydrophobic interactions, magnetism, and combinations thereof.

***Biological Sample*:** As used herein, the term "biological sample" typically refers to a sample obtained or derived from a biological source (e.g., a tissue or organism or cell culture) of interest, as described herein. In some aspects of the present disclosure, a source of interest comprises an organism, such as an animal or human. In some aspects of the disclosure, a biological sample is or comprises biological tissue or fluid. In some aspects of the present disclosure, a biological sample may be or comprise bone marrow; blood; blood cells; ascites; tissue or fine needle biopsy samples; cell-containing body fluids; free floating nucleic acids; sputum; saliva; urine; cerebrospinal fluid, peritoneal fluid; pleural fluid; feces; lymph; gynecological fluids; skin swabs; vaginal swabs; oral swabs; nasal swabs; washings or lavages such as a ductal lavages or broncheoalveolar lavages; aspirates; scrapings; bone marrow specimens; tissue biopsy specimens; surgical specimens; feces, other body fluids, secretions, and/or excretions; and/or cells therefrom, etc. In some aspects of the present disclosure, a biological sample is or comprises cells obtained from an individual. In some aspects of the present disclosure, obtained cells are or include cells from an individual from whom the sample is obtained. In some aspects of the present disclosure, a sample is a "primary sample" obtained directly from a source of interest by any appropriate means. For example, in some aspects of the present disclosure, a primary biological sample is obtained by methods selected from the group consisting of biopsy (e.g., fine needle aspiration or tissue biopsy), surgery, collection of body fluid (e.g., blood, lymph, feces etc.), etc. In some aspects of the present disclosure, as will be clear from context, the term "sample" refers to a preparation that is obtained by processing (e.g., by removing one or more components of and/or by adding one or more agents to) a primary sample. For example, filtering using a semi-permeable membrane. Such a "processed sample" may comprise, for example nucleic acids or proteins extracted from a sample or obtained by subjecting a primary sample to techniques such as amplification or reverse transcription of mRNA, isolation and/or purification of certain components, etc.

***Combination therapy*:** As used herein, the term "combination therapy" refers to those situations in which a subject is simultaneously exposed to two or more therapeutic regimens (e.g., two or more therapeutic agents). In some aspects of the present disclosure, the two or more regimens may be administered simultaneously; in some aspects of the present disclosure, such regimens may be administered sequentially (e.g., all "doses" of a first regimen are administered prior to administration of any doses of a second regimen); in some aspects of the present disclosure, such agents are administered in overlapping dosing regimens. In some aspects of the present disclosure, "administration" of combination therapy may involve administration of one or more agent(s) or modality(ies) to a subject receiving the other agent(s) or modality(ies) in the combination. For clarity, combination therapy does not require that individual agents be administered together in a single composition (or even necessarily at the same time), although in some aspects of the present disclosure, two or more agents, or active moieties thereof, may be administered together in a combination composition, or even in a combination compound (e.g., as part of a single chemical complex or covalent entity).

***Dosage form or unit dosage form*:** Those skilled in the art will appreciate that the term "dosage form" may be used to refer to a physically discrete unit of an active agent (e.g., a therapeutic or diagnostic agent) for administration to a subject. Typically, each such unit contains a predetermined quantity of active agent. In some aspects of the present disclosure, such quantity is a unit dosage amount (or a whole fraction thereof) appropriate for administration in accordance with a dosing regimen that has been determined to correlate with a desired or beneficial outcome when administered to a relevant population (i.e., with a therapeutic dosing regimen). Those of ordinary skill in the art appreciate that the total amount of a therapeutic composition or agent administered to a particular subject is determined by one or more attending physicians and may involve administration of multiple dosage forms.

***Dosing regimen or therapeutic regimen*:** Those skilled in the art will appreciate that the terms "dosing regimen" and "therapeutic regimen" may be used to refer to a set of unit doses (typically more than one) that are administered individually to a subject, typically separated by periods of time. In some aspects of the present disclosure, a given therapeutic agent has a recommended dosing regimen, which may involve one or more doses. In some aspects of the present disclosure, a dosing regimen comprises a plurality of doses each of which is separated in time from other doses. In some aspects of the present disclosure, individual doses are separated from one another by a time period of the same length; in some aspects of the present disclosure, a dosing regimen comprises a plurality of doses and at least two different time periods separating individual doses. In some aspects of the present disclosure, all doses within a dosing regimen are of the same unit dose amount. In some aspects of the present disclosure, different doses within a dosing regimen are of different amounts. In some aspects of the present disclosure, a dosing regimen comprises a first dose in a first dose amount, followed by one or more additional doses in a second dose amount different from the first dose amount. In some aspects of the present disclosure, a dosing regimen comprises a first dose in a first dose amount, followed by one or more additional doses in a second dose amount same as the first dose amount. In some aspects of the present disclosure, a dosing regimen is correlated with a desired or beneficial outcome when administered across a relevant population (i.e., is a therapeutic dosing regimen).

***Excipient*:** As used herein, the term "excipient" refers to a non-therapeutic agent that may be included in a pharmaceutical composition, for example, to provide or contribute to a desired consistency or stabilizing effect. Suitable pharmaceutical excipients include, for example, starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like.

***Halo or halogen*:** As used herein, the term "halogen" or "halo" to fluorine, chlorine, bromine and iodine. "Halo" can modify another group to indicate optional substitution of a hydrogen atom with a halogen atom. For example, as used herein, a "haloalkyl" is a branched, straight-chain or cyclic alkyl group, substituted with 1 or more halogen atoms (e.g., "C₁-C₆ haloalkyl" groups have from 1 to 6 carbon atoms, wherein one or more hydrogen atoms are replaced with a halogen atom). Examples of haloalkyl groups include, but are not limited to, mono-, di- or tri-fluoromethyl; -CH₂-CH₂-fluoro, -CH₂-CH₂-CH₂-fluoro, -CH₂-CH₂-CH₂-CH₂- fluoro, and -CH₂-CF₃, -CH₂-C(CH₃)₂-F.

***Heteroaryl:*** The terms "heteroaryl" and "heteroar-", used alone or as part of a larger moiety, e.g., "heteroaralkyl", or "heteroaralkoxy", refer to monocyclic or bicyclic ring groups having 5 to 10 ring atoms (e.g., 5- to 6- membered monocyclic heteroaryl or 9- to 10-membered bicyclic heteroaryl); having 6, 10, or 14 π 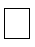 electrons shared in a cyclic array; and having, in addition to carbon atoms, from one to five heteroatoms. The term "heteroatom" refers to nitrogen, oxygen, or sulfur, and includes any oxidized form of nitrogen or sulfur, and any quaternized form of a basic nitrogen. Heteroaryl groups include, without limitation, thienyl, furanyl, pyrrolyl, imidazolyl, pyrazolyl, triazolyl, tetrazolyl, oxazolyl, isoxazolyl, oxadiazolyl, thiazolyl, isothiazolyl, thiadiazolyl, pyridyl, pyridazinyl, pyrimidinyl, pyrazinyl, indolizinyl, purinyl, naphthyridinyl, pteridinyl, imidazo[1,2-a]pyrimidinyl, imidazo[1,2-a]pyridinyl, thienopyrimidinyl, triazolopyridinyl, and benzoisoxazolyl. The terms "heteroaryl" and "heteroar-", as used herein, also include groups in which a heteroaromatic ring is fused to one or more aryl, cycloaliphatic, or heterocyclyl rings, where the radical or point of attachment is on the heteroaromatic ring (i.e., a bicyclic heteroaryl ring having 1 to 3 heteroatoms). Nonlimiting examples include indolyl, isoindolyl, benzothienyl, benzofuranyl, dibenzofuranyl, indazolyl, benzimidazolyl, benzothiazolyl, benzothiadiazolyl, benzoxazolyl, quinolyl, isoquinolyl, cinnolinyl, phthalazinyl, quinazolinyl, quinoxalinyl, 4*H*-quinolizinyl, carbazolyl, acridinyl, phenazinyl, phenothiazinyl, phenoxazinyl, tetrahydroquinolinyl, tetrahydroisoquinolinyl, pyrido[2,3-b]-1,4-oxazin-3(4H)-one, and benzoisoxazolyl. A heteroaryl group may be mono- or bicyclic. The term "heteroaryl" may be used interchangeably with the terms "heteroaryl ring", "heteroaryl group", or "heteroaromatic", any of which terms include rings that are optionally substituted. The term "heteroaralkyl" refers to an alkyl group substituted by a heteroaryl, wherein the alkyl and heteroaryl portions independently are optionally substituted.

***Heteroatom:*** The term "heteroatom" as used herein refers to nitrogen, oxygen, or sulfur, and includes any oxidized form of nitrogen or sulfur, and any quaternized form of a basic nitrogen.

***Heterocycle:*** As used herein, the terms "heterocycle", "heterocyclyl", "heterocyclic radical", and "heterocyclic ring" are used interchangeably and refer to a stable 3- to 8-membered monocyclic or 7- to 10-membered bicyclic heterocyclic moiety that is either saturated or partially unsaturated, and having, in addition to carbon atoms, one or more, such as one to four, heteroatoms, as defined above. When used in reference to a ring atom of a heterocycle, the term "nitrogen" includes a substituted nitrogen. As an example, in a saturated or partially unsaturated ring having 0-3 heteroatoms selected from oxygen, sulfur or nitrogen, the nitrogen may be N (as in 3,4-dihydro-2H-pyrrolyl), NH (as in pyrrolidinyl), or NR⁺ (as in N-substituted pyrrolidinyl). A heterocyclic ring can be attached to its pendant group at any heteroatom or carbon atom that results in a stable structure and any of the ring atoms can be optionally substituted. Examples of such saturated or partially unsaturated heterocyclic radicals include, without limitation, tetrahydrofuranyl, tetrahydrothienyl, piperidinyl, decahydroquinolinyl, oxazolidinyl, piperazinyl, dioxanyl, dioxolanyl, diazepinyl, oxazepinyl, thiazepinyl, morpholinyl, and thiamorpholinyl. A heterocyclyl group may be mono-, bi-, tri-, or polycyclic, preferably mono-, bi-, or tricyclic, more preferably mono- or bicyclic. The term "heterocyclylalkyl" refers to an alkyl group substituted by a heterocyclyl, wherein the alkyl and heterocyclyl portions independently are optionally substituted. A bicyclic heterocyclic ring also includes groups in which the heterocyclic ring is fused to one or more aryl rings. Exemplary bicyclic heterocyclic groups include indolinyl, isoindolinyl, benzodioxolyl, 1,3-dihydroisobenzofuranyl, 2,3-dihydrobenzofuranyl, tetrahydroquinolinyl, and 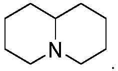 A bicyclic heterocyclic ring can also be a spirocyclic ring system (e.g., 7- to 11-membered spirocyclic fused heterocyclic ring having, in addition to carbon atoms, one or more heteroatoms as defined above (e.g., one, two, three or four heteroatoms)).

***Oral*:** The phrases "oral administration" and "administered orally" as used herein have their art-understood meaning referring to administration by mouth of a compound or composition.

***Parenteral*:** The phrases "parenteral administration" and "administered parenterally" as used herein have their art-understood meaning referring to modes of administration other than enteral and topical administration, usually by injection, and include, without limitation, intravenous, intramuscular, intra-arterial, intrathecal, intracapsular, intraorbital, intracardiac, intradermal, intraperitoneal, transtracheal, subcutaneous, subcuticular, intraarticulare, subcapsular, subarachnoid, intraspinal, and intrasternal injection and infusion.

***Patient or subject*:** As used herein, the term "patient" or "subject" refers to any organism to which a provided composition is or may be administered, e.g., for experimental, diagnostic, prophylactic, cosmetic, and/or therapeutic purposes. Typical patients or subjects include animals (e.g., mammals such as mice, rats, rabbits, non-human primates, and/or humans). In some aspects of the present disclosure, a patient is a human. In some aspects of the present disclosure, a patient or a subject is suffering from or susceptible to one or more disorders or conditions. In some aspects of the present disclosure, a patient or subject displays one or more symptoms of a disorder or condition. In some aspects of the present disclosure, a patient or subject has been diagnosed with one or more disorders or conditions. In some aspects of the present disclosure, a patient or a subject is receiving or has received certain therapy to diagnose and/or to treat a disease, disorder, or condition.

***Pharmaceutical composition*:** As used herein, the term "pharmaceutical composition" refers to an active agent, formulated together with one or more pharmaceutically acceptable carriers. In some aspects of the present disclosure, the active agent is present in unit dose amounts appropriate for administration in a therapeutic regimen to a relevant subject (e.g., in amounts that have been demonstrated to show a statistically significant probability of achieving a predetermined therapeutic effect when administered), or in a different, comparable subject (e.g., in a comparable subject or system that differs from the subject or system of interest in presence of one or more indicators of a particular disease, disorder or condition of interest, or in prior exposure to a condition or agent, etc.). In some aspects of the present disclosure, comparative terms refer to statistically relevant differences (e.g., that are of a prevalence and/or magnitude sufficient to achieve statistical relevance). Those skilled in the art will be aware, or will readily be able to determine, in a given context, a degree and/or prevalence of difference that is required or sufficient to achieve such statistical significance.

***Pharmaceutically acceptable carrier*:** As used herein, the term "pharmaceutically acceptable carrier" means a pharmaceutically-acceptable material, composition or vehicle, such as a liquid or solid filler, diluent, excipient, or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; pH buffered solutions; polyesters, polycarbonates and/or polyanhydrides; and other nontoxic compatible substances employed in pharmaceutical formulations.

***Pharmaceutically acceptable salt*:** The term "pharmaceutically acceptable salt", as used herein, refers to salts of such compounds that are appropriate for use in pharmaceutical contexts, i.e., salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge, et al. describes pharmaceutically acceptable salts in detail in J. Pharmaceutical Sciences, 66: 1-19 (1977). In some aspects of the disclosure, pharmaceutically acceptable salts include, but are not limited to, nontoxic acid addition salts, which are salts of an amino group formed with inorganic acids such as hydrochloric acid, hydrobromic acid, phosphoric acid, sulfuric acid and perchloric acid or with organic acids such as acetic acid, maleic acid, tartaric acid, citric acid, succinic acid or malonic acid or by using other methods used in the art such as ion exchange. In some aspects of the disclosure, pharmaceutically acceptable salts include, but are not limited to, adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Representative alkali or alkaline earth metal salts include sodium, lithium, potassium, calcium, magnesium, and the like. In some aspects of the disclosure, pharmaceutically acceptable salts include, when appropriate, nontoxic ammonium, quaternary ammonium, and amine cations formed using counterions such as halide, hydroxide, carboxylate, sulfate, phosphate, nitrate, alkyl having from 1 to 6 carbon atoms, sulfonate and aryl sulfonate.

***Substituted or optionally substituted*:** As described herein, compounds of the invention may contain "optionally substituted" moieties. In general, the term "substituted," whether preceded by the term "optionally" or not, means that one or more hydrogens of the designated moiety are replaced with a suitable substituent. "Substituted" applies to one or more hydrogens that are either explicit or implicit from the structure (e.g., 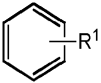 refers to at least 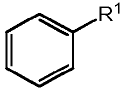 ; and 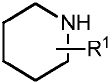 refers to at least 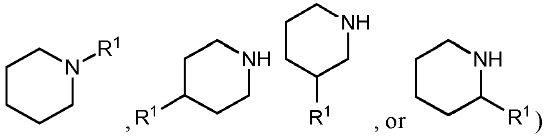 . Unless otherwise indicated, an "optionally substituted" group may have a suitable substituent at each substitutable position of the group, and when more than one position in any given structure may be substituted with more than one substituent selected from a specified group, the substituent may be either the same or different at every position. Combinations of substituents envisioned by this invention are preferably those that result in the formation of stable or chemically feasible compounds. The term "stable," as used herein, refers to compounds that are not substantially altered when subjected to conditions to allow for their production, detection, and, in certain aspects of the disclosure, their recovery, purification, and use for one or more of the purposes provided herein. Groups described as being "substituted" preferably have between 1 and 4 substituents, more preferably 1 or 2 substituents. Groups described as being "optionally substituted" may be unsubstituted or be "substituted" as described above.

Suitable monovalent substituents on a substitutable carbon atom of an "optionally substituted" group are independently halogen; -(CH2)0-4R°; -(CH2)0-4OR°; -O(CH2)0-4R°, - O-(CH2)0-4C(O)OR°; -(CH2)0-4CH(OR)2°; -(CH2)0-4SR°; -(CH2)0-4Ph, which may be substituted with R°; -(CH2)0-40(CH2)0-1Ph which may be substituted with R°; -CH=CHPh, which may be substituted with R°; -(CH2)0-40(CH2)0-1-pyridyl which may be substituted with R°; -NO2; -CN; -N3; -(CH2)0-4N(R°)2; -(CH2)0-4N(R°)C(O)R°; -N(R°)C(S)R°; -(CH2)0-4N(R°)C(O)NR°2; -N(R°)C(S)NR°2; -(CH2)0-4N(R°)C(O)OR°; - N(R°)N(R°)C(O)R°; -N(R°)N(R°)C(O)NR°2; -N(R°)N(R°)C(O)OR°; -(CH2)0-4C(O)R°; C(S)R°; -(CH2)0-4C(O)OR°; -(CH2)0-4C(O)SR°; -(CH2)0-4C(O)OSiR°3; -(CH2)0-4OC(O)R°; -OC(O)(CH2)0-4SR°; -(CH2)0-4SC(O)R°; -(CH2)0-4C(O)NR°2; -C(S)NR°2; - C(S)SR°; -SC(S)SR°, -(CH2)0-4OC(O)NR°2; -C(O)N(OR°)R°; -C(O)C(O)R°; - C(O)CH2C(O)R°; -C(NOR°)R°; -(CH2)0-4SSR°; -(CH2)0-4S(O)2R°; -(CH2)0-4S(O)2OR°; - (CH2)0-4OS(O)2R°; -S(O)2NR°2; -(CH2)0-4S(O)R°; -N(R°)S(O)2NR°2; -N(R°)S(O)2R°; - N(OR°)R°; -C(NH)NR°2; -P(O)2R°; -P(O)R°2; -OP(O)R°2; -OP(O)(OR°)2; SiR°3; -(C1-4 straight or branched alkylene)O-N(R°)2; or -(C1-4 straight or branched alkylene)C(O)O-N(R°)2, wherein each R° may be substituted as defined below and is independently hydrogen, C1-6 aliphatic, -CH2Ph, -O(CH2)0-1Ph, -CH2-(5- to 6-membered heteroaryl ring), or a 3- to 6-membered saturated, partially unsaturated, or aryl ring having 0-4 heteroatoms independently selected from nitrogen, oxygen, or sulfur, or, notwithstanding the definition above, two independent occurrences of R°, taken together with their intervening atom(s), form a 3- to 12-membered saturated, partially unsaturated, or aryl mono- or bicyclic ring having 0-4 heteroatoms independently selected from nitrogen, oxygen, or sulfur, which may be substituted as defined below.

Suitable monovalent substituents on R° (or the ring formed by taking two independent occurrences of R° together with their intervening atoms), are independently halogen, -(CH2)0-2R●, -(halo●), -(CH2)0-2OH, -(CH2)0-2OR●, -(CH2)0-2CH(OR●)2, -O(haloR●), -CN, - N3, -(CH2)0-2C(O)R●, -(CH2)0-2C(O)OH, ((CH2)0-2C(O)OR●, -(CH2)0-2SR●, -(CH2)0-2SH, -(CH2)0-2NH2, -(CH2)0-2NHR●, -(CH2)0-2NR●2, -NO2, -SiR●3, - OSiR●3, -C(O)SR●, (C1-4 straight or branched alkylene)C(O)OR●, or -SSR● wherein each R● is unsubstituted or where preceded by "halo" is substituted only with one or more halogens, and is independently selected from C1-4 aliphatic, -CH2Ph, -O(CH2)0-1Ph, or a 3- to 6-membered saturated, partially unsaturated, or aryl ring having 0-4 heteroatoms independently selected from nitrogen, oxygen, or sulfur. Suitable divalent substituents on a saturated carbon atom of R° include =O and =S.

Suitable divalent substituents on a saturated carbon atom of an "optionally substituted" group include the following: =O ("oxo"), =S, =NNR*2, =NNHC(O)R*, =NNHC(O)OR*, =NNHS(O)2R*, =NR*, =NOR*, -O(C(R*2))2-3O-, or -S(C(R*2))2-3S-, wherein each independent occurrence of R* is selected from hydrogen, C1-6 aliphatic which may be substituted as defined below, or an unsubstituted 5- to 6-membered saturated, partially unsaturated, or aryl ring having 0-4 heteroatoms independently selected from nitrogen, oxygen, or sulfur. Suitable divalent substituents that are bound to vicinal substitutable carbons of an "optionally substituted" group include: -O(CR*2)2-3O-, wherein each independent occurrence of R* is selected from hydrogen, C1-6 aliphatic which may be substituted as defined below, or an unsubstituted 5-6-membered saturated, partially unsaturated, or aryl ring having 0-4 heteroatoms independently selected from nitrogen, oxygen, or sulfur.

Suitable substituents on the aliphatic group of R* include halogen, -R●, -(haloR●), -OH, -OR●, -O(haloR●), -CN, -C(O)OH, -C(O)OR●, -NH2, -NHR●, -NR●2, or -NO2, wherein each R● is unsubstituted or where preceded by "halo" is substituted only with one or more halogens, and is independently C1-4 aliphatic, -CH2Ph, -O(CH2)0-1Ph, or a 5- to 6-membered saturated, partially unsaturated, or aryl ring having 0-4 heteroatoms independently selected from nitrogen, oxygen, or sulfur.

Suitable substituents on a substitutable nitrogen of an "optionally substituted" group include -R†, -NR†2, -C(O)R†, -C(O)OR†, -C(O)C(O)R†, - C(O)CH2C(O)R†, -S(O)2R†, -S(O)2NR†2, -C(S)NR†2, -C(NH)NR†2, or -N(R†)S(O)2R†; wherein each R† is independently hydrogen, C1-6 aliphatic which may be substituted as defined below, unsubstituted -OPh, or an unsubstituted 3- to 6-membered saturated, partially unsaturated, or aryl ring having 0-4 heteroatoms independently selected from nitrogen, oxygen, or sulfur, or, notwithstanding the definition above, two independent occurrences of R†, taken together with their intervening atom(s) form an unsubstituted 3- to 12-membered saturated, partially unsaturated, or aryl mono- or bicyclic ring having 0-4 heteroatoms independently selected from nitrogen, oxygen, or sulfur.

Suitable substituents on the aliphatic group of R† are independently halogen, - R●, -(haloR●), -OH, -OR●, -O(haloR●), -CN, -C(O)OH, -C(O)OR●, -NH2, -NHR●, - NR●2, or -NO2, wherein each R● is unsubstituted or where preceded by "halo" is substituted only with one or more halogens, and is independently C1-4 aliphatic, -CH2Ph, -O(CH2)0-1Ph, or a 3- to 6-membered saturated, partially unsaturated, or aryl ring having 0-4 heteroatoms independently selected from nitrogen, oxygen, or sulfur.

***Small molecule:*** As used herein, the term "small molecule" means a low molecular weight organic and/or inorganic compound. In general, a "small molecule" is a molecule that is less than about 5 kilodaltons (kD) in size. In some aspects of the present disclosure, a small molecule is less than about 4 kD, 3 kD, about 2 kD, or about 1 kD. In some aspects of the present disclosure, the small molecule is less than about 800 daltons (D), about 600 D, about 500 D, about 400 D, about 300 D, about 200 D, or about 100 D. In some aspects of the present disclosure, a small molecule is less than about 2000 g/mol, less than about 1500 g/mol, less than about 1000 g/mol, less than about 800 g/mol, or less than about 500 g/mol. In some aspects of the present disclosure, a small molecule is not a polymer.

In some aspects of the present disclosure a small molecule does not include a polymeric moiety. In some aspects of the present disclosure, a small molecule is not and/or does not comprise a protein or polypeptide (e.g., is not an oligopeptide or peptide). In some aspects of the present disclosure, a small molecule is not and/or does not comprise a polynucleotide (e.g., is not an oligonucleotide). In some aspects of the present disclosure, a small molecule is not and/or does not comprise a polysaccharide; for example, in some aspects of the present disclosure, a small molecule is not a glycoprotein, proteoglycan, glycolipid, etc.). In some aspects of the present disclosure, a small molecule is not a lipid.

In some aspects of the present disclosure, a small molecule is a modulating agent (e.g., is an inhibiting agent or an activating agent). In some aspects of the present disclosure, a small molecule is biologically active. In some aspects of the present disclosure, a small molecule is detectable (e.g., comprises at least one detectable moiety). In some aspects of the present disclosure, a small molecule is a therapeutic agent.

Those of ordinary skill in the art, reading the present disclosure, will appreciate that certain small molecule compounds described herein may be provided and/or utilized in any of a variety of forms such as, for example, crystal forms (e.g., polymorphs, solvates, etc), salt forms, protected forms, pro-drug forms, ester forms, isomeric forms (e.g., optical and/or structural isomers), isotopic forms, etc.

Those of ordinary skill in the art will appreciate that certain small molecule compounds have structures that can exist in one or more steroisomeric forms. In some aspects of the present disclosure, such a small molecule may be utilized in accordance with the present disclosure in the form of an individual enantiomer, diastereomer or geometric isomer, or may be in the form of a mixture of stereoisomers; in some aspects of the present disclosure, such a small molecule may be utilized in accordance with the present disclosure in a racemic mixture form.

Those of skill in the art will appreciate that certain small molecule compounds have structures that can exist in one or more tautomeric forms. In some aspects of the present disclosure, such a small molecule may be utilized in accordance with the present disclosure in the form of an individual tautomer, or in a form that interconverts between tautomeric forms.

Those of skill in the art will appreciate that certain small molecule compounds have structures that permit isotopic substitution (e.g., 2H or 3H for H;, 11C, 13C or 14C for 12C; , 13N or 15N for 14N; 170 or 180 for 160; 36Cl for XXC; 18F for XXF; 131I for XXXI; etc). In some aspects of the present disclosure, such a small molecule may be utilized in accordance with the present disclosure in one or more isotopically modified forms, or mixtures thereof.

In some aspects of the present disclosure, reference to a particular small molecule compound may relate to a specific form of that compound. In some aspects of the present disclosure, a particular small molecule compound may be provided and/or utilized in a salt form (e.g., in an acid-addition or base-addition salt form, depending on the compound); in some such aspects of the present disclosure, the salt form may be a pharmaceutically acceptable salt form.

In some aspects of the present disclosure, where a small molecule compound is one that exists or is found in nature, that compound may be provided and/or utilized in accordance in the present disclosure in a form different from that in which it exists or is found in nature. Those of ordinary skill in the art will appreciate that, in some aspects of the present disclosure, a preparation of a particular small molecule compound that contains an absolute or relative amount of the compound, or of a particular form thereof, that is different from the absolute or relative (with respect to another component of the preparation including, for example, another form of the compound) amount of the compound or form that is present in a reference preparation of interest (e.g., in a primary sample from a source of interest such as a biological or environmental source) is distinct from the compound as it exists in the reference preparation or source. Thus, in some aspects of the present disclosure, for example, a preparation of a single stereoisomer of a small molecule compound may be considered to be a different form of the compound than a racemic mixture of the compound; a particular salt of a small molecule compound may be considered to be a different form from another salt form of the compound; a preparation that contains only a form of the compound that contains one conformational isomer ((Z) or (E)) of a double bond may be considered to be a different form of the compound from one that contains the other conformational isomer ((E) or (Z)) of the double bond; a preparation in which one or more atoms is a different isotope than is present in a reference preparation may be considered to be a different form; etc.

***Therapeutic agent:*** As used herein, the phrase "therapeutic agent" in general refers to any agent that elicits a desired pharmacological effect when administered to an organism. In some aspects of the present disclosure, an agent is considered to be a therapeutic agent if it demonstrates a statistically significant effect across an appropriate population. In some aspects of the present disclosure, the appropriate population may be a population of model organisms. In some aspects of the present disclosure, an appropriate population may be defined by various criteria, such as a certain age group, gender, genetic background, preexisting clinical conditions, etc. In some aspects of the present disclosure, a therapeutic agent is a substance that can be used to alleviate, ameliorate, relieve, inhibit, prevent, delay onset of, reduce severity of, and/or reduce incidence of one or more symptoms or features of a disease, disorder, and/or condition. In some aspects of the present disclosure, a "therapeutic agent" is an agent that has been or is required to be approved by a government agency before it can be marketed for administration to humans. In some aspects of the present disclosure, a "therapeutic agent" is an agent for which a medical prescription is required for administration to humans.

***Treat*:** As used herein, the terms "treat," "treatment," or "treating" refer to any method used to partially or completely alleviate, ameliorate, relieve, inhibit, prevent, delay onset of, reduce severity of, and/or reduce incidence of one or more symptoms or features of a disease, disorder, and/or condition. Treatment may be administered to a subject who does not exhibit signs of a disease, disorder, and/or condition. In some aspects of the present disclosure, treatment may be administered to a subject who exhibits only early signs of the disease, disorder, and/or condition, for example, for the purpose of decreasing the risk of developing pathology associated with the disease, disorder, and/or condition.

***Therapeutically effective amount:*** As used herein, the term "therapeutically effective amount" refers to an amount of a substance (e.g., a therapeutic agent, composition, and/or formulation) that elicits a desired biological response when administered as part of a therapeutic regimen. In some aspects of the present disclosure, a therapeutically effective amount of a substance is an amount that is sufficient, when administered to a subject suffering from or susceptible to a disease, disorder, and/or condition, to treat, diagnose, prevent, and/or delay the onset of the disease, disorder, and/or condition. As will be appreciated by those of ordinary skill in this art, the effective amount of a substance may vary depending on such factors as the desired biological endpoint, the substance to be delivered, the target cell or tissue, etc. For example, the effective amount of compound in a formulation to treat a disease, disorder, and/or condition is the amount that alleviates, ameliorates, relieves, inhibits, prevents, delays onset of, reduces severity of and/or reduces incidence of one or more symptoms or features of the disease, disorder and/or condition. In some aspects of the present disclosure, a therapeutically effective amount is administered in a single dose; in some aspects of the present disclosure, multiple unit doses are required to deliver a therapeutically effective amount.

The symbol 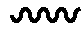 , as used herein, refers to a point of attachment between two atoms.

It is understood by one skilled in the art that compounds referred to herein may be enriched at any or all atoms above naturally occurring isotopic ratios with one or more isotopes such as, but not limited to, deuterium (²H or D).

The compounds of the invention, or their pharmaceutically acceptable salts, may contain chiral centers, which, unless specified otherwise, may be either of the (R) or (S) configuration, or which may comprise a mixture thereof. Accordingly, the present application includes stereoisomers of the compounds described herein, where applicable, either individually or admixed in any proportions. Stereoisomers may include, but are not limited to, enantiomers, diastereomers, racemic mixtures, and combinations thereof. Such stereoisomers can be prepared and separated using conventional techniques, either by reacting enantiomeric starting materials, or by separating isomers of compounds of the present application.

### Estrogen Receptor-Associated Diseases and Disorders

The estrogen receptor ("ER") is involved in a variety of biological processes, relating, for example, to development of the female reproductive system, maintenance of bone mass, protection of cardiovascular and/or central nervous system components, etc. (see, for example, Pearce & Jordan Crit. Rev. Onc/Hem 50:3, 2004; Heldring Phys. Rev. 87:905, 2007). The ER has been implicated in a variety of cancers. In many tumors that express the estrogen receptor (i.e., ER⁺ tumors), active ERα signaling has been demonstrated to drive cell proliferation (although ERβ signaling has been reported to be able to achieve tumor suppressor effects; see, for example, Nilsson & Gustafson Clin. Pharmacol. Ther. 89:44, 2011). Typically, tumors (e.g., breast tumors) with as few as 1% of cells staining positive for ER are classified as "ER⁺". Therapies targeting the ER are standard of care for many patients with ER⁺ tumors (see, for example, Cardoso et al Annals Onc. https://doi.org/10.1093/announc/mdmx036, 2017; Rugo et al. J. Clin. Oncol. 34:3069, 2016; Senkus et al Annal Onc. 26:v8, 2015; Sareddy & Vadlamudi Clin. J Nat. Med, 13:801, 2015). For early stage breast cancer patients, for example, recommended therapy typically involves tumor resection, followed by ER-targeted therapy (e.g., as discussed below). For advanced breast cancer, including metastatic breast cancer, ER-targeted therapy is the mainstay.

Given the importance of ER signaling in many cancers, as well as in certain cardiovascular, inflammatory, and neurodegenerative diseases, significant effort has been invested in developing therapeutic agents and modalities that target the ER. There is some fluidity/flexibility in terminology that has been used to describe ER-targeting agents, but a variety of agents, with different mechanisms, have been developed and/or studied.

Some ER-targeting agents are designed and/or documented to reduce levels of estrogen (i.e., 17β estradiol) production.

Some ER-targeting agents are designed and/or documented to bind directly to the ER; in some cases, such agents compete with estrogen for binding to the ER and/or interfere with the allosteric changes that estrogen binding would naturally produce. Often, the term "antiestrogen" is used to refer to agents that bind to the ER, and sometimes is specifically used to indicate those agents that compete with estrogen for ER binding.

The term "selective estrogen receptor modulator, "SERM", has been used to refer to compounds that are designed and/or documented to alter some aspect of ER activity. Some writings refer to "SERMs" as representing a particular type of anti-estrogens; other writings, however, use the term "SERM" more generally, to refer to a compound that specifically impacts some feature of ER (particularly ERα) expression and/or activity.

The term "selective estrogen receptor degrader" ("SERD") has been used to refer to compounds that are designed and/or documented to trigger or enhance degradation of the ER. In many instances, if presence of a compound correlates with reduced level of ER, the compound may be referred to as a SERD. Some writings classify compounds *either* as SERMs *or* as SERDs; others refer to SERDs as a particular type, or *species,* of compounds that are SERMs.

Regardless of mechanism of action of a particular agent, clinical experience thus far has revealed that incomplete effects (e.g., within an individual patient and/or across patient populations) and/or development of resistance remain a problem.

Among other things, presence or development of certain ER mutations has been reported to impact effectiveness of various ER-targeted therapies (see, for example, Jeselsohn et al Nature Rev. Clin. Onc. 12, 573, 2015; Gelsomino et al. Breast Cancer Res. Treat 157:253, 2016; Toy *et al.* 2013 ). Some particularly problematic mutations are those that "activate" one or more aspects of ER expression and/or function; some activating mutations have been reported that can render the ER ligand-independent (*i.e.,* constitutively active). For example, particular mutations in the ER ligand binding domain, including D538G and Y537S, have been demonstrated to constitutively activate the ER; other mutations including deletions and/or fusions that remove the ligand binding domain, can have similar effects (see, for example, Li et al. Cell Repts 4:1116, 2013; Veeraraghavan et al Breast Cancer Research and Treatment 158, 219-232, 2016; Veeraraghavan, et al. Nature Comms 5:4577, 2014). Some reports have indicated that as many as 50% of women with metastatic breast cancer may have activating ER mutations detectible in circulating tumor DNA.

Brain metastases occurs in about 10 to 16% of breast cancer patients, the second most common cause of brain metastases after lung cancer. Leone, Exp Hematol Oncol 4, 33 (2015) do1:10.1186/s40164-015-0028-8. The prognosis is poor, with overall survival from diagnosis ranging from a few months to a few years. Frisk, et al., Breast Cancer Res. Treat. 166:887-896

(2017). In particular, for hormone positive disease e.g., ER-positive breast cancer, the incidence of breast cancer brain metastases (BCBMs) is 14%, with a median overall survival after the development of brain metastases of 9-10 months. Brosnan & Anders, Ann Transl Med., 2016;6(9):163. There are no FDA-approved therapies for treating breast cancer brain metastasis, only surgery and radiotherapy, including whole brain radiotherapy.

The challenge of treating breast cancer brain metastases is providing a therapy that is capable of crossing the blood brain barrier. As noted by Brosnan & Anders, the BBB exists to selectively regulate what enters the brain and protects it from toxic substances, including chemotherapeutics and targeted drugs. In particular, "the unpredictable nature and heterogeneity in permeability of the BBB, in addition to inherent drug efflux pumps, makes it challenging to effectively deliver drugs in sufficient quantity to brain metastases to achieve apoptosis." Brosnan & Anders, Ann Transl Med., 2016;6(9):163.

The present disclosure establishes certain classes of compounds that are complete estrogen receptor antagonists that are capable of crossing the blood brain barrier, thereby providing a viable mode of treatment of breast cancer brain metastases.

### Estrogen Receptor Antagonists

Enormous investment has been, and continues to be, made in the pursuit of effective therapies that target the ER (reviewed, for example, in Patel & Bihani Pharmacol. & Therap. 186:1, 2018*).*

Among the most advanced compounds under clinical development are:
a. Tamoxifen, which has been an important breast cancer therapeutic, credited with having "saved the lives of half a million women around the world" (see "Bringing the Investigational Breast Cancer Drug Endoxifen From Bench to Bedside with NCI Support, *available at* https://www.cancer.gov/news-events/cancer-currents-blog/2017/endoxifen-breast-cancer-NCI-support (last accessed July 7, 2019), but known to be less effective in women with low CYP2D6 activity, and also susceptible to development of resistance.
b. Endoxifen, the active metabolite of Tamoxifen, originally developed to address Tamoxifen's failure in women with low CYP2D6 activity, which reduces their ability to convert Tamoxifen into Endoxifen. (see Cancer Currents Blog, National Cancer Institute, Aug 31, 2017)
c. ARN-810 (Brilanestrant; GDC-810), which has been described as "a novel, potent, non-steroidal, orally bioavailable, selective ER antagonist/ER degrader that induces tumor regression in tamoxifen-sensitive and resistant ER⁺ BC xenograft models" (see Dickler et al. Cancer Res. 75(15 Suppl): Abstract nr CT231, 2015), and which was carried into Phase II clinical trials for treatment of patients with ER⁺ bereast cancer who had failed other hormonal agents, but whose further development may subsequently have been dropped (see, for example, Biospace April 27, 2017)
d. AZD9496, which has been described as "an oral nonsteroidal, small-molecule inhibitor of estrogen receptor alpha (ERα) and a potent and selective antagonist and degrader of ERα" (see Hamilton et al Clin Cancer Res 1:3519, 2018); AZD9496 has been reported to "antagoni[ze] and degrad[e] ER with anti-tumour activity in both endocrine-sensitive and endocrine-resistant models", and has been described as "comparable to fulvestrant in antagonising ER and circumventing endocrine resistance" (see, Nardone et al. Br. J. Cancer 120:331, 2019),
e. RAD-1901 (Elacestrant) has been described as "a novel, non-steroidal oral SERD that has demonstrated single-agent activity in heavily pre-treated patients with ER⁺ advanced breast cancer (see de Vries et al, Cancer Res. Abstract P1-10-04, 2018; see also Bardia et al. J. Clin. Onc. 35:15_suppl, 1014, 2017). Pre-clinical studies have also reported that "Elacestrant significantly inhibited the growth of xenograft models harboring ESR1 mutations, including those harboring Y537S or D538G mutations and models that were insensitive to fulvestrant and tamoxifen" (see Patel et al. Cancer Res 79:Abstract nr P6-20-08, 2019).
f. Fulvestrant (Faslodex^{™}) was the first SERD to earn FDA approval, and has been approved for treatment of certain ER⁺ cancers, including in combination with palbociclib or abemaciclib. Fulvestrant is a "selective estrogen receptor degrader that binds, blocks and degrades the estrogen receptor (ER), leading to complete inhibition of estrogen signaling through the ER" (see Nathan & Schmid Oncol Ther 5:17, 2017). Fulvestrant has achieved significant clinical success, and is often considered to be the "gold standard" against which ER-targeted therapies are compared. However, Fulvestrant is administered by injection rather than orally, and in fact requires administration of 500 mg via intramuscular injection once per month (after initial dosing). Also, although certain retrospective analyses have offered hope that Fulvestrant might have some usefulness in treatment of patients with ER mutants, conclusive evidence of activity has not been achieved (see, for example, Fribbens et al. J Clin Oncol. 34:2961, 2916; Spoerke et al. Nat Commun 7:11579, 2016):

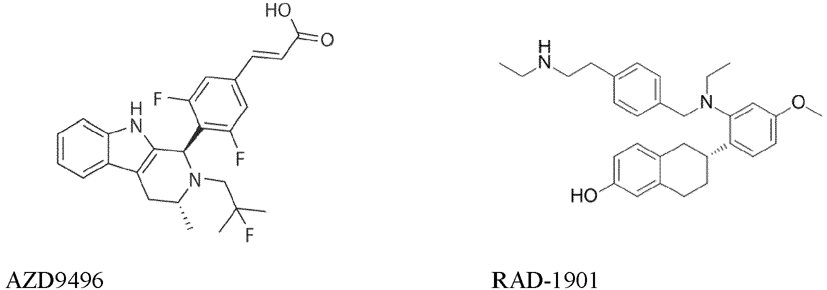

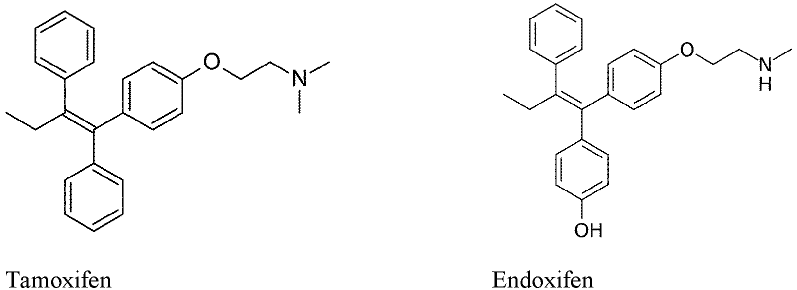

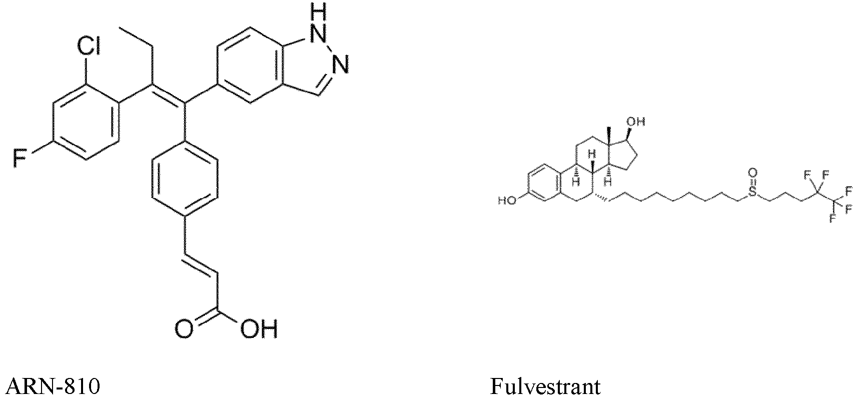

Fulvestrant remains the gold standard for treatment of ER-associated diseases and disorders for patients who have developed resistance to currently-approved therapies, such as tamoxifen and endoxifen. The present disclosure appreciates that fulvestrant's success stems from its ability to function as a *complete* estrogen receptor antagonist (a "CERAN") that (1) inhibits *both* AF1 and AF2, so that it can inhibit AF1 activity that remains present in constitutively active ER mutants; (2) promotes ER degradation; and (3) lacks the partial ER agonist activity observed with certain other agents. As compared, for example, with therapies that limit estrogen production (e.g., anastrozole) or with partial antagonists (e.g., tamoxifen), fulvestrant exhibits superior activity and is the preferred treatment option for patients with hormone receptor-positive locally advanced or metastatic breast cancer. *See* Robertson, et al., The Lancet, 388(10063):2997-3005 (Dec. 17, 2016). Without wishing to be bound by any particular theory, it is proposed that fulvestrant's ability to inhibit both AF1 and AF2 may be attributable to its recruitment of corepressors to the ER complex.

Regardless, the present disclosure further appreciates that many other compounds, including for example, ARN-810, AZD9496, tamoxifen, and others, are less effective than fulvestrant at least in part because they only partially antagonize ER, and specifically because they inhibit activation of AF2 but not AF1. Moreover, it is well known that fulvestrant suffers from numerous deficiencies, however, including poor oral bioavailability and inability to cross the blood brain barrier.

In some aspects, the present disclosure encompasses the insight that treatment of ER-associated brain metastases comprises administration of certain classes of complete estrogen receptor antagonists that are orally bioavailable and also capable of crossing the blood brain barrier. Accordingly, in some aspects, the present disclosure provides a method of treating cancer, the method comprising steps of: administering to a subject suffering from an ER-associated cancer a composition that delivers to the subject's brain a complete estrogen receptor antagonist, wherein the subject has been determined or is suspected of having brain metastases.

### Complete Estrogen Receptor Antagonists

In some aspects, the present disclosure teaches particular usefulness of compound(s) that are complete estrogen receptor antagonists. In some aspects of the present disclosure, a "complete estrogen receptor antagonist," as that term is used herein, is characterized by complete antagonism of the estrogen receptor without residual estrogen receptor agonist activity. For example, it is understood that a complete estrogen antagonist is an agent (e.g., a small molecule compound) that shows ER antagonism and no ER agonism in one or more of ERα protein level assays, MCF-7 cell line assays, Ishikawa cell line assays (measuring wild type ER and certain mutants including mutants lacking AF1 and/or AF2 domains), and rodent uterine weight gain assays. *See, generally,* WO 2017/059139 and U.S. 9,018,244. Alternatively or additionally, in some aspects of the present disclosure, a complete estrogen receptor antagonist has three characteristics: it (1) inhibits *both* activating function 1 (AF1) and activating function 2 (AF2), as *complete* anti-estrogen activity requires inactivation of both AF1 and AF2; (2) promotes ER degradation; and (3) lacks the partial ER agonist activity observed with certain other agents. Without being bound by theory, it is understood that complete inhibition of both AF1 and AF2 is required for complete estrogen receptor activity, activating mutations in the gene that codes for estrogen receptor 1 allows for activation of both AF1 and AF2 even in the absence of estrogen.

Given the importance of ER signaling in many cancers, as well as in certain cardiovascular, inflammatory, and neurodegenerative diseases, significant effort has been invested in developing therapeutic agents and modalities that target the ER. There is some fluidity/flexibility in terminology that has been used to describe ER-targeting agents, but a variety of agents, with different mechanisms, have been developed and/or studied.

Currently, fulvestrant is the only approved therapy that has each characteristic. But, as noted above, fulvestrant suffers from numerous shortcomings, including poor oral bioavailability, and an inability to cross the blood brain barrier, making it entirely ineffective for the treatment of brain metastases related to ER-associated diseases or disorders.

### Certain Structural Features of Exemplary Compounds

In some aspects, the present disclosure encompasses the insight that certain classes of compounds are capable of completely antagonizing the estrogen receptor (i.e., are complete estrogen receptor antagonists), while also being able to cross the blood brain barrier, making them suitable for treatment of brain metastases related to ER-associated diseases or disorders. For example, among other things, the present disclosure reports certain complete estrogen receptor antagonists that exhibit complete estrogen receptor antagonism that is comparable to fulvestrant in various assays.

Suitable complete estrogen receptor antagonists include those reported in WO 2012/084711, WO 2014/191726, WO 2016/097072, WO 2017/059139, and WO 2019/245974. For example, in some aspects of the present disclosure, a complete estrogen receptor antagonist is selected from the group consisting of consisting of GDC-9545, SAR439859, AZD9833, and a compound of formula I 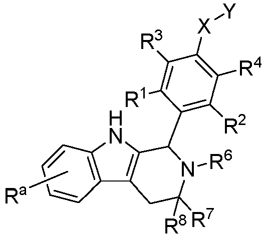 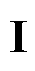 or a pharmaceutically acceptable salt thereof,
wherein:
X is -NH-, -CH₂-, or -O-;
Y is 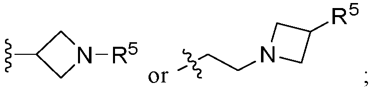
R^{a} is hydrogen or halo;
R¹, R², R³, and R⁴ are each independently selected from hydrogen and halo;
R⁵ is hydrogen or an optionally substituted group selected from C₁-C₆ alkyl, C₁-C₆ haloalkyl, C₂-C₆ alkenyl, and C₁-C₆ heteroalkyl;
R⁶ is hydrogen or an optionally substituted group selected from C₁-C₆ alkyl and C₁-C₆ haloalkyl;
R⁷ and R⁸ are each independently selected from hydrogen and optionally substituted C₁-C₆ alkyl.

In some aspects of the present disclosure, a complete estrogen receptor antagonist is SAR439859.

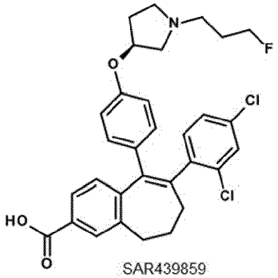

In some aspects of the present disclosure, a complete estrogen receptor antagonist is AZD9833.

In some aspects of the present disclosure, a complete estrogen receptor antagonist is GDC-9545.

In some aspects of the present disclosure, a complete estrogen receptor antagonist is

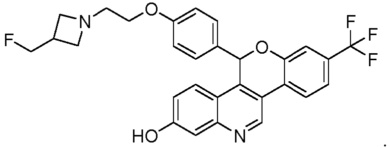

In some aspects of the present disclosure, a complete estrogen receptor antagonist is

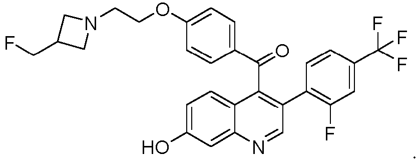

In some aspects of the present disclosure, a complete estrogen receptor antagonist is a compound of Formula I 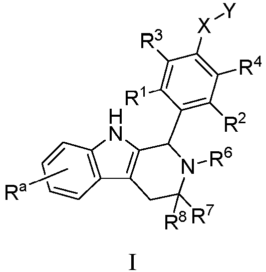 or a pharmaceutically acceptable salt thereof, wherein R^{a}, R¹, R², R³, R⁴, R⁶, R⁷, R⁸, X, and Y are described in classes and subclasses herein.

In some aspects of the present disclosure, as described generally above, X is -NH-, -CH₂-, or -O-. In some aspects of the present disclosure, X is -NH- or -O-. In some aspects of the present disclosure, X is -CH₂- or -O-. In some aspects of the present disclosure, X is -NH- or - CH₂-. In some aspects of the present disclosure, X is -NH-. In some aspects of the present disclosures, X is -CH₂-. In some embodiments, X is -O-.

In some aspects of the present disclosure, as described generally above, R^{a} is hydrogen or halo. In some aspects of the present disclosure, R^{a} is hydrogen. In some aspects of the present disclosure, R^{a} is halo. In some aspects of the present disclosure, R^{a} is fluoro, bromo, or chloro. In some aspects of the present disclosure, R^{a} is fluoro.

In some aspects of the present disclosure, as described generally above, R¹, R², R³, and R⁴ are each independently selected from hydrogen and halo. In some aspects of the present disclosure, R¹, R², R³, and R⁴ are each hydrogen. In some aspects of the present disclosure, R¹, R², and R³ are each hydrogen and R⁴ is halo. In some aspects of the present disclosure, R¹, R², and R³ are each hydrogen and R⁴ is fluoro. In some aspects of the present disclosure, R², R³, and R⁴ are each hydrogen and R¹ is halo. In some aspects of the present disclosure, R², R³, and R⁴ are each hydrogen and R¹ is fluoro. In some aspects of the present disclosure, R¹, R³, and R⁴ are each hydrogen and R² is halo. In someaspects of the present disclosure, R¹, R³, and R⁴ are each hydrogen and R² is fluoro. In some aspects of the present disclosure, R¹, R², and R⁴ are each hydrogen and R³ is halo. In some aspects of the present disclosure, R¹, R², and R⁴ are each hydrogen and R³ is fluoro. In some aspects of the present disclosure, R¹ and R² are each hydrogen and R³ and R⁴ are each halo. In some aspects of the present disclosure, R¹ and R² are each hydrogen and R³ and R⁴ are each fluoro. In some aspects of the present disclosure, R³ and R⁴ are hydrogen and R¹ and R² are halo. In some aspects of the present disclosure, R³ and R⁴ are hydrogen and R¹ and R² are fluoro.

In some aspects of the present disclosure, R¹ and R² are each hydrogen, and R³ and R⁴ are each hydrogen or halo, and when one of R³ or R⁴ is halo, the other of R³ or R⁴ is hydrogen. In some aspects of the present disclosure, R¹ and R² are each hydrogen, and R³ and R⁴ are each hydrogen or fluoro, and when one of R³ or R⁴ is fluoro, the other of R³ or R⁴ is hydrogen.

In some aspects of the present disclosure, as described generally above, Y is 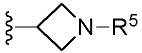 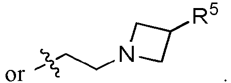 In some aspects of the present disclosures, Y is 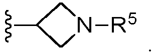 In some aspects of the present disclosure, Y is

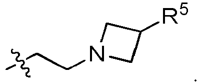

In some aspects of the present disclosure, as described generally above, R⁵ is hydrogen or an optionally substituted group selected from C₁-C₆ alkyl, C₁-C₆ haloalkyl, C₂-C₆ alkenyl, and C₁-C₆ heteroalkyl. In some aspects of the present disclosure, R⁵ is an optionally substituted group selected from C₁-C₆ alkyl, C₁-C₆ haloalkyl, C₂-C₆ alkenyl, and C₁-C₆ heteroalkyl. In some aspects of the present disclosure, R⁵ is an optionally substituted group selected from C₁-C₆ alkyl, C₁-C₆ haloalkyl, and C₂-C₆ alkenyl. In some aspects of the present disclosure, R⁵ is an optionally substituted group selected from C₁-C₆ alkyl and C₁-C₆ haloalkyl.

In some aspects of the present disclosure, R⁵ is an optionally substituted C₁-C₆ alkyl. In some embodiments, R⁵ is unsubstituted C₁-C₆ alkyl. In some aspects of the present disclosure, R⁵ is methyl, ethyl, propyl, butyl, pentyl, or hexyl. In some aspects of the present disclosure, R⁵ is methyl, ethyl, n-propyl, n-butyl, n-pentyl, or n-hexyl. In some aspects of the present disclosure, R⁵ is n-propyl.

In some aspects of the present disclosure, R⁵ is an optionally substituted C₁-C₆ haloalkyl. In some aspects of the present disclosure R⁵ is -CH₂-halo, -CH₂-CH₂-halo, -CH₂-CH₂-CH₂-halo, or -CH₂-CH₂-CH₂-CH₂-halo. In some aspects of the present disclosure, R⁵ is -CH₂-fluoro, -CH₂-CH₂-fluoro, -CH₂-CH₂-CH₂-fluoro, or -CH₂-CH₂-CH₂-CH₂-fluoro. In some aspects of the present disclosure, R⁵ is -CH₂-F or -CH₂-CH₂-CH₂-F. In some aspects of the present disclosure, R⁵ is - CH₂-F. In some aspects of the present disclosure, R⁵ is -CH₂-CH₂-CH₂-F.

In some aspects of the present disclosure, Y is 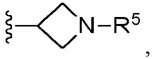 wherein R⁵ is C₁-C₆ alkyl. In some aspects of the present disclosure, Y is 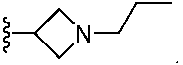 In some aspects of the present disclosure, Y is 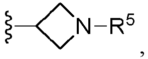 wherein R⁵ is C₁-C₆ haloalkyl. In some aspects of the present disclosure is

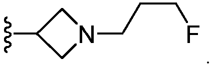

In some aspects of the present disclosure, Y is 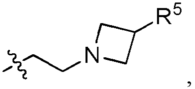 wherein R⁵ is C₁-C₆ haloalkyl. In some aspects of the present disclosure, Y is

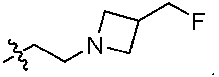

In some aspects of the present disclosure, as described generally above, R⁶ is hydrogen or an optionally substituted group selected from C₁-C₆ alkyl and C₁-C₆ haloalkyl. In some aspects of the present disclosure, R⁶ is hydrogen. In some aspects of the present disclosure, R⁶ is an optionally substituted group selected from C₁-C₆ alkyl and C₁-C₆ haloalkyl.

In some aspects of the present disclosure, R⁶ is optionally substituted C₁-C₆ alkyl. In some aspects of the present disclosure R⁶ is C₁-C₆ alkyl substituted with one or more groups selected from halogen and -(CH2)0-4OR°. In some aspects of the present disclosure, R⁶ is C₁-C₆ alkyl substituted with one or more groups selected from halogen and -OR°. In some aspects of the present disclosure, R⁶ is C₁-C₆ alkyl substituted with one or more groups selected form halogen and -OH. In some aspects of the present disclosure, R⁶ is

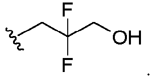

In some aspects of the present disclosure, R⁶ is C₁-C₆ haloalkyl. In some embodiments, R⁶ is C₁-C₄ haloalkyl. In some aspects of the present disclosure, R⁶ is -CH₂-C(CH₃)₂-F or -CH₂-CF₃.

In some aspects of the present disclosure, as described generally above, R⁷ and R⁸ are each independently selected from hydrogen and optionally substituted C₁-C₆ alkyl. In some aspects of the present disclosure, one of R⁷ and R⁸ is hydrogen and the other of R⁷ and R³ is C₁-C₆ alkyl. In some aspects of the present disclosure, one of R⁷ and R⁸ is hydrogen and the other of R⁷ and R⁸ is methyl. In some aspects of the present disclosure, R⁷ is hydrogen and R⁸ is R-methyl (i.e., methyl having a stereochemical orientation designated as R). In some easpects of the present disclosure, R⁷ is hydrogen and R⁸ is S-methyl (i.e., methyl having a stereochemical orientation designated as S).

Accordingly, in some aspects of the present disclosure, a complete estrogen receptor antagonist is a compound of formula (I), wherein the complete estrogen receptor antagonist is selected from: 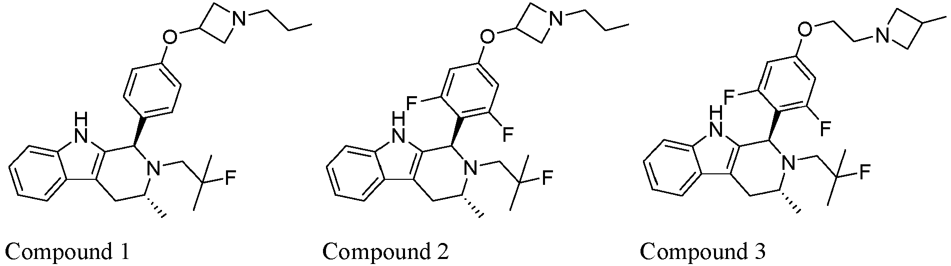 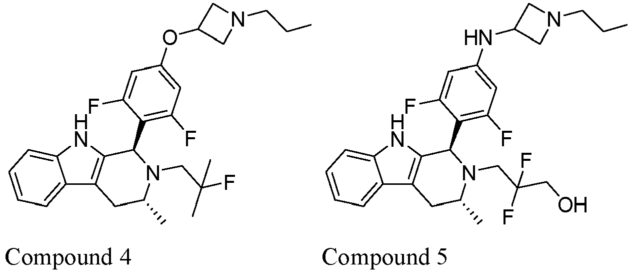 or a pharmaceutically acceptable salt thereof.

In some aspects of the present disclosure, a complete estrogen receptor antagonist is Compound 1

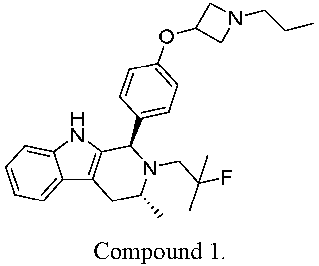

In some aspects of the present disclosure, a complete estrogen receptor antagonist is a free base form of Compound 1

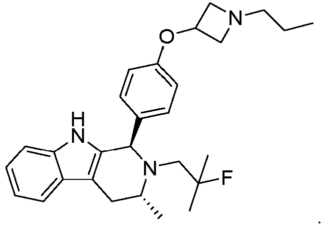

In some aspects of the present disclosure, a complete estrogen receptor antagonist is Compound 2

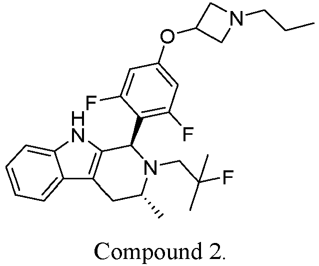

In some aspects of the present disclosure, a complete estrogen receptor antagonist is Compound 3

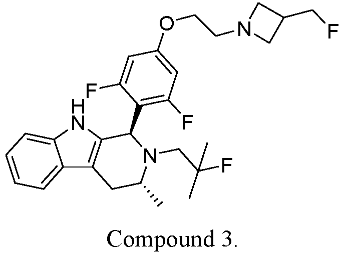

In some aspects of the present disclosure, a complete estrogen receptor antagonist is Compound 4

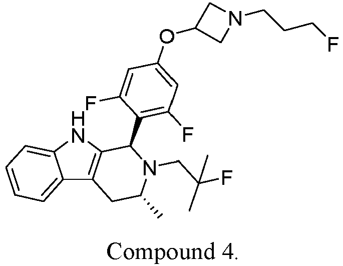

In some aspects of the present disclosure, a complete estrogen receptor antagonist is Compound 5

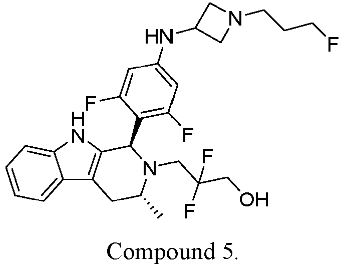

In some aspects of the present disclosure, a complete estrogen receptor antagonist is Compound 5a:

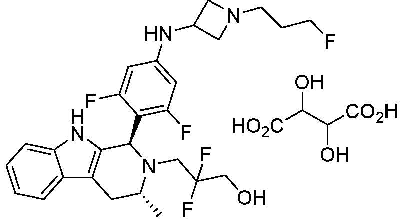

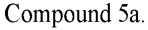

In some aspects of the present disclosure, a complete estrogen receptor antagonist is Compound 5b:

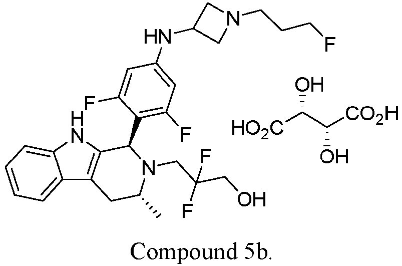

In some aspects of the present disclosure, a complete estrogen receptor antagonist is a compound selected from:
(1R,3R)-2-(2-fluoro-2-methylpropyl)-3-methyl-1-(4-((1-propylazetidin-3-yl)oxy)phenyl)-2,3,4,9,-tetrahydro-1H-pyrido[3,4-b]indole;
(1R,3R)-1-(2,6-difluoro-4-((1-propylazetidin-3-yl)oxy)phenyl)-2-(2-fluoro-2-methylpropyl)-3-methyl-2,3,4,9,-tetrahydro-1H-pyrido[3,4-b]indole;
(1R,3R)-1-(2,6-difluoro-4-(2-(3-(fluoromethyl)azetidin-1-yl)ethoxy)phenyl)-2-(2-fluoro-2-methylpropyl)-3-methyl-2,3,4,9,-tetrahydro-1H-pyrido[3,4-b]indole;
(1R,3R)-1-(2,6-difluoro-4-((1-(3-fluoropropyl)azetidin-3-yl)oxy)phenyl)-2-(2-fluoro-2-methylpropyl)-3-methyl-2,3,4,9,-tetrahydro-1H-pyrido[3,4-b]indole; and
3-((1R,3R)-1-(2,6-difluoro-4-((1-(3-fluoropropyl)azetidin-3-yl)amino)phenyl)-3-methyl-1,3,4,9,-tetrahydro-2H-pyrido[3,4-b]indol-2-yl)-2,2-difluoropropan-1-ol.

In some aspects of the present disclosure, a complete estrogen receptor antagonist is a free base. In some aspects of the present disclosure, a complete estrogen receptor antagonist is in the form of a pharmaceutically acceptable salt. In some aspects of the present disclosure, a pharmaceutically acceptable salt is as described herein. In some aspects of the present disclosure, a pharmaceutically acceptable salt is selected from succinate (e.g., a succinic acid salt form), tartrate (e.g., a tartaric acid salt form), and fumarate (e.g., a fumaric acid salt form).

In some aspects of the present disclosure, compounds described herein can be optionally labeled with a radiolabel, e.g., an isotope of certain atoms. These radiolabeled compounds are useful in the detection and visualization of certain ER⁺ tumors (e.g., via positron emission tomography (PET)). Exemplary radiolabels include, but are not limited to ¹¹C, ¹⁸F, ¹⁵O, ¹³N, and ¹³¹I.

In particular, it is understood that certain features of the presently described compounds allow them to be a combination of complete estrogen antagonists, orally bioavailable, and able to cross the blood brain barrier. Similar compounds, however, lack these three qualities. For example, AZD9496, a selective estrogen receptor degrader (SERD) is found to be orally bioavailable, but fails to completely antagonize the estrogen receptor at least because there remains some residual agonistic activity. Without being bound by theory, it is noted that AZD9496 lacks a heterocyclic moiety (e.g., an azetidinyl moiety) present in the compounds described herein. In particular, without being bound by theory, it is theorized that the combination of a heterocyclic moiety (e.g., an azetidinyl moiety) and a pyrido[3,4-b]indole provide a compound with certain desirable properties.

It is also theorized that the lack of halogen substitution on the phenyl bridge contributes to the ability of certain compounds to cross the blood brain barrier.

### Assessment of ER Antagonists

Among other things, the present disclosure teaches that useful ER antagonist agents are ones with CERAN activity as described herein.

One aspect of the present disclosure is an insight that conventional strategies for assessing or characterizing ER antagonist (and/or potential antagonist) agents were insufficient at least in that they typically did not distinguish between SERDs and CERANs. In particular, most such conventional strategies did not assess an agent's ability to specifically impact AF1.

Among other things, the present disclosure teaches that particularly useful ER antagonist agents are those that can inhibit ligand-independent ER activity; in some aspects of the present disclosure including activity observed with constitutive ER variant(s) such as, for example, AF2 deletions or truncations and/or LBD mutants (e.g., D538G and Y537S).

Furthermore, the present disclosure teaches that particularly useful ER antagonist agents are characterized by *each* of:
a. Inhibition of AF1 (e.g., inhibition of at least one, and preferably all known, constitutive ER variants)
b. Inhibition of AF2 (e.g., inhibition of ligand-dependent ER activity)
c. Promotion of ER degradation

Additionally, in some aspects of the present disclosure, particularly useful ER antagonist agents are further characterized by one or more of:
a. Oral bioavailability and a long half-life.
b. Blood brain barrier penetration.

In particular aspects of the present disclosure, activity of ER antagonist agent(s) may be assessed relative to that of one or more of ARN-810, AZD9496, Endoxifen, Fulvestrant, RAD1901, Tamoxifen, and/or compounds of formula I; in some such aspects of the present disclosure, comparison is contemporaneous, or alternatively, in some aspects of the disclosure, it may be with a historical record or future result.

### Methods of Use

The present disclosure encompasses the insight that certain complete estrogen receptor antagonists have a number of uses, including treatment of a ER-associated disorder (e.g., an ER-associated cancer, such as breast cancer, including metastatic brain cancer), detection, and/or characterization of certain tumors.

### Methods of Treatment

For example, in some aspects of the present disclosure, the present disclosure provides certain compositions for use in methods of treatment in a subject having an ER-associated disease, disorder, or condition. For example, in some aspects of the present disclosure, the present disclosure provides a composition for use in a method of treating an ER-associated disorder in a subject, wherein the subject has been determined or is suspected of having brain metastases. In some aspects of the present disclosure, the subject has developed brain metastases related to an ER-associated cancer, e.g., breast cancer, or a mutation to the estrogen receptor.

In some aspects of the present disclosure, the present disclosure provides in a composition for use in a method of treating cancer in a subject suffering from an ER-associated cancer, the improvement that comprises administering to the subject a composition that delivers to the subject's brain a complete estrogen receptor antagonist complete estrogen receptor antagonist, wherein the subject has been determined or is suspected of having brain metastases.

In some aspects of the present disclosure, the present disclosure provides, in a composition for use in a method of treating cancer in a subject with a complete estrogen receptor antagonist, the improvement that comprises administering to the subject the complete estrogen receptor antagonist, wherein the subject has been determined or is suspected of having brain metastases.

In some aspects of the present disclosure an ER-associated disorder is a cancer. In some aspects of the present disclosure, an ER-associated disorder is a breast cancer. In some aspects of the present disclosure, a subject suffering from an ER-associated disorder has developed a brain metastasis.

The present inventionprovides a composition for use in a method of preventing metastatic spread of an ER-associated cancer to the brain of a subject, the method comprising administering Compound 1: 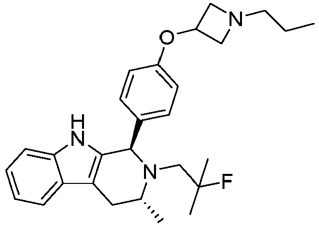 or a pharmaceutically acceptable salt thereof. In some embodiments, preventing metastatic spread of a cancer refers to inhibition of spread of a cancer localized in one part of the subject's body to the brain.

### Methods of Detection

The present disclosure further encompasses the insight that certain compounds described herein, e.g., radiolabeled versions of any compound described herein, are useful for detecting certain ER-associated diseases, disorders, and conditions. For example, a compound described herein where a non-radioactive fluorine atom is substituted with ¹⁸F, can be used to detect an ER-associated tumor via, for example PET.

### Methods of Characterizing

The present disclosure further encompasses the insight that efficacy of certain compounds described herein is assessed by in vitro and in vivo model assays. For example, certain compounds described herein are characterized according to any of the model assays described herein, including, for example, ERα protein level assays, MCF-7 cell line assays, Ishikawa cell line assays (measuring wild type ER and certain mutants including mutants lacking AF1 and/or AF2 domains), and rodent uterine weight gain assays. Further, in some aspects of the disclosure, the compounds described herein, when characterized by any referenced assay, exhibit complete estrogen receptor antagonism, as that term has been defined herein.

### Dosing

Aspects of the present disclosure provide a composition for use in a method of treating a subject suffering from an ER-associated disorder, wherein the subject has developed or is suspected to have developed brain metastases, the method comprising administering a composition comprising a complete estrogen receptor antagonist. In some aspects of the present disclosure, the composition comprises a complete estrogen receptor antagonist and a pharmaceutically acceptable excipient, carrier, or diluent. Said composition may be administered orally, parenterally, by inhalation or nasal spray, topically (e.g., as by powders, ointments, or drops), rectally, buccally, intravaginally, intraperitoneally, intracisternally or via an implanted reservoir, depending on the severity of the condition being treated. Preferably, the compositions are administered orally, intraperitoneally or intravenously. In certain aspects of the disclosure, provided compounds are administered orally or parenterally at dosage levels of about 0.01 mg/kg to about 50 mg/kg, of subject body weight per day, one or more times a day, to obtain the desired therapeutic effect.

Pharmaceutically acceptable compositions described herein may be orally administered in any orally acceptable dosage form including, but not limited to, capsules, tablets, aqueous suspensions or solutions. In such solid dosage forms the active compound may be admixed with at least one inert diluent such as sucrose, lactose or starch. Such dosage forms may also comprise, as is normal practice, additional substances other than inert diluents, e.g., lubricants and other tableting aids such a magnesium stearate and microcrystalline cellulose. When aqueous suspensions are required for oral use, the active ingredient is combined with emulsifying and suspending agents. If desired, certain sweetening, flavoring or coloring agents may also be added.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar--agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and/or i) lubricants such as talc, calcium stearate, magnesium stearate, solid polyethylene glycols, sodium lauryl sulfate, and mixtures thereof. In the case of capsules, tablets and pills, the dosage form may also comprise buffering agents. The active compounds can also be in micro-encapsulated form with one or more excipients as noted above.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings (i.e. buffering agents) and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes.

Liquid dosage forms for oral administration include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Alternatively, pharmaceutically acceptable compositions described herein may be administered in the form of suppositories for rectal or vaginal administration. These can be prepared by mixing the compounds of the present application with suitable non-irritating excipients or carriers that are solid at room temperature but liquid at body (e.g. rectal or vaginal) temperature and therefore will melt in the rectum or vaginal cavity to release the active compound. Such materials include cocoa butter, a suppository wax (e.g., beeswax) and polyethylene glycols.

A person of skill in the art would readily understand how a therapeutically effective dose determined for an animal can be converted to the corresponding human equivalent dose. Accordingly, a person of skill in the art would understand that certain provided data for an animal (e.g., a mouse) can be used to determine a suitable dose in a human, for example by using the table provided by Nair & Jacob, J. Basic Clin. Pharm., 7(2):27-31 (2016).

Aspects of the present disclosure provide dosing regimens where compounds reported herein are dosed at levels and/or according to regimens corresponding to those exemplified herein for Compound 1 (see, e.g., Example 4). That is, a dose (i.e., a composition optionally comprising additional pharmaceutically acceptable excipients) refers to a particular ratio of compound weight per kilogram of subject. For example, a dose of 3 mg/kg refers to a composition, optionally comprising pharmaceutically acceptable excipients, where the compound is administered to the subject in an amount that is 3 milligrams for every kilogram of subject weight. It is understood that a weight of a compound is determined according to the free base weight of a compound (e.g., if a compound is a salt, the corresponding free base weight of the compound is used to determine the amount of compound in the dose). Accordingly, in aspects of the present disclosure, a human subject is provided a dose that corresponds to a 3 mg/kg to 30 mg/kg in a mouse. In some aspects of the present disclosure, a human subject is provided a dose that correspondence to 3, 4, 5, 6, 7, 8, 9, 10, 11, 12, 13, 14, 15, 16, 17, 18, 19, 20, 21, 22, 23, 24, 25, 26, 27, 28, 29, or 30 mg/kg in a mouse. In some aspects of the present disclosure, a human subject is provided a dose that corresponds to greater than or equal to 3 mg/kg in a mouse. In some aspects of the present disclosure, a human subject is provided a dose that corresponds to greater than or equal to 5 mg/kg in a mouse. In some aspects of the present disclosure, a human subject is provided a dose that corresponds to greater than or equal to 10 mg/kg in a mouse. In some aspects of the present disclosure, a human subject is provided a dose that corresponds to greater than or equal to 15 mg/kg in a mouse. In some aspects of the present disclosure, a human subject is provided a dose that corresponds to greater than or equal to 20 mg/kg in a mouse. In some aspects of the present disclosure, a human subject is provided a dose that corresponds to greater than or equal to 25 mg/kg in a mouse. In some aspects of the present disclosure, a human subject is provided a dose that corresponds to greater than or equal to 30 mg/kg in a mouse.

In some aspects of the present disclosure, a composition comprising a complete estrogen receptor antagonist is administered as a unit dosage form. In some aspects of the present disclosure, a composition comprising a complete estrogen receptor antagonist is administered in the form of a capsule. In some aspects of the present disclosure, a composition comprising a complete estrogen receptor antagonist is administered in the form of a tablet. In some aspects of the present disclosure, a composition comprising a complete estrogen receptor antagonist is administered as a suspension. In some aspects of the present disclosure, a composition comprising a complete estrogen receptor antagonist is administered as a solution.

In some aspects of the present disclosure, a composition comprising a complete estrogen receptor antagonist is administered as a daily dose (QD). In some aspects of the present disclosure, a composition comprising a complete estrogen receptor antagonist is administered as a twice daily dose (BID). In some aspects of the present disclosure, a composition comprising a complete estrogen receptor antagonist is administered every other day (QOD). In some aspects of the present disclosure, a composition comprising a complete estrogen receptor antagonist is administered as a weekly dose (QW). In some aspects of the present disclosure, a composition comprising a complete estrogen receptor antagonist is administered as a monthly dose (Q4W).

Aspects of the present disclosure also encompass the recognition that Compound 1 advantageously can be used to treat metastasized cancers, e.g., cancers that have spread to the brain, bones, lungs, liver, or the central nervous system. As illustrated the table below, Compound 1, when administered in a single oral 300 mg/kg dose, is able to penetrate the blood brain barrier. Other estrogen receptor antagonists, e.g., fulvestrant, are unable to penetrate the blood-brain barrier in analogous quantities.

| Matrix | Group | Dose | Dose Unit | Concentration (ng/ml) |
|---|---|---|---|---|
| Plasma | Compound 1 10 mpk | 10 | mg/kg | 1919 |
| Plasma | Compound 1 30 mpk | 30 | mg/kg | 5558 |
| Brain | Compound 1 10 mpk | 10 | mg/kg | 2147 |
| Brain | Compound 1 30 mpk | 30 | mg/kg | 9708 |
| Brain | Fulvestrant 5 mg qw | 5 | mg | 501 |

### Combination Therapies

Aspects of the present disclosure encompasse the recognition that a combination of certain agents can beneficially be used to completely antagonize the estrogen receptor. Accordingly, in some aspects of the present disclosure, the present disclosure provides a method of treating a subject suffering from an ER-associated disorder (e.g., a cancer or breast cancer) comprising administering a complete estrogen receptor antagonist and an anti-cancer agent. For example, in some aspects of the present disclosure, the anti-cancer agent is a CDK 4/6 inhibitor, a PI3KCA inhibitor, or an mTOR inhibitor.

In some aspects of the present disclosure, the present disclosure provides a composition for use in a method of treating a patient or subject suffering from a cancer, the method comprising administering a complete estrogen receptor antagonist and an anti-cancer agent is a CDK4/6 inhibitor (i.e., inhibits one or both of CDK4 and CDK6). In some aspects of the present disclosure, an anti-cancer agent is a CDK4/6 inhibitor selected from palbocociclib, ribociclib, abemaciclib, lerociclib, trilaciclib, and SHR6390. In some embodiments, the CDK 4/6 inhibitor is palbocociclib. In some aspects of the present disclosure, the CDK4/6 inhibitor is ribociclib. In some aspects of the present disclosures, the CDK4/6 inhibitor is abemaciclib. In some aspects of the present disclosure, the CDK4/6 inhibitor is lerociclib. In some aspects of the present disclosure, the CDK4/6 inhibitor is trilaciclib. In some aspects of the present disclosure, the CDK 4/6 inhibitor is SHR6390.

In some aspects of the present disclosure, the present disclosure provides a composition for use in a method of treating a patient or subject suffering from a cancer, the method comprising administering a complete estrogen receptor antagonist and an anti-cancer agent, wherein the secondary agent is a PIK3CA inhibitor. In some aspects of the present disclosure, the PIK3CA inhibitor is selected from alpelisib, taselisib, and LY3023414. In some aspects of the present disclosure, the PIK3CA inhibitor is alpelisib. In some aspects of the present disclosure, the PIK3CA inhibitor is taselisib. In some aspects of the present disclosure, the PIK3CA inhibitor is LY3023414.

In some aspects of the present disclosure, the present disclosure provides a method of treating a patient or subject suffering from a cancer, the method comprising administering a complete estrogen receptor antagonist and an anti-cancer agent, wherein an anti-cancer agent is an mTOR inhibitor. In some aspects of the present disclosures, the mTOR inhibitor is selected from sirolimus, temsirolimus, everolimus, and LY3023414. In some aspects of the present disclosure, the mTOR inhibitor is sirolimus. In some aspects of the present disclosure, the mTOR inhibitor is temsirolimus. In some aspects of the present disclosure, the mTOR inhibitor is everolimus. In some embodiments, the mTOR inhibitor is LY3023414.

It is understood that administering a complete estrogen receptor antagonist and an anti-cancer agent described herein can be administered simultaneously or separately. For example, in some aspects of the present disclosure, a complete estrogen receptor antagonist and an anti-cancer agent are administered simultaneously. In some aspects of the present disclosure, an anti-cancer agent is administered prior to administration of a complete estrogen receptor antagonist. In some aspects of the present disclosure, an anti-cancer agent is administered after administration of a complete estrogen receptor antagonist.

### EXEMPLIFICATION

The following abbreviations may be used in the Examples below: aq. (aqueous); ACN (acetonitrile); CSA (camphorsulfonic acid); d (day or days); DCM (dichloromethane); DEA (diethylamine); DHP (dihydropyran); DMF (N,N-dimethylformamide); DIPEA (N,N-diisopropylethylamine); DMAP (4-dimethylaminopyridine); DMSO (dimethyl sulphoxide); EA (ethyl acetate); ee (enantiomeric excess); equiv. (equivalent); ethanol (EtOH); h (hour or hours); Hex (hexanes); HPLC (high-performance liquid chromatography); IPA (isopropyl alcohol); KHMDS (potassium bis(trimethylsilyl)amide); LAH (lithium aluminum hydride); LCMS (liquid chromatography-mass spectrometry); LDA (lithium diisopropylamide); LiHMDS (lithium bis(trimethylsilyl)amide); MeOH (methanol); min (minute or minutes); NMR (nuclear magnetic resonance); Pd/C (palladium on carbon); PPh3O (triphenylphosphine oxide); Pt/C (platinum on carbon); rb (round-bottomed); Rf (retention factor); rt or RT (room temperature); SM (starting material); TEA (triethylamine); THF (tetrahydrofuran); THP (tetrahydropyran); TLC (thin layer chromatography); TsOH (p-toluenesulfonic acid or tosylic acid); and UV (ultraviolet).

### Example 1: Synthesis of Compound 1

The complete synthesis of Compound 1 is provided in PCT App. Pub. No. WO 2017/059139 (referred to as Compound B, or, (1R,3R)-2-(2-fluoro-2-methylpropyl)-3-methyl-1-(4-((1-propylazetidin-3-yl)oxy)phenyl)-2,3,4,9-tetrahydro-1H-pyrido[3,4-b]indole).

### Preparation of 4-((1-propylazetidin-3-yl)oxy)benzaldehyde

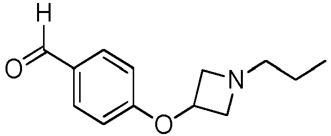

### Step 1: Preparation of 1-propionylazetidin-3-one

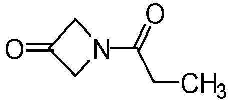

The compound 3-azetidinone hydrochloride (10.000 g, 93.0 mmol, 1.0 equiv.), anhydrous 1,2-dichloroethane (200 mL) and diisopropylethylamine (38.9 mL, 223 mmol, 2.4 equiv.) were added to a round bottom flask (500 mL) to provide a light yellow suspension. The suspension was sonicated for 1 h and then cooled to -10 °C (dry-ice/MeOH) for 10 min. Propionyl chloride (9.8 mL, 112 mmol, 1.2 equiv.) was added dropwise to the cooled suspension to provide an orange solution. The reaction was removed from the bath and stirred at room temperature for 16 h. The solvent was removed to provide a semi-solid. The semi-solid was suspended into EA (300 mL) and the suspension was filtered. The solid was rinsed with EA (2 x 100 mL). TLC analysis (10% MeOH/DCM, KMnO₇ stain/Heat) indicated there were three spots: Rf: 0.2, 0.5, 0.7. TLC (50% EA/Hex, KMnO₇ stain/Heat) indicated there were two spots: Rf: 1, 0.3. The filtrate was concentrated, adsorbed onto silica gel (25 g) and chromatographed through silica gel (100 g cartridge) with DCM (5 min) then 0-10 % MeOH over 15 min. The product came off early from the column in DCM and continued to elute from the column with up to 10 % MeOH. TLC in both solvent systems was carried out to determine if any propionyl chloride was present in early fractions. Fractions containing product were pooled and concentrated to afford the title compound as a yellow liquid (11.610 g, 98.2%).
¹H NMR (300 MHz, CDCl₃) δ: 4.80 (d, *J =* 5.6 Hz, 4H), 2.29 (q, *J =* 7.5 Hz, 2H), 2.01 (s, 3H), 1.18 (t, *J* = 7.5 Hz, 3H).

### Step 2. Preparation of 1-propylazetidin-3-ol

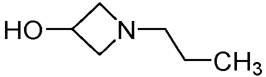

Lithium aluminum hydride (10.397 g, 273.9 mmol, 3.0 equiv.) was suspended into THF (200 mL) and cooled in an ice bath. A solution of 1-propionylazetidin-3-one (11.610 g, 91.3 mmol, 1.0 equiv.) in THF (100 mL) was added dropwise to the reaction mixture via a pressure equalizing addition funnel over 30 min. The addition funnel was removed. The flask was then fitted with a condenser and the reaction was heated at reflux in an oil bath at 75 °C for 16 h. The reaction was cooled in an ice bath for 20 min and sodium sulfate decahydrate (Glauber's salt, 25 g) was added in small portions over 20 min. After complete addition, the mixture was stirred at room temperature for 2 h. The mixture was filtered through a bed of Celite^{®} (2 cm) and the solids rinsed with EA (2 x 250 mL). The clear solution was concentrated to a pale yellow liquid (9.580 g, 91.1%). NMR indicated the presence of THF and EA. This material was used without further purification in the preparation of the compounds of the examples below.
¹H NMR (300 MHz, CDCl₃) δ: 4.39 (pent, *J =* 6 Hz, 1H), 3.62 - 3.56 (m, 2H), 2.90 - 2.85 (m, 2H), 2.41 (t, *J* = 7.5 Hz, 2H), 1.34 (hextet, *J* = 7.2 Hz, 2H), 0.87 (t, *J* = 7.8 Hz, 3H).

### Preparation of (R)-1-(1H-indol-3-yl)-N-((R)-1-phenylethyl)propan-2-amine:

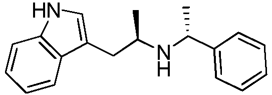

Indole-3-acetone (25.0 g, 144 mmol, 1.0 equiv.) was added to a solution of (*R*)-(+)-1-phenylethylamine (23.0 mL, 181 mmol, 1.3 equiv.) in dichloromethane (600 mL) under N₂ at 25 °C and the mixture was allowed to stir for 1 hr. The reaction was cooled to 0-5 °C and sodium triacetoxyborohydride (100 g, 472 mmol, 3.3 equiv.) was added over 30 minutes via powder addition funnel to the ice cooled solution. The orange solution was stirred for 1 h at 0 °C and then was allowed to warm to RT. The reaction was stirred at RT for 19 h. At this time, ESI+ indicated that no indole starting material was present. Saturated NaHCO₃ solution (100mL) was added in 5 mL portions over 15 min at 10 °C with vigorous stirring. The solution was stirred for 15 min and sat. Na₂CO₃ solution (200 mL) was added over 15 minutes. Solid K₂CO₃ (9 g) was added in 3 g portions at which point the aqueous layer was pH 12 and bubbles had stopped forming. The layers were filtered and separated. The red organic layer was washed with sat. aq. NaHCO₃ (2 x 100 mL). The aqueous layers were combined and extracted with DCM (2 x 100 mL). The combined organic layers were dried over Na₂SO₄, filtered and concentrated to give the crude product (49 g). TLC (90:10 DCM:MeOH) showed four spots (Rf = 0.63, 0.50, 0.16, 0.26), two of which were the separated diastereomeric major products (Rf = 0.16 and 0.26). The crude was adsorbed onto silica gel and purified via flash chromatography (330 g cartridge, 0-100% EA:Hex). Fractions containing the R,R diastereomer were pooled and purified a second time with the same flash chromatography conditions to afford 24 g of product (~82% ee). Previous successful separation was achieved by a silica gel:crude ratio of 40:1, so the mixture was divided into 3 portions and separated on 3 x 330 g silica gel cartridges (0-40% EA/Hex for 20 min, isocratic 40% EA/Hex 40 min). All fractions containing the desired product were > 99 % diastereomerically pure. Pure fractions were concentrated and pooled to yield (R)-1-(1H-indol-3-yl)-N-((R)-1-phenylethyl)-propan-2-amine as an orange semi-solid (11.91 g, 29.6 %).

¹H NMR (CDCl₃, 300 MHz) R,R diastereomer: δ 0.96 (d, *J* = 6.6 Hz, 3H), 1.30 (d, *J* = 6.6 Hz, 3H), 2.68 (q, *J* = 7.2 Hz, 1H), 2.97 (m, 2H) 4.00 (q, *J* = 6.3 Hz, 1H), 7.43-6.97 (m, 10H), 7.96 (br s, 1H). R,S diastereomer: δ 1.11 (d, *J* = 5.7 Hz, 3H), 1.30 (d, *J* = 5.4 Hz, 3H) 2.80 (m, 3H), 3.92 (q, *J* = 6.9 Hz, 1H), 6.93-7.40 (m, 10H), 8.13 (br s, 1H); the aromatic region was difficult to distinguish from the R,R diastereomer due to lack of purity.
LCMS: ES+ [M+H]+ 279.0.

### Preparation of (2R)-1-(1H-indol-3-yl)propan-2-amine

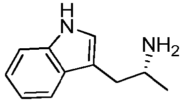

The compound (*R*)-1-(1H-indol-3-yl)-N-((R)-1-phenylethyl)propan-2-amine (11.91 g, 42.8 mmol, 1.0 equiv.) was dissolved in methanol (250 mL) and added to a 2 L Parr bottle and the solution was sparged with N₂ for 10 min. 20% Pd(OH)₂ on carbon wet with water (10.71 g, 76.3 mmol, 1.8 equiv.) was added and the bottle was pressurized with 50 psi of hydrogen and shaken in a Parr apparatus for 22 h, LCMS analysis indicated that the reaction was completed. The suspension was filtered through Celite^{®} and concentrated to remove MeOH. The crude was dissolved into DCM and washed with saturated Na₂CO₃ solution (50 mL) and the aqueous layer was extracted with DCM (2 x 50 mL). The organic layers were combined, dried, and concentrated to yield (2R)-1-(1H-indol-3-yl)propan-2-amine as a light brown solid that did not require further purification (6.68 g, 89.6 %).

¹H NMR (CDCl₃, 300 MHz) δ 1.17 (d, *J* = 6.6 Hz, 3H), 2.66 (dd, *J* = 8.4, 14.7 Hz, 1H), 2.88 (dd, *J* = 5.4, 14.1 Hz, 1H), 3.27 (sextet, *J* = 1.5 Hz, 1H), 7.05-7.22 (m, 3H), 7.37 (d, *J* = 7.5 Hz, 1H), 7.62 (d, *J* = 8.7 Hz, 1H), 8.00 (br s, 1H).
LCMS: ES+ [M+H]+ 174.9.

### Preparation of 2-fluoro-2-methylpropanol

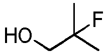

Methyl 2-fluoro-2-methylpropionate (5.01 g, 40.5 mmol, 1.0 equiv.) was added dropwise over 15 min to a stirred suspension of lithium aluminum hydride (2.50 g, 65.9 mmol, 1.6 equiv.) in anhydrous diethyl ether (100 mL) cooled in an ice bath. After 2 hours, 2.0 mL water, 2.0 mL 15% w/v NaOH, and 5.0 mL water were added sequentially dropwise. After 15 min, the white suspension was diluted with DCM, gravity filtered through Celite^{®}, and the solids were washed with DCM. The filtrate was concentrated (200 mbar, 25 °C) to afford 2-fluoro-2-methylpropanol as a colorless oil (2.09 g, 56.1 %).
¹H NMR (300 MHz, CDCl₃) δ 1.34 (d, J = 21.3 Hz, 6H), 1.95 (br t, 1H), 3.56 (dd, J = 6.6, 20.7 Hz, 2H).

### Preparation of 2-fluoro-2-methylpropyl trifluoromethanesulfonate

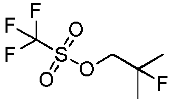

Trifluoromethanesulfonic anhydride (5.0 mL, 29.7 mmol, 1.3 equiv.) was added dropwise to a 0 °C solution of 2-fluoro-2-methylpropanol (2.090 g, 22.7 mmol, 1.0 equiv.) and 2,6 lutidine (3.40 mL, 29.4 mmol, 1.3 equiv.) in DCM (25 mL) over 30 minutes. After 2 hours, the red solution had turned light brown. TLC (20:80 EA:Hex, KMnO₄ stain) indicated that the starting material was not present. The reaction mixture was washed with 1M HCl solution (2 x 20 mL) and sat. NaHCO₃ solution (2 x 20 mL). The aqueous layers were each back extracted with DCM (20 mL). The combined organic layers were dried with Na₂SO₄, filtered and concentrated under reduced pressure (150 mbar, 25 °C) to afford 2-fluoro-2-methylpropyl trifluoromethanesulfonate as a red oil (4.39 g, 86.3%).

¹H NMR (300 MHz, CDCl₃) δ 1.46 (d, J = 20.4 Hz, 6H), 4.41 (d, J = 18.6 Hz, 2H). ¹⁹F NMR (282 MHz, CDCl₃) δ -147.1, -74.5.

### Preparation of (R)-N-(1-(1H-indol-3-yl)propan-2-yl)-2-fluoro-2-methylpropan-1-amine:

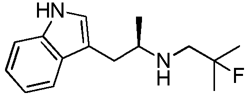

The compound 2-fluoro-2-methylpropyl trifluoromethanesulfonate (9.587 g, 42.8 mmol, 1.1 equiv.) (solution in DCM, 16% DCM by wt%, 11.4384 g) was added to a solution of (2R)-1-(1H-indol-3-yl)propan-2-amine (6.680 g, 38.3 mmol, 1.0 equiv.), anhydrous 1,4-dioxanes (60.000 ml, 701.4 mmol, 18.3 equiv.), and freshly-distilled diisopropylethylamine (8.500 ml, 48.8 mmol, 1.3 equiv.). The dark brown solution was heated at 90 °C for 3 hours. After 3h, LCMS indicated that a small amount of indolamine starting material was still present. TLC (10% MeOH/DCM) indicated triflate (Rf = 0.54) had been used up. NMR of unused triflate SM (286-30) indicated the triflate had not decomposed overnight, so another 0.1 equiv (0.9883 g, 13% DCM wt%, 0.8563 g triflate SM) was added and the reaction was heated for 2 h at 90 °C. LCMS indicated the reaction had completed and TLC (10% MeOH/DCM) showed one spot (Rf = 0.24) (TLC with 50% EA/Hex, 1 streaked spot Rf <= 0.12, another spot at Rf = 0). EtOAc (50 mL) was added and the solution was washed with NaHCO₃ (2 x 50 mL) and the combined aqueous layer was washed with EtOAc (50 mL). The combined organic extracts were dried over Na₂SO₄ and concentrated under reduced pressure. The crude (brown oil, 14.8 g) was purified via flash silica chromatography (240 g cartridge, 0-100% EA/Hex). The desired product eluted as a long tailing peak. Pure fractions were concentrated to yield (R)-N-(1-(1H-indol-3-yl)propan-2-yl)-2-fluoro-2-methylpropan-1-amine (4.211 g, 17.0 mmol) as a dark yellow oil.

¹H NMR (300 MHz, CDCl₃) δ 1.10 (d, J = 6.3 Hz, 3H), 1.34 (dd, J = 3.0, 21.9 Hz, 6H), 2.68-2.95 (m, 4H), 3.02 (sextet, J = 6.6 Hz, 1H), 7.05 (d, J = 2.4 Hz, 1H), 7.26-7.11 (m, 2H), 7.36 (d, J = 6.9 Hz, 1H), 7.62 (d, J = 7.5 Hz, 1H), 8.18 (br s, 1H). ¹⁹F NMR (282 MHz, CDCl₃) δ -144.2. m/z: ES+ [M+H]+ 249.0.

### Preparation of Compound 1

4-((1-propylazetidin-3-yl)oxy)benzaldehyde (0.096 g, 0.4 mmol, 1.3 equiv.) was added to a solution of (*R*)-N-(1-(1H-indol-3-yl)propan-2-yl)-2-fluoro-2-methylpropan-1-amine (0.070 g, 0.3 mmol, 1.0 equiv.) in anhydrous toluene (1.50 mL) and glacial acetic acid (0.100 mL, 1.7 mmol, 6.2 equiv.). Molecular sieves were added and the solution was stirred under N₂ in the dark at 80 °C for 8 hours. The reaction solution was diluted in DCM, filtered, and washed with saturated Na₂CO₃ solution. The aqueous layer was extracted with DCM and the combined organic layers were dried over Na₂SO₄. The solution was filtered and concentrated. The residue was dissolved into acetonitrile (2 mL) and filtered through a syringe filter before purification via prep LC (40 to 90% ACN:H₂O over 18 min, followed by isocratic 90% ACN for 7 min). Pure fractions were concentrated and dried to afford (1R,3R)-2-(2-fluoro-2-methylpropyl)-3-methyl-1-(4-((1-propylazetidin-3-yl)oxy)phenyl)-2,3,4,9,-tetrahydro-1H-pyrido[3,4-b]indole as a white powder.

### Example 2: Synthesis of Compounds 2-4

Specific synthetic methods for each of Compounds 2, 3, and 4 can be found in WO 2016/097072.

### Example 3: Synthesis of Compounds 5-5b

Specific synthetic methods for each of Compounds 5, 5a, and 5b can be found in WO 2019/245974.

### Example 4: In vivo Assessment of Compound 1

### Model development

### Intracranial tumor implantation

A tumor from a patient with subcutaneous ST941 tumor was used for intracranial inoculation of 8 NMRI nude mice.

The procedure was performed according to Minerva Imaging SOP 18.1.2: Single cell suspension from fresh tissue and intracranial injection.

In short: Subcutaneous ST941 PDX tumors grown in NMRI nude mice were harvested. The tumors were cut into small pieces and enzymatically digested to yield a suspension of single cells. The digestion was stopped, filtered through a 100 µm filter, washed in PBS and resuspended in PBS. The viability of the tumor cells was checked by trypan blue staining and the final concentration was 20 million viable cells/mL. The cells were kept on ice until inoculation.

Mice were anaesthetized by hypnorm/midazolam (1 ml/100 g body weight) and placed in a stereotactic frame for fixation of the head. A longitudinal incision was made in the scalp exposing the calvarium. A hole was drilled in the skull 1.5 mm right of the sutura saggitalis and 1.0 mm posterior to the bregma using a micro-drill. 10 µl of the cell suspension (200.000 cells) was injected at a depth of 2-2.5 mm at a rate of 60 nl/sec using a 100 µl syringe with a 25-gauge needle placed in a micro infusion pump. The needle was left for 3 minutes before being withdrawn. Bupivacain (0.2 mg/100 g body weight) and Lidocain (1 mg/100 g body weight) were administrated in the incision site for local anesthetic and the skin was closed with a suture.

The mice were earmarked for identification and returned to their cages where they were monitored until fully recovered from the anesthesia.

### MR imaging, tumor monitoring and euthanasia

Tumor growth was monitored by T2-weighted MR imaging. First scan was performed one week after inoculation and afterwards two times per week. Weight and scoring was performed at least two times per week. Daily scoring of animals was performed if weight loss ≥10% is observed.

Mice were euthanized by cervical dislocation when they meet the humane endpoints.

### Efficacy

### Intracranial tumor implantation

A tumor from a parent with subcutaneous ST941 tumor was used for intracranial inoculation of 54 athymic nude mice.

The procedure was performed according to Minerva Imaging SOP 18.1.2: Single cell suspension from fresh tissue and intracranial injection. See short description above.

### MR imaging for inclusion

Mice were continuously enrolled into the study based on MR imaging. The first MR imaging session was initiated 1-3 weeks after implantation depending on the results of the model establishment. MR imaging was performed two times per week afterwards until inclusion.

Mice were enrolled in the study when tumor volume reaches 2-5 mm³. Inclusion was the day after the actual MR imaging. The first 40 mice that met the enrolment criterion were randomized into 5 groups, see Table 1. Mice were randomized so that all groups had the same mean tumor volume at enrolment.

**Table 1**

| **Group** | **N** | **Compound** | **Dose (mg/kg)** | **Dose conc. (Stock) (mg/mL)** | **Dosing volume (mL/kg)** | **Route** | **Dosing schedule** |
|---|---|---|---|---|---|---|---|
| A | 8 | Vehicle | - | - | 10 | PO | QD for 49 days |
| B | 8 | Compound 1 | 10 | 1 | 10 | PO | QD for 49 days |
| C | 8 | Compound 1 | 3 | 0.3 | 10 | PO | QD for 49 days |
| D | 8 | Compound 1 + Ribociclib | 3 + 75 | 0.6 + 15 | 10 | PO | QD for 49 days |
| E | 8 | Ribociclib | 75 | 15 | 5 | PO | QD for 49 days |

### Supplementation and withdrawal of estrogen

Animals were supplemented with 17β-Estradiol in the drinking water from two days before intracranial inoculation. Estrogen was withdrawn when the animals are included into the study and dosing was initiated two days later.

### Therapy, weight monitoring and MR imaging

Mice were treated according to Table 1 (above). Therapy was given by oral gavage daily between 9 and 10 AM. First dose was given two days after inclusion.

Weight and visual assessment were monitored daily during therapy. Animals were scored three times per week according to the table below, and more often if a weight loss of 5% is evident.

| **Variable** | **Score** |
|---|---|
| ***Body weight changes^{#}*** | |
| <15% | 0 |
| 15-20% | 2 |
| >20% | 3 |

| ***Body Condition Score (BCS)** (see diagram for details)* | |
|---|---|
| **BCS** >3 | 0 |
| BCS ≤ 2 | 2 |

| ***Physical appearance*** | |
|---|---|
| Normal | 0 |
| Lack of grooming. Ocular discharge | 1 |
| Small bites or scratches. Nasal discharge | 2 |
| Serious bites or scratches. Abnormal posture, limb, tremor etc. | 3 |

| ***Unprovoked behavior*** | |
|---|---|
| Normal | 0 |
| Minor changes | 1 |
| Abnormal, reduced mobility, decreased alertness, inactive | 2 |
| Unsolicited vocalizations, self-mutilation, either very restless or immobile. Severe abnormal behavior. Repetitive behavior. | 3 |

| ***Behavioral responses to external stimuli*** | |
|---|---|
| Normal | 0 |
| Minor depression/exaggeration of response | 1 |
| Moderately abnormal responses | 2 |
| Violent reactions or comatose. Severe abnormal responses | 3 |

| ***Occipital tumor*** | |
|---|---|
| None | 0 |
| Palpable | 1 |
| ***Total score**** | |

Tumor growth was monitored by MR on day 5, 10, 15, 20 and 25 relative to the day of inclusion.

### Post therapy monitoring and euthanasia

Weight monitoring and scoring were performed three times per week post therapy and daily if weight loss of 5% is observed. Mice were euthanized by cervical dislocation when they meet the humane endpoints.

### Tissue preservation

Brain with tumor tissue was resected and preserved in 10% neutral buffered formalin for 48 hours (fixative ratio will be at least 1:20) and transferred to 70% ethanol. Samples were stored at 4°C until shipment.

FIG. 1A is a scatter plot measuring the volume (mm³) by MRI at inclusion in ST941 brain metastases for each mouse within each group A-E.

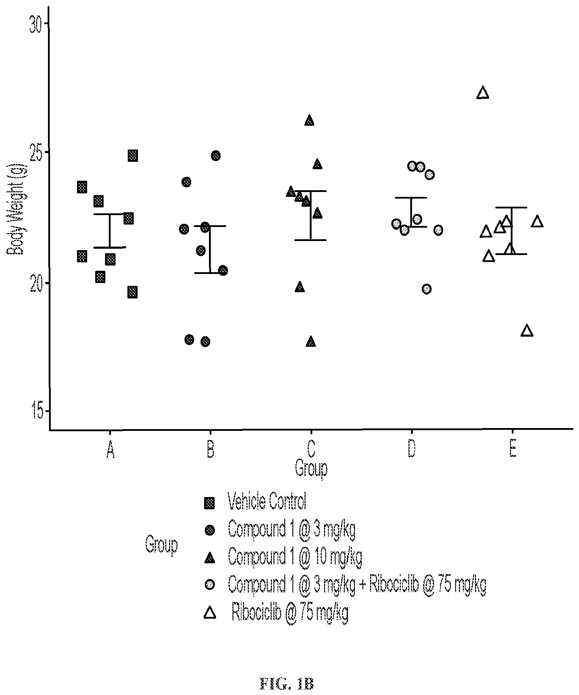

FIG. 1B is a scatter plot measuring the weight (g) by MRI at inclusion in ST941 brain metastases for each mouse within each group A-E.

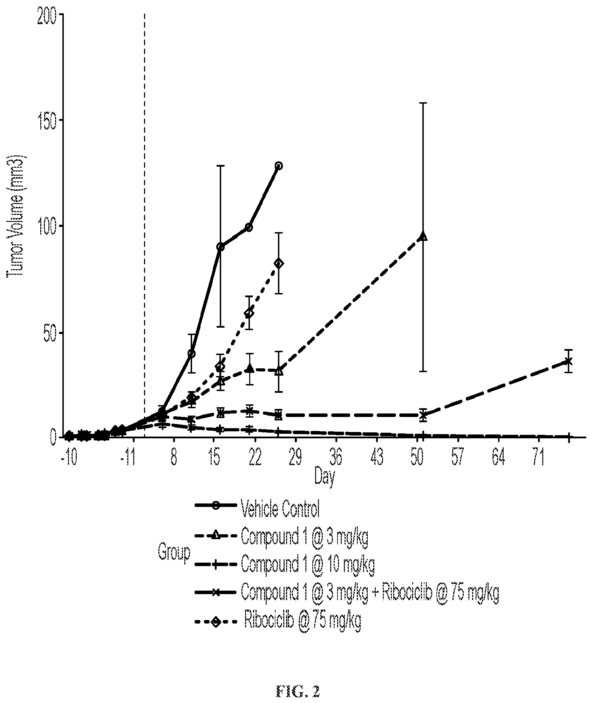

FIG. 2 is a scatter plot measuring the mean tumor volume for each group A-E over time.

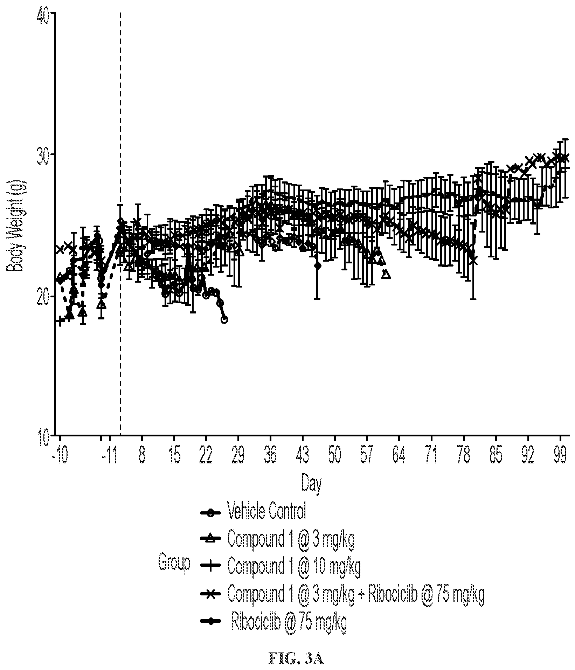

FIG. 3A is a scatter plot measuring the mean body weight for each group A-E over time.

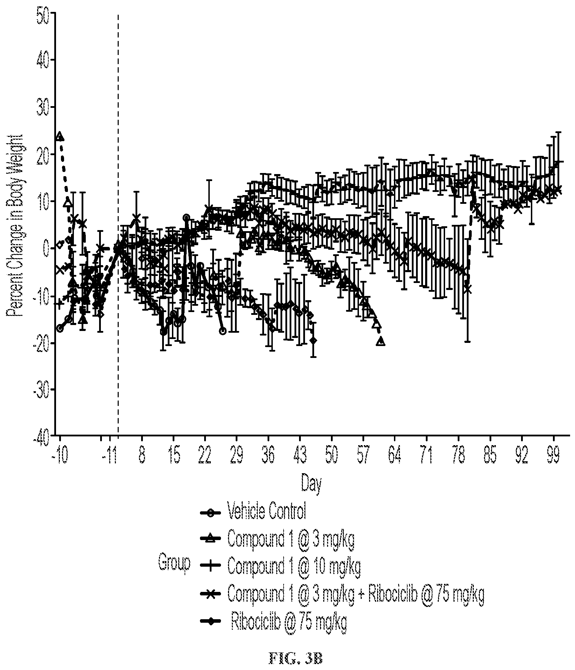

FIG. 3B is a scatter plot measuring the percent change in mean body weight for each group A-E over time.

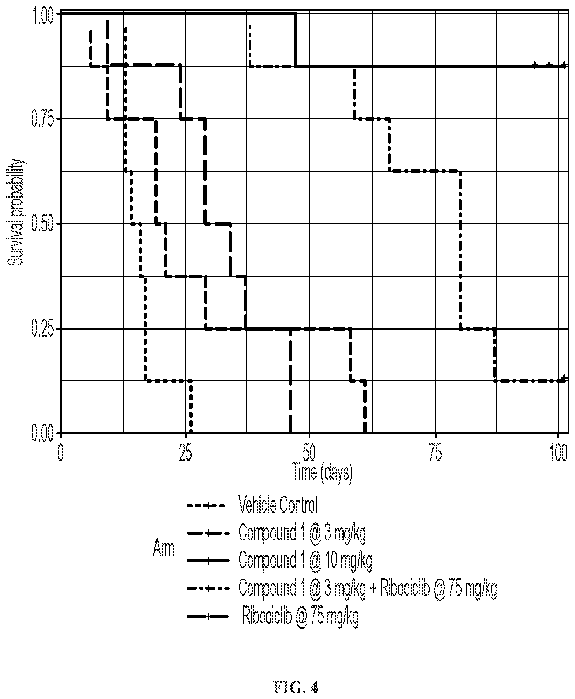

FIG. 4 is a Kaplan-Meier plot showing the percentage of mice surviving within each of groups A-E over time. Tick marks indicate censored data reflecting that mice were enrolled on different days.

### Example 5: Estrogen Receptor Protein Level Assay

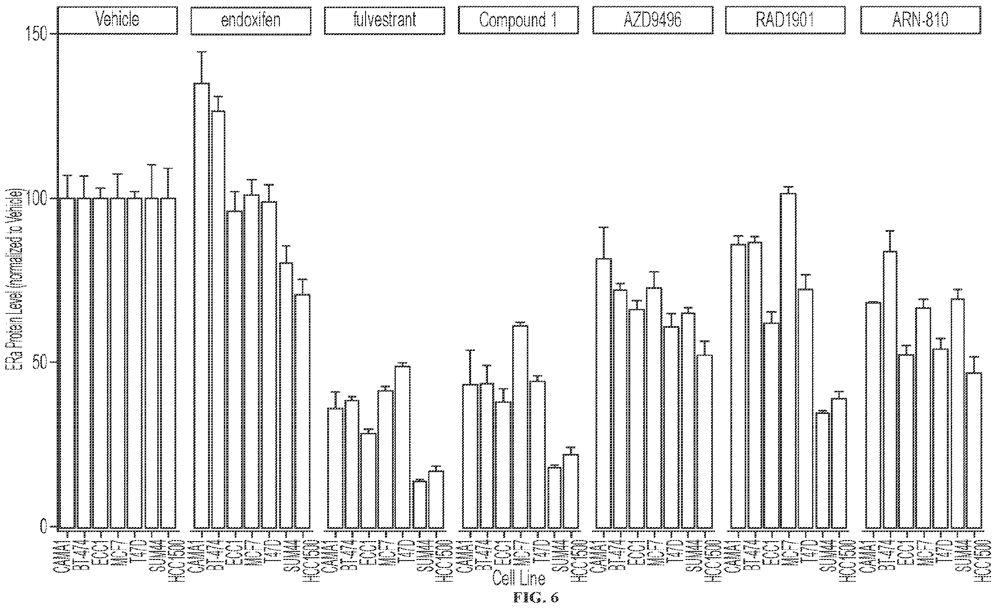

The present Example describes assessment of various compounds (ARN-810, AZD9496, Compound 1, Endoxifen, and Fulvestrant) on ERα protein level in a variety of cell lines. Depending on the cell type, 90,000-500,000 cells per well were previously plated into each well of a 12-well dish and incubated in phenol red-free media containing 5% charcoal dextran stripped fetal bovine serum (stripped FBS) (HyClone) for at least 24 hours. Cells were treated with 300 nM antiestrogen for 4 hours in serum-free medium and lysates subsequently lysed with RIPA buffer supplemented with protease and phosphatase inhibitors (ThermoFisher Scientific). Whole protein extracts were separated on 10% SDS-PAGE TGX gels and transferred to nitrocellulose membranes (BioRad). Blots were incubated mouse monoclonal anti-ERα, either D12 (#sc-8005, SantaCruz Biotechnology) or SP1 (#MA5-14501, ThermoFisher Scientific). β-actin monoclonal antibodies (#MA5-15739 or #MA5-16410 (ThermoFisher Scientific) or #sc-47778 (SantaCruz Biotechnology)) were used as loading controls. Blots were incubated with appropriate secondary antibodies conjugated to horse radish peroxidase (ThermoFisher Scientific). Signal was detected with Super Signal Femto chemiluminescent reagent (ThermoFisher Scientific). Results are depicted in FIG 6. As can be seen, all compounds other than Endoxifen showed significant ability to reduce ER protein level in most cell lines; Compound 1 and Fulvestrant were most effective at reducing ER protein level, and showed comparable activity in this respect.

### Example 6: Cell Proliferation Assays

The present Example describes assays that assess impact of tested compounds on human MCF-7 cells, which are a human ER⁺ breast cancer cell line. Specifically, 1000 MCF-7 cells (Cheryl Walker, Baylor College of Medicine) per well were plated into a 96-well plate in phenol red-free media (ThermoFisher Scientific) containing 5% stripped FBS. At least 4 hours later cells were treated with antiestrogens and media was diluted to 2.5% stripped FBS in the presence of 100 pM E2 for 6-8 days. Proliferation was measured using CyQuant fluorescent DNA-binding dye kit (ThermoFisher Scientific) using 1:200 GR dye and reading fluorescence at 485 nm excitation and 538 nm.

### Example 7: Estrogen Receptor Antagonism Analysis

### Transient Transfection of Estrogen Receptors and Variants

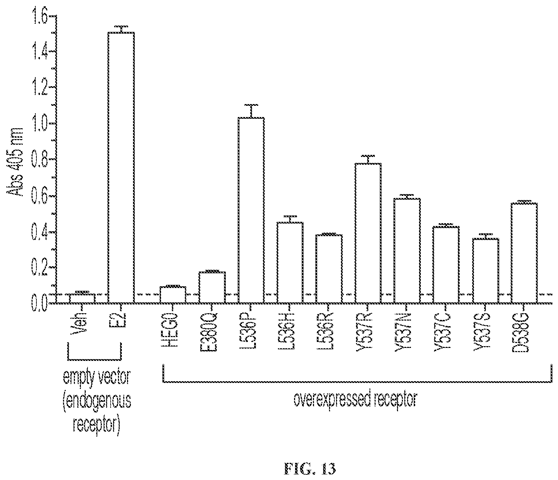

The present Example describes studies in which certain estrogen receptor constructs were transfected into Ishikawa cells, which are a human endometrial cancer cell line, endogenous alkaline phosphatase is assayed. 15,000 Ishikawa cells per well were plated into a 96-well plate in phenol-red media containing 5% stripped FBS. At the time of plating, cells in each well were transiently transfected with 75-100 ng of an estrogen receptor construct (or empty vector, pSG5) using Lipofectamine LTX (ThermoFisher Scientific). Approximately 4 hours later, cells were treated with indicated amount of anti-estrogen (in the absence of E2), or 500 pM E2 (FIG. 13), and media was diluted to 2.5% stripped FBS. Cells were incubated for 3 days, media removed, and plates frozen at -80°C. Thawed plates were incubated with p-nitrophenyl phosphate (ThermoFisher Scientific), which is a chromogenic substrate of AP and therefore reveals AP activity levels. After 40-80 minutes at 40°C, absorbance was read at 405 nm.

### Compound 1 has ER Antagonist, and not Agonist, Activity

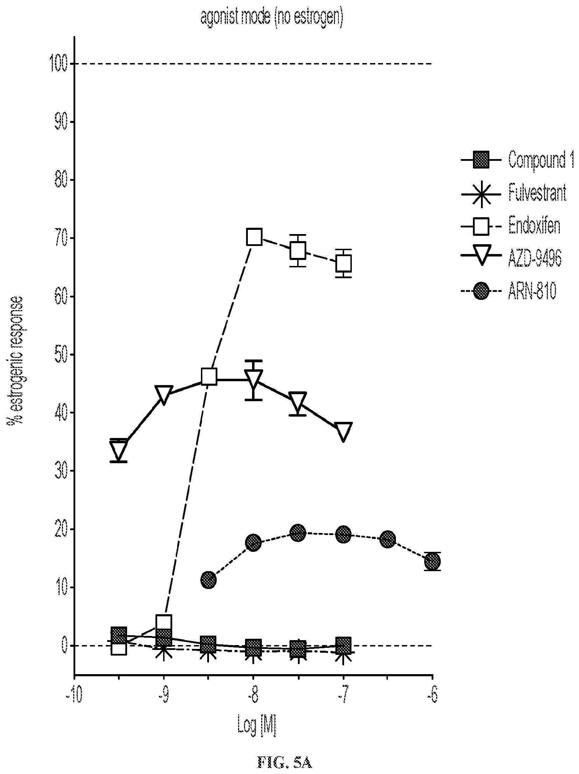

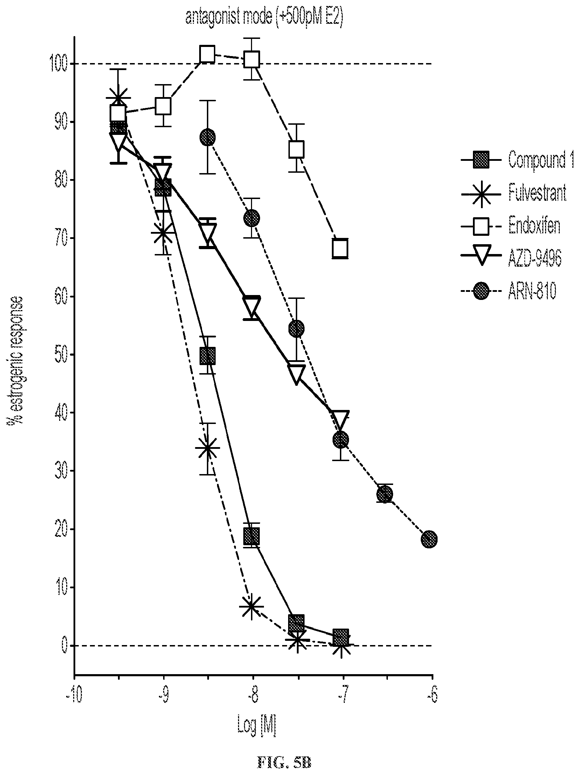

AP activity of endogenous wild type ER in un-transfected Ishikawa cells was assayed as described above. Cells were treated with indicated compound (ARN-810, AZD-9496, Compound 1, Endoxifen, or Fulvestrant) alone (agonist mode), or in the presence of 500 pM 17*β*-estradiol (E2) (antagonist mode). Results are presented in FIG 5A (agonist mode) and FIG 5B (antagonist mode). As can be seen, all compounds showed meaningful antagonist activity, with Compound 1 and Fulvestrant being most potent. All compounds other than Compound 1 and Fulvestrant also showed significant agonist activity.

### Certain Mutant ERs Increase Ligand-Independent ER Activity

Wild type ER (HEGO), empty vector (pSG5) or indicated LBD mutant ER was transiently transfected into Ishikawa cells as described above. Only the empty vector was treated with 500 pM 17*β*-estradiol (E2). 72 hours later, cells were assayed for AP activity. Results are presented in FIG. 13. Bars represent mean absorbance at 405 nm from triplicate wells, +s.e.m. As can be seen, various of the tested ER mutants were observed to be "activating mutants" in that they showed more activity than did the wild type ER when ligand was not present.

### Activation Domain 1 (AF1) is Required for Ligand-Independent Activity Observed with Certain ER Mutants

AF1 Wild type ER (HEGO, AA 1-595), empty vector (pSG5) or indicated ER missing the activation domain 2 ("AF2") (AA 1-282) or activation domain 1 ("AF1") (AA 178-595, with and without Y537S mutation), was transiently transfected into Ishikawa cells as described above. 72 hours later, cells were assayed for AP activity. Bars represent mean absorbance at 405 nm from quadruplicate wells, + s.e.m. As can be seen, F1AF1 is required for ligand-independent ER activity that is observed when the ER is truncated (ΔAF2), even in the presence of the activating Y537S mutation (ΔAF1/Y5372).

### Compound 1 Inhibits Activity of Ligand-Independent ER Mutants

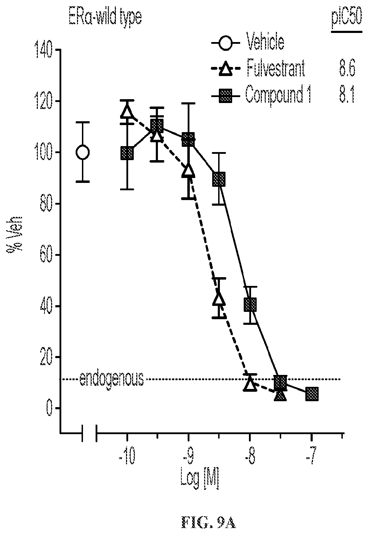

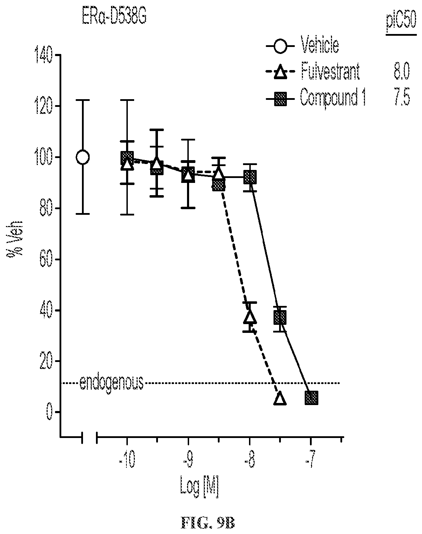

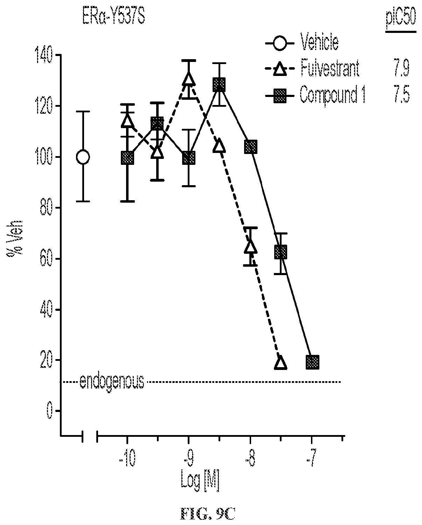

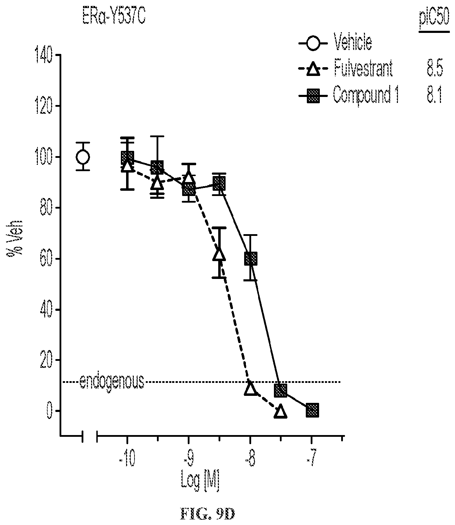

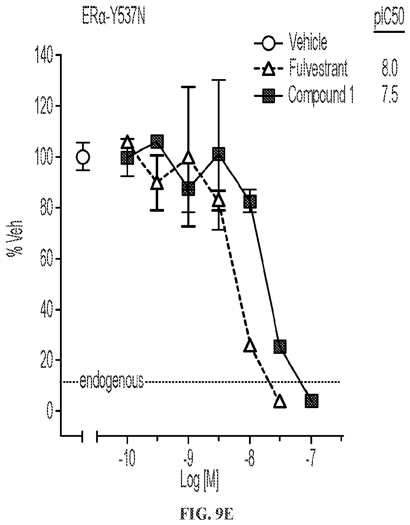

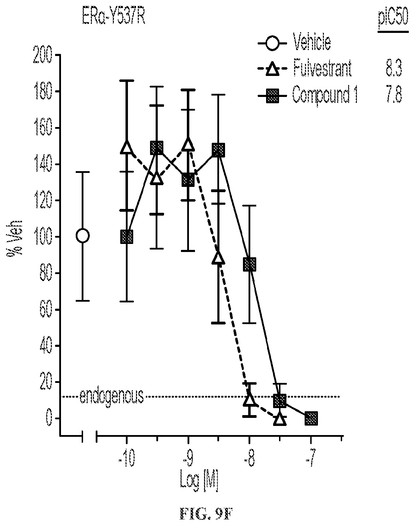

Wild type ER or an indicated ER variant transiently transfected into Ishikawa cells as described above, and activity was assayed in the presence of Compound 1 or fulvestrant. Results are presented in FIGs. 9A-9F. Points represent mean AP activity normalized to vehicle, +/- s.e.m. from duplicate wells. Dose response curves of Compound 1 and Fulvestrant were fit using the least squares fit method and pIC₅₀ (-Log IC₅₀) were calculated using a variable slope sigmoid dose-response model. Line represents the normalized AP activity of the endogenous receptor (transfected with empty vector (pSG5)). As can be seen, Compound 1 inhibited activity of each of the ligand-independent ER mutants with an IC50 comparable to that of fulvestrant.

### Certain Clinical Candidates Fail to Inhibit Activity of Ligand-Independent ER Mutants

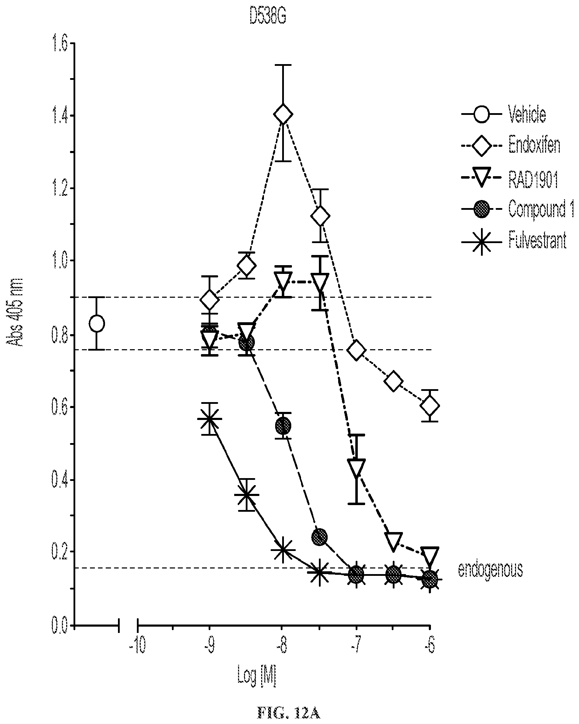

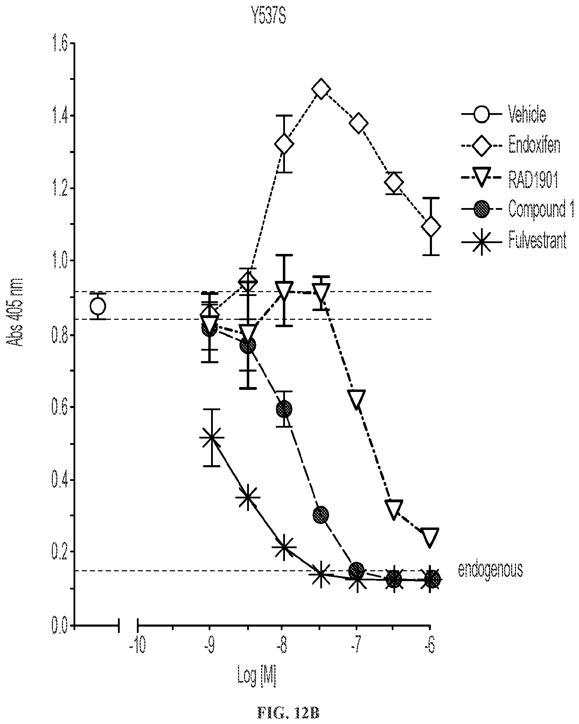

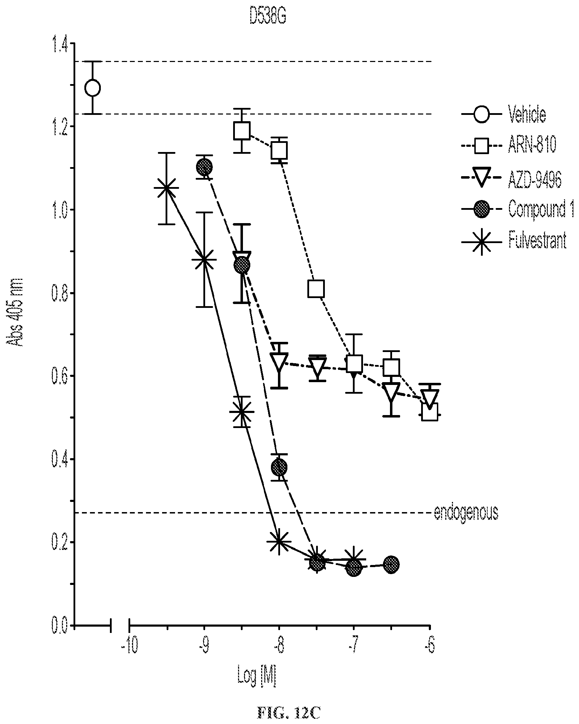

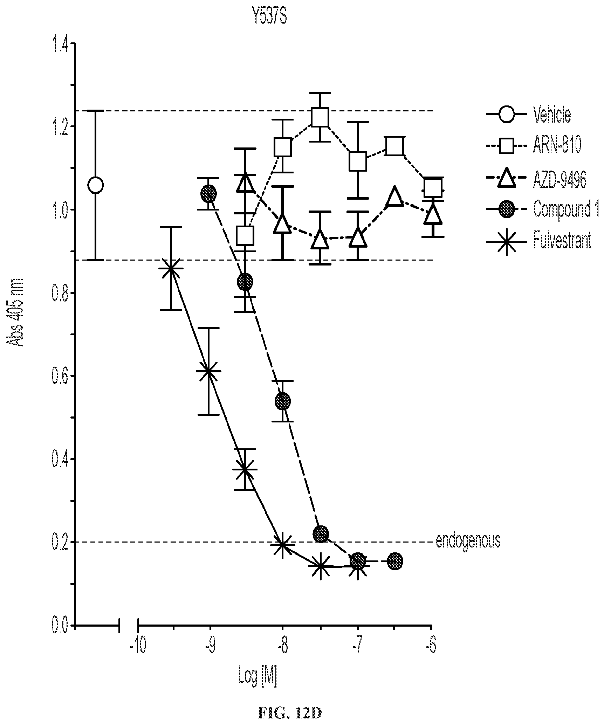

Wild type ER or an indicated ER variant transiently transfected into Ishikawa cells as described above, and activity was assayed in the presence of Compound 1 or Fulvestrant, as compared with Endoxifen, RAD-1901, ARN-810 (GDC-0810) or AZD-9496 (Results are depicted in FIGs. 12A-12B, where Compound 1 and Fulvestrant are compared with Endoxifen and RAD-1901 in FIGs. 12A and 12B, or with ARN-810 (GDC-0810) and AZD-9496 in FIGs. 12C-12D. Points represent mean absorbance at 405 nm from triplicate wells, + s.e.m. Line represents AP activity of the endogenous receptor (transfected with empty vector (pSG5)). As can be seen, none of Endoxifen, RAD-1901, ARN-810 (GDC-0810) or AZD-9496 can inhibit activity of the ligand-independent ER variants as Compound 1 and Fulvestrant do.

### Example 8: Xenograft Analysis of Compound 1 in ST941 PDX Brain Metastases

The present example describes the effects of Compound 1 on tumors derived from the patient derived xenograft (PDX) model ST941 that have been implanted directly into mouse brains.

Among other things, this Example describes effects of Compound 1 on Estrogen Receptor (ER) positive tumors that have been implanted directly into mouse brains, using patient derived human breast cancer cells containing an activating mutation in the estrogen receptor, the Y537S ESR1 mutation.

Compared regimens included ovariectomy plus vehicle, fulvestrant, tamoxifen, Compound 1, and a combination of ribociclib and Compound 1 monotherapy, against vehicle in the ST941 intracranial breast cancer brain metastisis model.

### Protocol and Materials

### In vitro procedures

A tumor from a parent with subcutaneous ST941 tumor was used for intracranial inoculation of 64 athymic nude mice. The procedure was performed according to the following method: Subcutaneous ST941 PDX tumors grown in NMRI nude mice was harvested. The tumors were cut into small pieces and enzymatically digested to yield a suspension of single cells. The digestion was stopped, filtered through a 100 µm filter, washed in PBS and resuspended in PBS. The viability of the tumor cells was checked by trypan blue staining and cells were suspended according the table below. The cells were kept on ice until inoculation.

| Inoculation Site | Animal Strain | n | Tumor Model | Cells/animal | Inoculation Medium |
|---|---|---|---|---|---|
| IC | Athymic Nude | 64 | ST941 | 200.000 in 10 µ | RPML 1640 |

### In vivo procedures

8 female NMRI nude mice (ordered age matched with a one week time frame, approximately 6 weeks of age) from Janvier Labs (France) were used in the model development.

### Tumor Implantation and Estrogen Administration

Tumors were prepared and implanted by the following method:

Animals were anaesthetized by hypnorm/midazolam (1 ml/100 g body weight) and placed in a stereotactic frame for fixation of the head. A longitudinal incision was made in the scalp exposing the calvarium. A hole was drilled in the skull 1.5 mm right of the sutura saggitalis and 1.0 mm posterior to the bregma using a micro-drill. 10 µl of the cell suspension (200.000 cells) was injected at a depth of 2-2.5 mm at a rate of 60 nl/sec using a 100 µl syringe with a 25-gauge needle placed in a micro infusion pump. The needle was left for 3 minutes before being withdrawn. Bupivacain (0.2 mg/100 g body weight) and Lidocain (1 mg/100 g body weight) was administrated in the incision site for local anesthetic and the skin was closed with a suture.

The animals were chipped for identification and returned to their cages where they were monitored until fully recovered from the anesthesia.

Mice were followed via MR imaging and were enrolled when tumor volume reached 2-5 mm³. Inclusion was performed either one or two days after MR imaging confirmed adequate tumor size. Mice were randomized such that all Groups had approximately equal tumor volume at enrollment. MR imaging was performed according to the following method:

The first MR imaging session was initiated 2 weeks after implantation. MR imaging was performed weekly or two times per week once tumor was established. Animals were enrolled when tumor volume reaches 2-5 mm3. Inclusion was the day after the actual MR imaging. Animals that met the enrolment criterion were stratified into 6 Groups according to Table 8-1. Animals were stratified so that all Groups have the same mean tumor volume at enrollment.

Estrogen supplement was provided via 17β-estradiol in drinking water from two days prior to intracranial innoculation. Estrogen supplement was withdrawn for each animal upon reaching sufficient tumor volume. Dosing for each mouse began two days after enrollment.

Table 8-1 summarizes treatments applied to different Groups:

**Table 8-1**

| **Arm Code** | **Arm** | **Route** | **Dose** | **Schedule** | **Animal Count** |
|---|---|---|---|---|---|
| A | Vehicle | Oral Gavage | 0.0 | QD for 49 days | 8 |
| B | Ovariectomy + Vehicle | Oral Gavage | 0.0 | QD for 49 days | 9 |
| C | Fulvestrant | IM Injection | 5.0 mg/mouse | QD for 49 days | 8 |
| D | Tamoxifen | Oral Gavage | 60.0 mg/kg | QD for 49 days | 8 |
| E | Compound 1 + Ribociclib | Oral Gavage | 10.0 +75.0 mg/kg | QD for 49 days | 8 |
| F | Compound 1 | Oral Gavage | 10.0 mg/kg | QD for 49 days | 9 |

The animals were treated according to Table 8-1. Therapy by oral gavage was given daily between 9 and 10 AM. The first dose was given two days after inclusion.

Compound 1 formulation was prepared by dissolving Compound 1 in DMSO to form a clear solution. This solution was transferred to a 0.5% CMC in millipore water such that the final concentration of DMSO was less than 5% v/v. During the addition of the DMSO solution, the Compound 1 precipitated to form a finely divided suspension in the vehicle. This suspension settled over time. Sonication and agitation were performed prior to administering the test article.

Ribociclib was formulated in an amount of 15 mg/mL. 100 mg ribociclib was suspended in 6.66 mL vehicle to reach a final concentration of 15 mg/mL.

A combination formulation of Compound 1 and ribociclib was prepared by mixing a 1:1 mL solution before administration.

### Ovariectomy

Animals in Group B were deprived of estrogen by an ovariectomy at the day of inclusion. Ovariectomy was performed according to the following method:

The animal was anesthetized (sevoflurane, 2-4% in ambient air supplemented with 100% O₂ at approximately 4:1 ratio) and placed in prone position on a heating pad. Carprofen (5 mg/kg) was administered subcutaneously before surgery and daily for 3 days post-surgery.

The area around the incision sites was disinfected using iodine. A 1 cm incision was made along the midline and the musculature was separated from the skin using curved scissors. A 1 cm lateral of the midline small incision was made through the musculature to reach the abdominal cavity. The white adipose tissue surrounding the ovary was removed using forceps. The proximal vessel and the uterine horn was ligated using a monofilament suture. The ovary was removed using a small scissor and the remaining tissue was placed back in the abdominal cavity.

The muscle layer wound and the skin layer wound were closed separately with resorbable sutures. The procedure was repeated on the opposite side.

### Therapy, weight monitoring and MR imaging

Weight and visual assessment was monitored daily during therapy. The animals were scored three times per week according to the table below and more often if a weight loss of 10% is evident.

| **Variable** | **Score** |
|---|---|
| ***Body weight changes^{#}*** | |
| <15% | 0 |
| 15-20% | 2 |
| >20% | 3 |

| ***Body Condition Score (BCS)** (see diagram for details)* | |
|---|---|
| **BCS** >3 | 0 |
| BCS ≤ 2 | 2 |

| ***Physical appearance*** | |
|---|---|
| Normal | 0 |
| Lack of grooming. Ocular discharge | 1 |
| Small bites or scratches. Nasal discharge | 2 |
| Serious bites or scratches. Abnormal posture, limb, tremor etc. | 3 |

| ***Unprovoked behavior*** | |
|---|---|
| Normal | 0 |
| Minor changes | 1 |
| Abnormal, reduced mobility, decreased alertness, inactive | 2 |

Tumor growth was monitored by MR on day 5, 10, 15, 20 and 25 relative to the day of inclusion.

The animals were euthanized by cervical dislocation when they meet the humane endpoints.

### Tissue preservation

Once the animals reached humane endpoint the following sampling was performed:
- Blood (plasma sample)
   ∘ 4 hours after last dosing whole blood was collected by cardiac puncture and transferred to an EDTA tube. Samples were centrifuged at 2000 x g for 10 minutes at 4°C. Plasma is transferred to a 2 mL round bottom Eppendorf tube and stored at -80°C.
- Brain with tumor tissue was resected.
   ∘ 4 of the brains from each Group was preserved in 10% neutral buffered formalin for 48 hours (fixative ratio will be at least 1:20) and transferred to 70% ethanol. Samples were stored at 4°C until shipment.
   ∘ 4 of the brains from each Group were snap frozen and stored at -80°C until shipment.

### Results

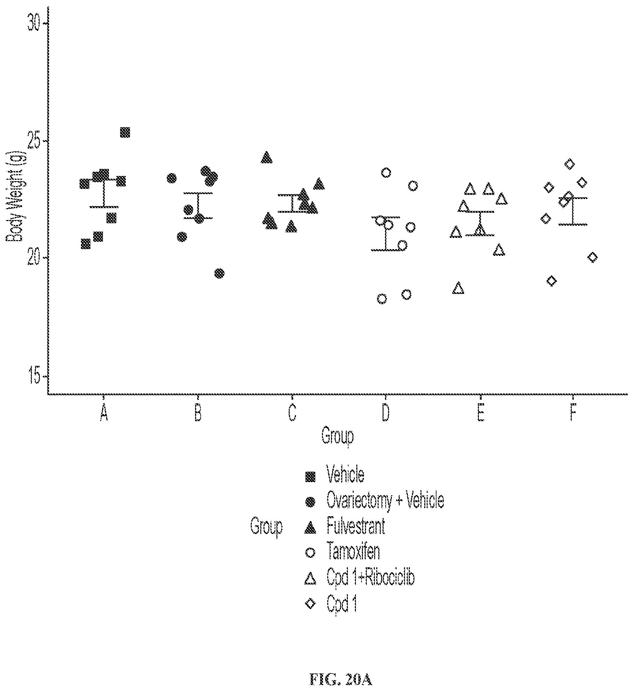

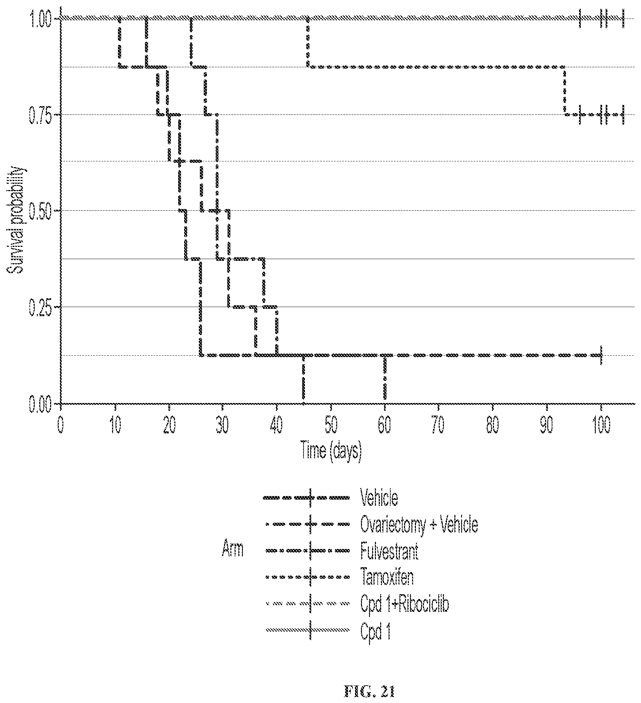

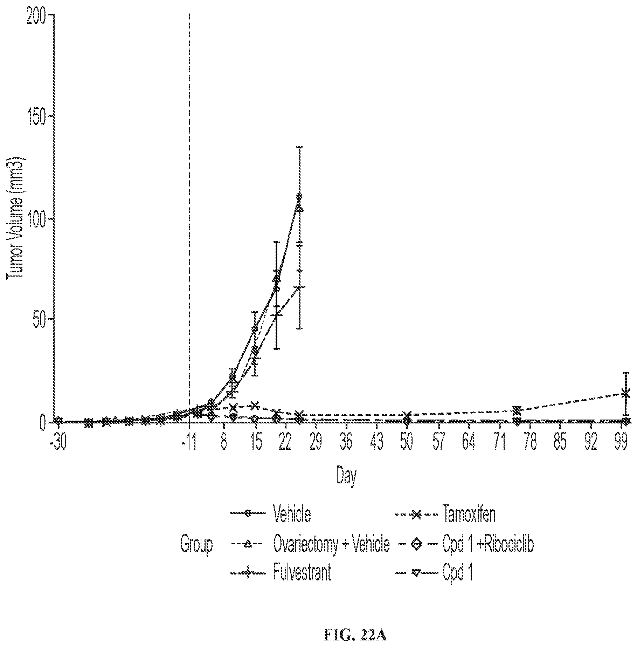

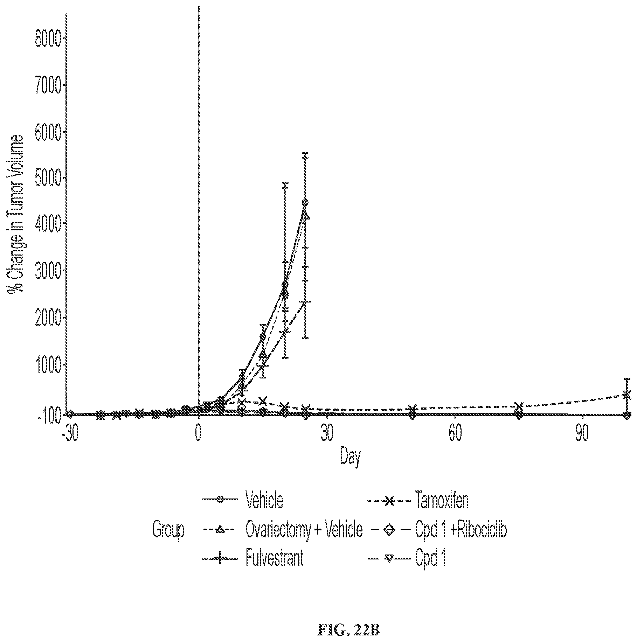

Results from the xenograft work described herein are provided in FIGs. 20A-27. These results illustrate that mice provided Compound 1, alone and in combination with ribociclib, exhibited improved survival rates as compared to other therapies, including fulvestrant. For example, almost all mice administered Compound 1 lived throughout the duration of the 100 day study, whereas at least half of the mice administered fulvestrant died in less than 30 days (see FIGs. 21, 24, and 25). The present disclosure, therefore, encompasses an insight that Compound 1, as a complete estrogen receptor antagonist, exhibits improved properties over fulvestrant. Tamoxifen was the only other therapy tested that slowed change in tumor volume, but not to the same extent as Compound 1 (see FIG. 22B). Animals treated with tamoxifen (Group D), however, after 100 days, started to see increases in tumor volume (see, e.g., FIGs. 22A, 22B, 24, 25), and the survival rate probability decreased after two members of the Group D died (see FIG. 21). The present disclosure also encompasses an insight that Compound 1 exhibits improved properties over tamoxifen in ability to treat brain metastases. Compound 1 is superior to tamoxifen for another reasons, including, but not limited to, Compound 1 being a complete estrogen receptor antagonist, whereas tamoxifen is a partial estrogen receptor agonist, as previously described.

FIG. 20A is a scatter plot measuring each animal's body weight at time 0.

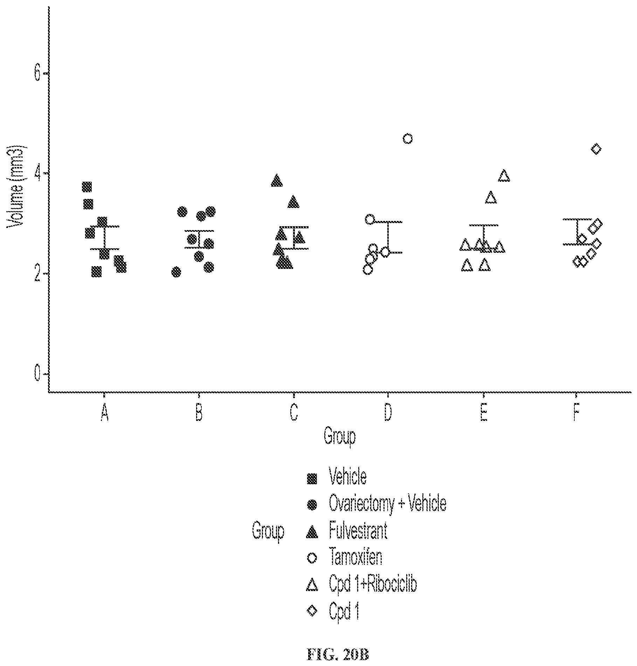

FIG. 20B is a scatter plot measuring each animal's tumor volume at time 0.

FIG. 21 is a Kaplan-Meier plot showing the percentage of surviving mice in each Group over time. Tick marks indicate censored data reflecting that mice were enrolled on different days. "Cmpd 1" refers to "Compound 1" as described herein.

FIG. 22A is a scatter plot measuring mean tumor volume over time for the each Group. "Cmpd 1" refers to "Compound 1" as described herein.

FIG. 22B is a scatter plot measuring percent change in tumor volume over time for each Group. "Cmpd 1" refers to "Compound 1" as described herein.

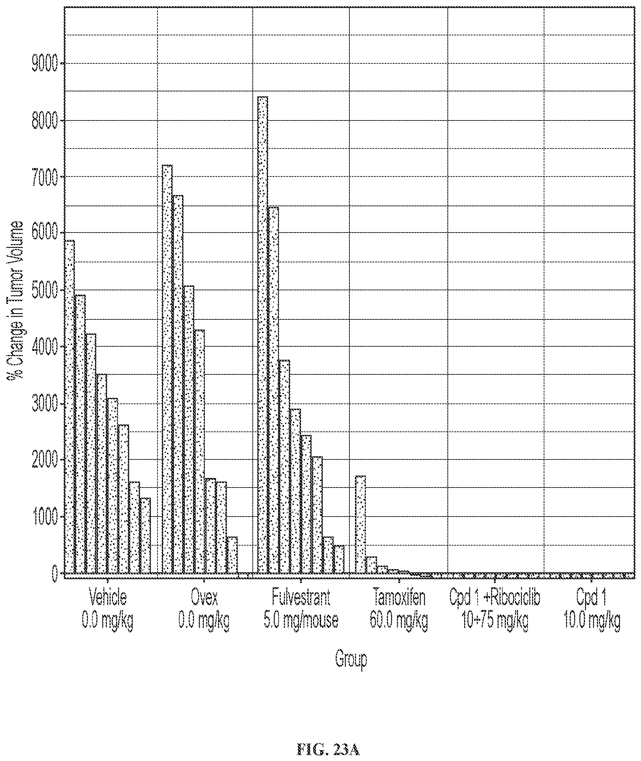

FIG. 23A is a waterfall plot measuring the percent change in tumor volume for each Group.

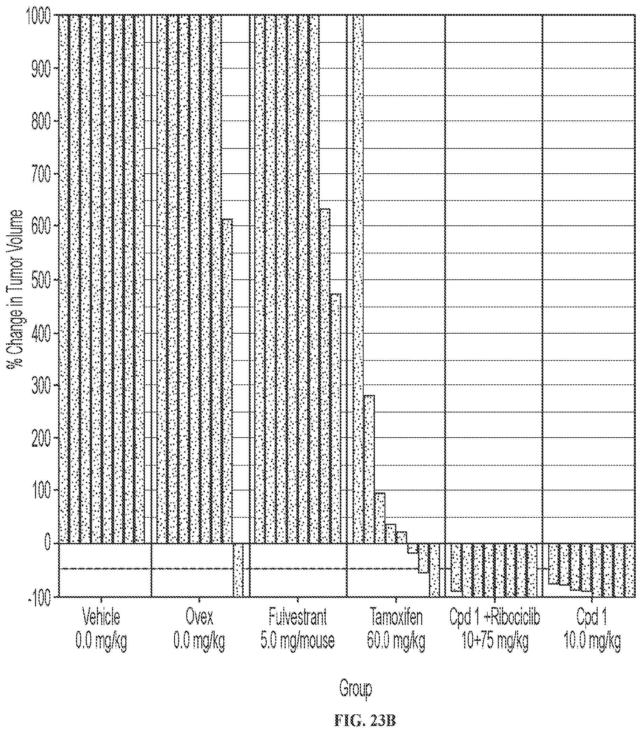

FIG. 23B is a zoomed in waterfall plot of FIG. 23A, illustrating the decrease in tumor volume size for certain Groups.

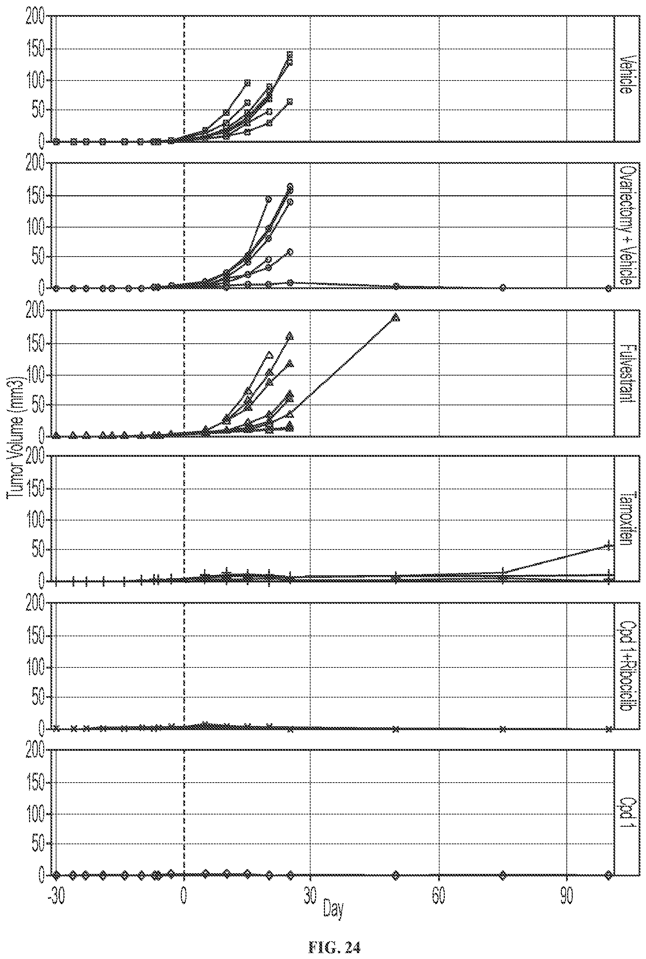

FIG. 24 is a scatter plot measuring mean tumor volume over time for individual animals of each Group.

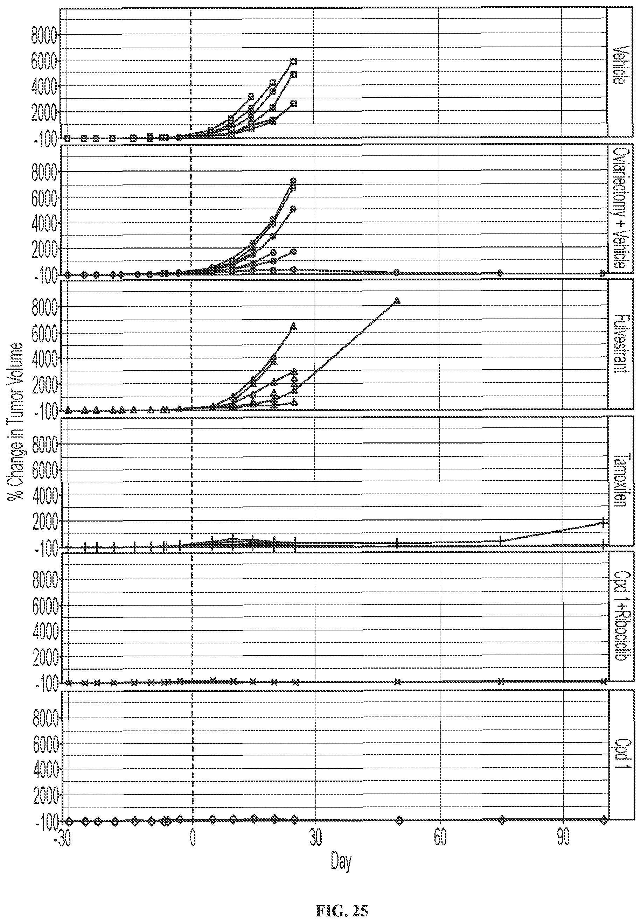

FIG. 25 is a scatter plot measuring percent change in tumor volume over time for individual animals of each Group.

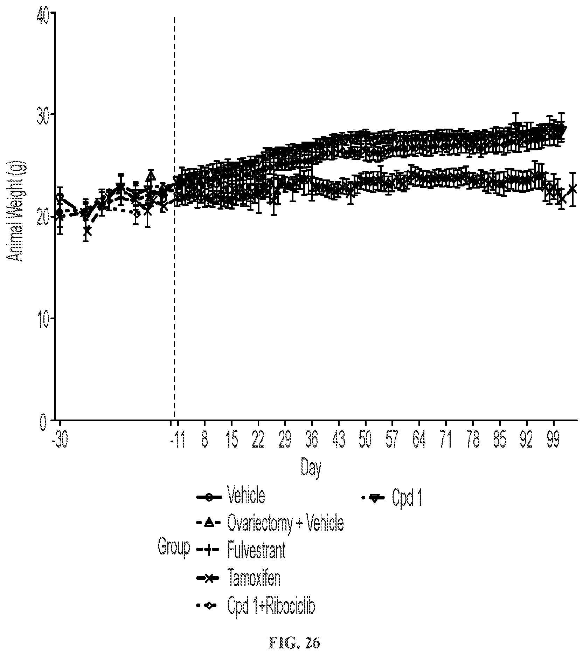

FIG. 26 is a scatter plot measuring animal weight over time for each Group.

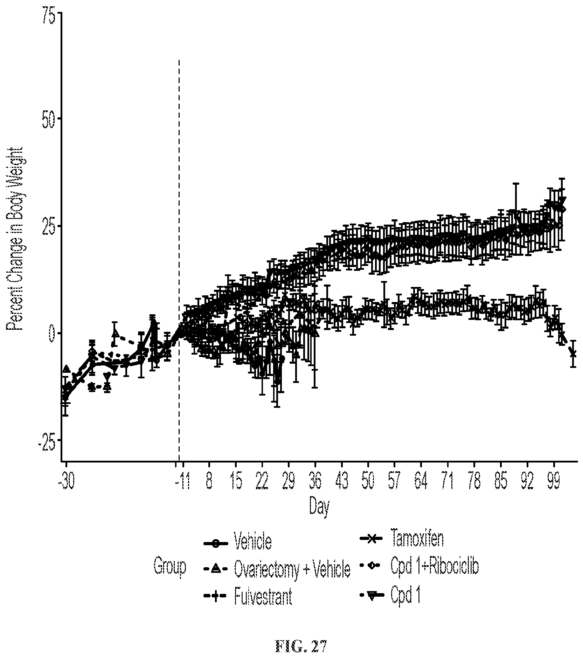

FIG. 27 is a scatter plot measuring percent change in animal weight over time for each Group.

The foregoing has been a description of certain non-limiting embodiments of the subject matter described within. Accordingly, it is to be understood that the embodiments described in this specification are merely illustrative of the subject matter reported within. Reference to details of the illustrated embodiments is not intended to limit the scope of the claims, which themselves recite those features regarded as essential.

## Claims

1. A composition for use in a method of treating an ER-associated cancer in a subject, the method comprising steps of:
administering to the subject a composition comprising a compound: 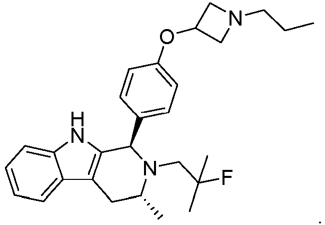
or a pharmaceutically acceptable salt thereof,
wherein the subject has been determined to have brain metastases.

2. The composition for use of claim 1, wherein the method further comprises administering a CDK 4/6 inhibitor, a PI3KCA inhibitor, or an mTOR inhibitor.

3. The composition for use of claim 2, wherein the method further comprises administering a CDK4/6 inhibitor;
preferably wherein the CDK4/6 inhibitor is selected from palbociclib, ribociclib, abemaciclib, lerociclib, and trilaciclib;
more preferably wherein the CDK4/6 inhibitor is selected from ribociclib, palbociclib, and abemaciclib;
still more preferably wherein the CDK4/6 inhibitor is ribociclib; or
still more preferably wherein the CDK4/6 inhibitor is palbociclib.

4. The composition for use of claim 2, wherein the method further comprises administering a PIK3CA inhibitor;
preferably wherein the PIK3CA inhibitor is selected from alpelisib and taselisib;
more preferably wherein the PIK3CA inhibitor is alpelisib.

5. The composition for use of claim 2, wherein the method further comprises administering an mTOR inhibitor;
preferably wherein the mTOR inhibitor is selected from sirolimus, temsirolimus, and everolimus.

6. The composition for use of any one of the preceding claims, wherein the ER-associated cancer is metastatic breast cancer and the subject has previously been treated with a selective estrogen receptor modulator; more preferably wherein the estrogen receptor modulator is tamoxifen, raloxifene, or toremifene.

7. The composition for use of any one of the preceding claims, wherein the composition is administered orally.

8. A compound for use in a method of preventing metastatic spread of an ER-associated cancer to the brain of a subject, the method comprising administering to the subject Compound 1: 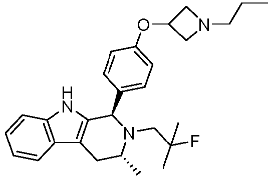 or a pharmaceutically acceptable salt thereof.

## Patentansprüche

1. Zusammensetzung zur Verwendung in einem Verfahren zur Behandlung einer ER-assoziierten Krebserkrankung in einem Subjekt, wobei das Verfahren die folgenden Schritte umfasst:
Verabreichung einer Zusammensetzung an das Subjekt, die eine Verbindung umfasst: 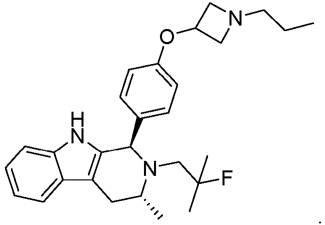
oder ein pharmazeutisch verträgliches Salz davon,
wobei bei dem Subjekt festgestellt wurde, dass es Hirnmetastasen hat.

2. Zusammensetzung zur Verwendung nach Anspruch 1, wobei das Verfahren ferner die Verabreichung eines CDK 4/6-Inhibitors, eines PI3KCA-Inhibitors oder eines mTOR-Inhibitors umfasst.

3. Zusammensetzung zur Verwendung nach Anspruch 2, wobei das Verfahren ferner die Verabreichung eines CDK4/6-Inhibitors umfasst;
vorzugsweise wobei der CDK4/6-Inhibitor aus Palbociclib, Ribociclib, Abemaciclib, Lerociclib und Trilaciclib ausgewählt ist;
bevorzugter, wobei der CDK4/6-Inhibitor aus Ribociclib, Palbociclib und Abemaciclib ausgewählt ist;
noch bevorzugter, wobei der CDK4/6-Inhibitor Ribociclib ist; oder noch bevorzugter, wobei der CDK4/6-Inhibitor Palbociclib ist.

4. Zusammensetzung zur Verwendung nach Anspruch 2, wobei das Verfahren ferner die Verabreichung eines PIK3CA-Inhibitors umfasst;
vorzugsweise wobei der PIK3CA-Inhibitor aus Alpelisib und Taselisib ausgewählt ist;
bevorzugter, wobei der PIK3CA-Inhibitor Alpelisib ist.

5. Zusammensetzung zur Verwendung nach Anspruch 2, wobei das Verfahren ferner die Verabreichung eines mTOR-Inhibitors umfasst;
bevorzugt, wobei der mTOR-Inhibitor aus Sirolimus, Temsirolimus und Everolimus ausgewählt ist.

6. Zusammensetzung zur Verwendung nach einem der vorhergehenden Ansprüche, wobei die ER-assoziierte Krebserkrankung ein metastasierender Brustkrebs ist und das Subjekt zuvor mit einem selektiven Östrogenrezeptormodulator behandelt wurde;
bevorzugter, wobei der Östrogenrezeptormodulator Tamoxifen, Raloxifen oder Toremifen ist.

7. Zusammensetzung zur Verwendung nach einem der vorhergehenden Ansprüche, wobei die Zusammensetzung oral verabreicht wird.

8. Verbindung zur Verwendung in einem Verfahren zur Verhinderung einer metastasierenden Ausbreitung einer ER-assoziierten Krebserkrankung auf das Gehirn eines Subjekts, wobei das Verfahren die Verabreichung der Verbindung 1 an das Probanden umfasst: 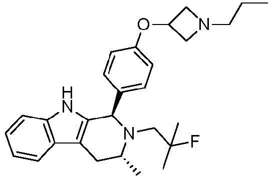 oder ein pharmazeutisch verträgliches Salz davon.

## Revendications

1. Composition destinée à être utilisée dans un procédé de traitement d'un cancer associé au RE chez un sujet, le procédé comprenant les étapes suivantes:
administration au sujet d'une composition comprenant un composé: 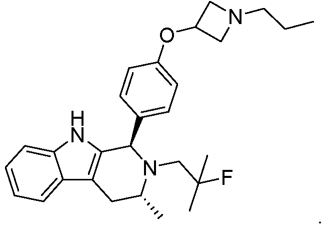
ou un sel pharmaceutiquement acceptable de celui-ci,
dans lequel il a été déterminé que le sujet a des métastases cérébrales.

2. Composition destinée à être utilisée selon la revendication 1, dans laquelle le procédé comprend en outre l'administration d'un inhibiteur de CDK 4/6, d'un inhibiteur de PI3KCA ou d'un inhibiteur de mTOR.

3. Composition destinée à être utilisée selon la revendication 2, dans laquelle le procédé comprend en outre l'administration d'un inhibiteur de CDK4/6;
de préférence, dans lequel l'inhibiteur de CDK4/6 est sélectionné parmi le palbociclib, le ribociclib, l'abémaciclib, le lérociclib et le trilaciclib;
plus préférablement, dans lequel l'inhibiteur de CDK4/6 est sélectionné parmi le ribociclib, le palbociclib et l'abémaciclib;
encore plus préférentiellement, dans lequel l'inhibiteur de CDK4/6 est le ribociclib; ou encore plus préférentiellement, dans lequel l'inhibiteur de CDK4/6 est le palbociclib.

4. Composition destinée à être utilisée selon la revendication 2, dans laquelle le procédé comprend en outre l'administration d'un inhibiteur de PIK3CA;
de préférence, l'inhibiteur de PIK3CA est sélectionné parmi l'alpelisib et le taselisib;
plus préférablement, dans lequel l'inhibiteur de PIK3CA est l'alpelisib.

5. Composition destinée à être utilisée selon la revendication 2, dans laquelle le procédé comprend en outre l'administration d'un inhibiteur de mTOR;
de préférence, dans lequel l'inhibiteur de mTOR est sélectionné parmi le sirolimus, le temsirolimus et l'évérolimus.

6. Composition destinée à être utilisée selon l'une quelconque des revendications précédentes, dans laquelle le cancer associé au RE est un cancer du sein métastatique et le sujet a déjà été traité avec un modulateur sélectif des récepteurs d'œstrogènes;
plus préférablement, dans laquel le modulateur des récepteurs d'œstrogènes est le tamoxifène, le raloxifène ou le torémifène.

7. Composition destinée à être utilisée selon l'une quelconque des revendications précédentes, dans laquelle la composition est administrée par voie orale.

8. Composé destiné à être utilisé dans un procédé de prévention de la propagation métastatique d'un cancer associé au RE dans le cerveau d'un sujet, le procédé comprenant l'administration au sujet du composé 1 : 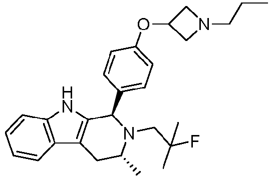 ou un sel pharmaceutiquement acceptable de celui-ci.
